# EUROPEAN PATENT SPECIFICATION

(11) **EP 0 669 827 B1**
(45) Date of publication and mention of the grant of the patent: **23.05.2001**
(21) Application number: 94901394.0
(22) Date of filing: 09.11.1993
(51) Int. Cl.: A61K 31/715, C08B 37/10, A61K 31/70, C07H 11/00

(54) **COMPOSITIONS FOR THE REGULATION OF CYTOKINE ACTIVITY**
ZUSAMMENSETZUNGEN FÜR DIE REGULATION DER CYTOKINAKTIVITÄT
COMPOSITIONS UTILISEES DANS LA REGULATION DE L'ACTIVITE DE LA CYTOKINE

(30) Priority: 10.11.1992 US 974750; 23.07.1993 US 96739
(43) Date of publication of application: 06.09.1995
(60) Divisional application: 00123134.9
(73) Proprietor: YEDA RESEARCH AND DEVELOPMENT CO. LTD., Rehovot 76100 (IL)
(72) Inventor: COHEN, Irun R., 76 100 Rehovot (IL); LIDER, Ofer, 76 100 Rehovot (IL); CAHALON, Liora, Givataim (IL); SHOSEYOV, Oded, Karme Yosef D.N., 72 910 Shimshon (IL); MARGALIT, Raanan, 76 503 Rehovot (IL)
(74) Representative: Hirsch, Marc-Roger
(86) International application number: US9310868
(87) International publication number: WO9411006

(56) References cited:
- EP-A- 0 084 999
- US-A- 4 401 662
- US-A- 4 727 063
- US-A- 4 818 816
- US-A- 4 933 326
- US-A- 4 990 502
- US-A- 5 034 520
- COMP. BIOCHEM. PHYSIOL., vol. 103B, no. 2, 1992, pages 473-479, XP000561260 Y. KARIYA ET AL.: "Preparation of unsaturated disaccharides by eliminative cleavage of heparin and heparan sulfate with heparitinases."

## Description

### 1. FIELD OF THE INVENTION

The present invention relates to 4-O-(2-deoxy-6-O-sulfo-2-sulfoamino-α-D-glycopyranosyl)-(2-O-sulfo-β-D-glucopyranoside)uronic acid, compositions, and the use thereof for the regulation of cytokine activity, for instance, the down regulation of Tumor Necrosis Factor alpha (TNF-α) activity. In particular, said substance and pharmaceutically acceptable compositions are disclosed which, when administered to a host in effective amounts, either inhibit the secretion of active TNF-α by host cells. It is thought that the secretion of active cytokines, for example TNF-α, by the host's immune effector cells (e.g., the host's activated macrophages) may be inhibited by the of the present invention.

The present invention is also useful in methods for the prevention and/or treatment of pathological processes or, conversely, the initiation of a beneficial immune system-related response involving the induction of cytokine production, secretion, and/or activity. Selected compositions of the present invention comprise an effective low dosage of a low molecular weight heparin (LMWH) to be administered at intervals of up to between five to eight days. Still other compositions include said substance in substantially purified form obtained from enzymatically degraded extracellular matrix (DECM).

Individually, the above described substance and pharmaceutical compositions especially suited for parenteral, oral, or topical administration, inhibit TNF-α secretion by resting T cells and/or macrophages *in vitro* in response to activation by immune effector cell activators, including, but not limited to, T cell-specific antigens, T cell mitogens, macrophage activators, residual extracellular matrix (RECM), fibronectin, laminin or the like. *In vivo* data, showing inhibition of experimental delayed type hypersensitivity (DTH), are also presented in further support of the *in vitro* results.

### 2. BACKGROUND OF THE INVENTION

### 2.1. Tumor Necrosis Factor Alpha

TNF-α, a cytokine produced by monocytes (macrophages) and T lymphocytes, is a key element in the cascade of factors that produce the inflammatory response and has many pleiotropic effects as a major orchestrator of disease states (Beutler, B. and Cerami, A., Ann. Rev. Immunol. (1989) 7:625-655).

The biologic effects of TNF-α depend on its concentration and site of production: at low concentrations, TNF-α may produce desirable homeostatic and defense functions, but at high concentrations, systemically or in certain tissues, TNF-α can synergize with other cytokines, notably interleukin-1 (IL-1) to aggravate many inflammatory responses.

The following activities have been shown to be induced by TNF-α (together with IL-1); fever, slow-wave sleep, hemodynamic shock, increased production of acute phase proteins, decreased production of albumin, activation of vascular endothelial cells, increased expression of major histocompatibility complex (MHC) molecules, decreased lipoprotein lipase, decreased cytochrome P450, decreased plasma zinc and iron, fibroblast proliferation, increased synovial cell collagenase, increased cyclo-oxygenase activity, activation of T cells and B cells, and induction of secretion of the cytokines, TNF-α itself, IL-1, IL-6, and IL-8. Indeed, studies have shown that the physiological effects of these cytokines are interrelated (Philip, R. and Epstein, L. B., Nature (1986) 323(6083):86-89; Wallach, D. et al., J. Immunol. (1988) 140(9):2994-2999).

How TNF-α exerts its effects is not known in detail, but many of the effects are thought to be related to the ability of TNF-α to stimulate cells to produce prostaglandins and leukotrienes from arachidonic acid of the cell membrane.

TNF-α, as a result of its pleiotropic effects, has been implicated in a variety of pathologic states in many different organs of the body. In blood vessels, TNF-α promotes hemorrhagic shock, down regulates endothelial cell thrombomodulin and enhances a procoagulant activity. It causes the adhesion of white blood cells and probably of platelets to the walls of blood vessels, and so, may promote processes leading to atherosclerosis, as well as to vasculitis.

TNF-α activates blood cells and causes the adhesion of neutrophils, eosinophils, monocytes/macrophages and T and B lymphocytes. By inducing IL-6 and IL-8, TNF-α augments the chemotaxis of inflammatory cells and their penetration into tissues. Thus, TNF-α has a role in the tissue damage of autoimmune diseases, allergies and graft rejection.

TNF-α has also been called cachectin because it modulates the metabolic activities of adipocytes and contributes to the wasting and cachexia accompanying cancer, chronic infections, chronic heart failure, and chronic inflammation. TNF-α may also have a role in anorexia nervosa by inhibiting appetite while enhancing wasting of fatty tissue.

TNF-α has metabolic effects on skeletal and cardiac muscle. It has also marked effects on the liver: it depresses albumin and cytochrome P450 metabolism and increases production of fibrinogen, 1-acid glycoprotein and other acute phase proteins. It can also cause necrosis of the bowel.

In the central nervous system, TNF-α crosses the blood-brain barrier and induces fever, increased sleep and anorexia. Increased TNF-α concentration is associated with multiple sclerosis. It further causes adrenal hemorrhage and affects production of steroid hormones, enhances collagenase and PGE-2 in the skin, and causes the breakdown of bone and cartilage by activating osteoclasts.

In short, TNF-α is involved in the pathogenesis of many undesirable inflammatory conditions in autoimmune diseases, graft rejection, vasculitis and atherosclerosis. It may have roles in heart failure and in the response to cancer. For these reasons, ways have been sought to regulate the production, secretion, or availability of active forms of TNF-α as a means to control a variety of diseases.

The prime function of the immune system is to protect the individual against infection by foreign invaders such as microorganisms. It may, however, also attack the individual's own tissues leading to pathologic states known as autoimmune diseases. The aggressive reactions of an individual's immune system against tissues from other individuals are the reasons behind the unwanted rejections of transplanted organs. Hyper-reactivity of the system against foreign substances causes allergy giving symptoms like asthma, rhinitis and eczema.

The cells mastering these reactions are the lymphocytes, primarily the activated T lymphocytes, and the pathologic inflammatory response they direct depends on their ability to traffic through blood vessel walls to and from their target tissue. Thus, reducing the ability of lymphocytes to adhere to and penetrate through the walls of blood vessels may prevent autoimmune attack, graft rejection and allergy. This would represent a new therapeutic principle likely to result in better efficacy and reduced adverse reactions compared to the therapies used today.

Atherosclerosis and vasculitis are chronic and acute examples of pathological vessel inflammation. Atherosclerosis involves thickening and rigidity of the intima of the arteries leading to coronary diseases, myocardial infarction, cerebral infarction and peripheral vascular diseases, and represents a major cause of morbidity and mortality in the Western world. Pathologically, atherosclerosis develops slowly and chronically as a lesion caused by fatty and calcareous deposits. The proliferation of fibrous tissues leads ultimately to an acute condition producing sudden occlusion of the lumen of the blood vessel.

TNF-α has been shown to facilitate and augment human immunodeficiency virus (HIV) replication in vitro (Matsuyama, T. et al., J. Virol. (1989) 63(6):2504-2509; Michihiko, S. et al., Lancet (1989) 1(8648):1206-1207) and to stimulate HIV-1 gene expression, thus, probably triggering the development of clinical AIDS in individuals latently infected with HIV-1 (Okamoto, T. et al., AIDS Res. Hum. Retroviruses (1989) 5(2):131-138).

Hence, TNF-α, like the inflammatory response of which it is a part, is a mixed blessing. Perhaps in understanding its physiologic function, one may better understand the purpose of inflammation as a whole and gain insight into the circumstances under which "TNF-α deficiency" and "TNF-α excess" obtain. How best to design a rational and specific therapeutic approach to diseases that involve the production of this hormone may thus be closer at hand.

### 2.2. Heparin

Heparin is a glycosaminoglycan, a polyanionic sulfated polysaccharide, which is used clinically to prevent blood clotting as an antithrombotic agent. In animal models, heparin has been shown to reduce the ability of autoimmune T lymphocytes to reach their target organ (Lider, O. et al., Eur. J. Immunol. (1990) 20:493-499). Heparin was also shown to suppress experimental autoimmune diseases in rats and to prolong the allograft survival in a model of skin transplantation in mice, when used in low doses (5 µg for mice and 20 µg for rats) injected once a day (Lider, O. et al., J. Clin. Invest. (1989) 83:752-756).

The mechanisms behind the observed effects are thought to involve inhibition of release by T lymphocytes of enzyme(s) necessary for penetration of the vessel wall, primarily the enzyme heparanase that specifically attacks the glycosaminoglycan moiety of the sub-endothelial extracellular matrix (ECM) that lines blood vessels (Naparstek, Y. et al., Nature (1984) 310:241-243). Expression of the heparanase enzyme is associated with the ability of autoimmune T lymphocytes to penetrate blood vessel walls and to attack the brain in the model disease experimental autoimmune encephalomyelitis (EAE).

European Patent Application EP 0114589 (Folkman et al.) describes a composition for inhibition of angiogenesis in mammals in which the active agents consist essentially of (1) heparin or a heparin fragment which is a hexasaccharide or larger and (2) cortisone or hydrocortisone or the 11-α isomer of hydrocortisone. According to the disclosure, heparin by itself or cortisone by itself are ineffective; only the combination of both gives the desired effects. Although there is no proof in the literature that there is a connection between angiogenesis and autoimmune diseases, the description on page 5 of the patent application connects angiogenesis with psoriasis and with arthritis, indicating the use of high doses of 25,000 units to 47,000 units of heparin per day (i.e., about 160 to about 310 mg per day).

Horvath, J. E. et al.,in Aust. N.Z.J. Med. (1975) 5(6):537-539, describe the effect of subanticoagulant doses of subcutaneous heparin on early renal allograft function. The daily dosage is high (5000 U or about 33 mg) and the conclusion of the study is that heparin in subanticoagulant doses has no effect on early graft function or graft survival and that it may be associated with increased hemorrhagic complications.

Toivanen, M. L. et al., Meth. and Find. Exp. Clin. Pharmacol. (1982) 4(6):359-363, examined the effect of heparin in high dosage (1000 U/rat or about 7 mg/rat) in the inhibition of adjuvant arthritis in rats and found that heparin enhanced the severity of the rat adjuvant arthritis.

PCT Patent: Application PCT/AU88/00017 published under No. WO88/05301 (Parish et al.) describes sulphated polysaccharides that block or inhibit endoglycosylase activity, such as heparanase activity, for use as antimetastatic and anti-inflammatory agents. Heparin and heparin derivatives, such as periodate oxidized, reduced heparins, that had negligible anticoagulant activity, were shown to have antimetastatic and anti-inflammatory activity when used in dosages within, the range of 1.6-6.6 mg per rat daily, administered by constant infusion (corresponding to 75-308 mg daily for an adult human patient).

Heparin and heparan sulfate are closely related glycosaminoglycan macromolecules. The degradation products of these polymeric macromolecules, which are termed low molecular weight heparins (LMWH), may have the same or greater pharmacologic effects on the blood clotting system as the parent macromolecules. Furthermore, because there is extensive but incomplete post-synthetic processing of the polymer's basic disaccharide subunit, glucuronic acid and N-acetyl glucosamine, the LMWH will be a heterogeneous mixture not only of sizes but also of chemical compositions (See Goodman and Gilman's The Pharmacological Basis of Therapeutics, 8th Ed., (Pergamon Press, New York, 1990) pp. 1313-1315. Methods to obtain low molecular weight products from heparin, which are useful as anticoagulants, are described in the art. These methods seek to optimize the persistence *in vivo* or the extent of hemorrhagic side effects of their products (See, for example, Alpino R. R., et al., U.S. Patent No. 5,010,063; Choay, J., et al., U.S. patent No. 4,990,502; Lopez, L. L., et al., U.S. Patent No. 4,981,955). Others teach the use of affinity chromatographic methods to obtain low molecular weight products (See, for example, Rosenberg, R. D., et al., U.S. Patent No. 4,539,398 and Jordan, R. E., et al., U.S. Patent No. 4,446,314).

Psuja, P., as reported in Folio Haematol. (Leipz), (1987) 114:429-436, studied the effect of the heterogeneity of heparins on their interactions with cell surfaces. Psuja reported that there are moderate affinity receptors for LMWH (D_{d}= 5.6 µM) found on cultured endothelial cells, but he determined that the upper limit of the fraction of LMWH bound to these receptors was less than 1% of total LMWH.

Other workers have demonstrated effects of LMWH on the metabolism of a variety of cultured cell types. Asselot-Chapel, C., et al., in Biochem. Pharmacol. (1989) 38:895-899 and Biochem. Biophys. Acta, (1989) 993:240-244, report that LMWH cause cultured smooth muscle cells to decrease the ratio of type III to type I collagen and fibronectin synthesis. Rappaport, R. in U.S. Patent No. 4,889,808, teaches that LMWH can cause human diploid pulmonary fibroblasts, cultured in the absence of serum, to respond to LMWH by increased secretion of tissue plasminogen activator and related proteins.

Effects of LMWH on complex multicellular systems have been reported. The work of Folkman et al. and Lider et al., in EPO Application 0114589 and J. Clin. Invest. (1989) 83:752:756, have been noted above. In addition, Diferrante, N., in published International Application WO 90/03791, teaches the use of LMWH to inhibit the reproduction of HIV in cultures of C8166 transformed human lymphocytes (ALL). However, none of the prior art experiments that have studied the effects of LMWH on cellular metabolism has considered that the heterogeneity of LMWH may produce antagonistic effects. Furthermore, none has shown or suggested a regulatory effect on cytokine activity based on the use of substantially pure oligosaccharide substances.

EP-A-0084999 discloses a process of preparation of oligosaccharides constituting or corresponding to fragments of muco-polysaccharides comprising the reaction between two compounds constituted or terminated by specifically substituted amino-sugar units and uronic acid unit. This process leading to therapeutically useful oligosaccharides.

### 3. SUMMARY OF THE INVENTION

In the present invention, 4-O-(2-deoxy-6-O-sulfo-2-sulfoamino-α-D-glycopyra-nosyl)-(2-O-sulfo-β-D-glucopyranoside)uronic acid is disclosed which is capable of inhibiting cytokine activity in a mammalian subject and which is in a substantially purified form. In particular, said substance exhibits a consistent: inhibitory "R" value of about 200,000 % x (µg/gm)⁻¹ or more as determined from an *in vivo* bioassay that measures the relative inhibition of experimental DTH reactions in mice that have been treated with varying dosages of said substance ranging from 0 to about 2 µg/gm mouse.

Furthermore, the substance of the present invention having an inhibitory effect on the secretion of active TNF-α may, in addition, exhibit a consistent inhibitory "R" value of at least about 0.4 % x (pg/ml) ¹ as determined from an *in vitro* bioassay that measures the relative activity of TNF-α that is secreted by activated human CD4 + T cells in the presence of varying concentrations of said substance from 0 to about 1 x 10⁷ pg/ml.

When purified this substance or the compositions containing same are substantially free of other substances that exert the opposite or antagonistic effect. Thus, the substance exhibiting inhibitory activity ("down" regulation) in a substantially purified form would be substantially free not only of other substances, in general, but of other substances that exhibit augmentation or retard the inhibitory activity of the "down" regulator.

The phrase "regulatory effect" includes down regulation of any process affecting the availability or resulting activity *in vivo* or *in vitro* of cytokines, in general, including IL-1, IL-6, IL-8 and, in particular, TNF-α. Thus, compositions of the present invention may exert a regulatory effect on the host production of TNF-α, on the host secretion of TNF-α, on the extracellular availability of TNF-α, or on the active forms of TNF-α in a host. For instance, but not wishing to be limited by theory, the instant invention may act to elicit the secretion of a substance, such as a protein, which may bind to TNF-α, change its conformation, and, consequently, affect its biological activity. It is also possible that the compositions of the present invention may, in penetrating activated T cells or macrophages, bind to particular oligonucleotide sequences and, thus, affect transcriptional or translational processes that ultimately alter protein synthesis. The compositions may also work through binding to cell surface receptors.

To simplify the following discussion, reference will be made, among others, to the "secretion of active TNF-α" or the regulation of the "activity of TNF-α" with the understanding that a much broader meaning is to be attached to these phrases which encompasses the actual mechanism that is responsible for or the actual manner by which the observed inhibition of TNF-α activity is effected by the substance and compositions of the present invention.

The oligosaccharide moiety may be obtained from natural sources, including living organisms, for extracellular matrices that have been degraded by the action of an enzyme, e.g., heparanase derived from animals (mammals) or microorganisms (bacteria).

Hence, the term "substantially purified form" means that specific steps have been taken to remove non-active components, or components that have an opposing effect, from the substance and to isolate the active moiety or moieties from mixtures or supernatants, such as those obtained from enzymatic degradation. Specifically, the substance claimed in the present invention is obtained from a rigorous chromatographic process, in which low pressure size-exclusion gel chromatography (i.e., chromatography on Sephadex columns) is but an initial step in the purification scheme. Subsequent to the low pressure separation, high pressure liquid chromatographic (HPLC) techniques are used to isolate individual component oligosaccharides. Preferably, these steps have resulted in the purification of the individual active substance to substantial homogeneity.

Such a preferred purification step may include, for example, passing mixtures containing the active substance (e.g., fractions obtained from low pressure gel chromatography) through gel permeation HPLC or strong anion exchange (SAX) HPLC columns.

Herein there is described a bioassay for quantifying the effect of a test substance on the secretion of active TNF-α. The bioassay comprises the steps of preincubating human CD4⁺ T cells in a medium with varying concentrations of a test substance, adding a constant amount of an activator effective to elicit the secretion of TNF-α by the T cells in the absence of said test substance, collecting the medium after a sufficient period of time, and subsequently testing the activity of the TNF-α in the medium. Preferably, the human CD4⁺ T cells are obtained from peripheral blood mononuclear leukocytes (PBL). Suitable immune effector cell activators include, but are not limited to, T cell-specific antigens, mitogens, macrophage activators, residual extracellular matrix (RECM, defined in Section 4, below), laminin, fibronectin, and the like.

The present invention relies on the specific regulatory activity of 4-O-(2-deoxy-6-O-sulfo-2-sulfoamino-α-D-glycopyranosyl)-(2-O-sulfo-β-D-glucopyranoside)uronic acid as determined by *in vitro* and *in vivo* bioassays described in greater detail, below. Briefly, said substance useful in the present invention displays an inhibitory activity relating to the induction of the secretion of active TNF-α which is dose dependent. That is, a plot of the percent inhibition versus the dose (e.g., pg/ml of substance) gives rise to a bell-shaped curve from which a maximum percent inhibition (Inhₘₐₓ) is readily apparent. Thus, for every point on such a plot, a "ratio" between the percent inhibition or augmentation and the concentration or dose can be calculated. In the present case, a "specific regulatory activity" or "R" value can be obtained from the ratio of the maximum percent inhibition (i.e., Inhₘₐₓ and the concentration or dose of test substance which gave rise to such maximum percent regulatory value. Furthermore, an "R" value can be obtained for each bioassay. Hence, an "R" value can be associated from an *in vitro* mouse spleen cell assay, an *ex vivo* mouse spleen assay, an *in vitro* human PBL assay, and an *in vivo* assay based on experimental DTH reaction. If no effect is observed, an "R" value of zero is assigned.

Another object of the present invention is a method of using 4-0-(2-deoxy-6-0-sulfo-2-sulfonamino-α-D-glycopyranosyl)-(2-0-sulfo-β-D-glucopyranoside)uronic acid for the preparation of a pharmaceutical composition for the prevention or treatment of a medical condition caused by or related to the inappropriate production of TNF-α.

The present invention also concerns a method of using the above-described substance for the preparation of a pharmaceutical composition for the treatment of a medical condition responsive to an inhibited production of TNF-α.

The preparation of a pharmaceutical preparation useful for the treatment of the host comprises combining the substance with a pharmaceutically acceptable carrier to provide a unit dose, preferably of low dosage, having an effective amount of the substance. The pharmaceutical preparation may also comprise a stabilizing agent, for example, protamine, in an amount sufficient to preserve a significant, if not substantial, proportion of the initial activity of the substance over an extended period, e.g., about 100 percent over about 3 days. At storage temperatures below room temperature, e.g., about -10 to about 10 °C, preferably 4 °C, more of the initial activity is preserved, for up to about 4 months.

Because the pharmaceutical compositions of the present invention are contemplated for administration into humans, the pharmaceutical compositions are preferably sterile. Sterilization is accomplished by any means well known to those having ordinary skill in the art, including use of sterile ingredients, heat sterilization or passage of the composition through a sterile filter.

It should also be evident that a primary object of the present invention is to provide a method of treating a host, such as a mammalian subject, suffering from a medical condition the severity of which can be affected by the activity of a cytokine in the host. Depending on the medical condition of the particular host, pharmaceutical composition can be administered which reduces the availability or activity of TNF-α. Such compositions or pharmaceutical preparations may be administered at low dosage levels and at intervals of up to about 5-8 days, preferably, once a week. Pharmaceutical compositions containing 4-O-(2-deoxy-6-O-sulfo-2-sulfoamino-α-D-glycopyranosyl)-(2-O-sulfo-β-D-glucopyranoside)uronic acid for parenteral, oral, or topical administration may be administered daily according to convenience and effectiveness and at dosages that would be readily determined by routine experimentation by one of ordinary skill.

Active substance and composition of the present invention are capable of inhibiting experimental delayed type hypersensitivity (DTH) reactions to an applied antigen as evidenced by a reduction in the induration observed after the application of the antigen to the skin up to about five to seven days after the administration of the substance or pharmaceutical composition of same relative to the induration observed after the application of the antigen to the skin in the absence of or after recovery from the administration of the substance or pharmaceutical composition of same. Examples of the applied antigen include, but are not limited to, tetanus, myelin basic protein, purified protein derivative, oxazolone, and the like.

Furthermore, it is an objective of the present invention to provide compositions or pharmaceutical preparations that may be administered in any manner as dictated by the particular application at hand including, but not limited to, enteral administration (including oral or rectal) or parenteral administration (including topical or inhalation with the aid of aerosols). In preferred embodiments, the pharmaceutical compositions of the present invention are administered orally, subcutaneously, intramuscularly, intraperitoneally or intravenously.

Thus, the present invention is useful, for example, in delaying or preventing allograft rejection and treating or preventing a variety of pathological processes such as those related to autoimmune diseases, allergy, inflammatory diseases (in particular, inflammatory bowel disease), or acquired immunodeficiency syndrome (AIDS). The present invention also finds utility in the treatment of diabetes type I, periodontal disease, skin diseases, liver diseases, uveitis, rheumatic diseases (in particular, rheumatoid arthritis), atherosclerosis, vasculitis, or multiple sclerosis.

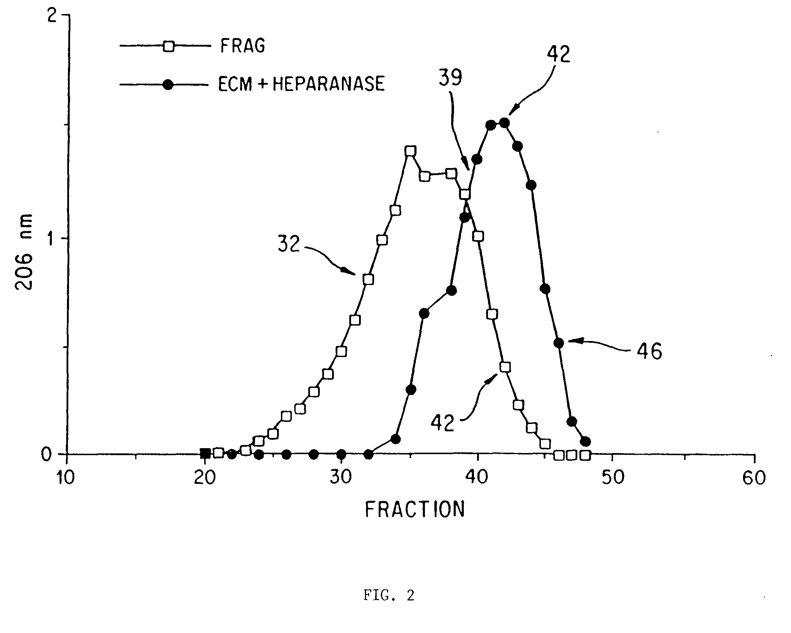

Fig. 2 illustrates the absorption at 206 nanometers versus fraction number for a number of fractions obtained from the Sepharose 4B column separation of Fragmin and heparanase-degraded ECM.

Figs. 1 and 3 compare the elution profiles of fractions obtained from the Sepharose 4B column separation of Fragmin and heparanase-degraded ECM.

Fig. 4 shows that an oligosaccharide product (fraction 5 from Fig. 1) demonstrated a similar bell-shaped dose/response curve in its ability to inhibit the secretion of active TNF-α.

Fig. 5 shows that the areas of greatest anti-TNF-α effect lie in the subfraction between about 5.65 and about 5.8.

Figs. 6A and 6B illustrate the chromatogram obtained from the HPLC separation of Fragmin and heparanase-degraded ECM, respectively.

Fig. 7 illustrates the absorption at 206 nanometers of two fractions, F5 and F8, obtained from the Sepharose 4B column separation of heparanase-degraded ECM.

Figs. 8A and 8B, on the other hand, illustrate the absorption at 206 and 232 nanometers, respectively, of a peak obtained from the HPLC separation of fraction F5.

Figs. 9A and 9B illustrate the uv absorption of additional HPLC fractions obtained from fraction F5.

Fig. 10 illustrates the uv absorption of fractions F7 and F8 obtained from the Sepharose 4B column separation of heparanase-degraded ECM.

Fig. 11 illustrates the substantially pure peak obtained from the SAX-HPLC chromatography of combined fractions F7 and F8.

Fig. 12 illustrates another peak labeled "A23/4" obtained from desalted preparations of the peak labeled "1" from Fig. 11.

Figs. 13A, 13B and 13C illustrate the chromatograms that are obtained from the SAX-HPLC column separation of disaccharide standards obtained from Sigma labeled H-0895, H-1020 and H-9267, respectively.

Fig. 14 illustrates the Sepharose 4B column separation of a mixture obtained from the heparanase (MM 5) treatment of Heparin, yielding fractions F7 and F8.

Fig. 15 illustrates the absorption at 206 nanometers of various fractions obtained from the Sepharose 4B chromatography of PC3 heparanase alone and Heparin + PC3.

Figs. 16A and 16B illustrate additional fractions obtained from the HPLC separation of fraction F7 from Figure 14.

Fig. 17 illustrates fraction F90 obtained from the HPLC separation of Fragmin.

Fig. 18, on the other hand, illustrates the chromatogram that is obtained from a SAX-HPLC separation of an aged sample of A23/4.

Fig. 19 illustrates the proton NMR spectrum of a 20 microgram sample of an ECM-derived disaccharide obtained from HPLC chromatography, as shown in Fig. 11.

Fig. 20 illustrates a two-dimensional COSY spectrum of the sample of Fig. 19.

Fig. 21 illustrates an expanded portion of the NMR spectrum of Fig. 20, showing the signal for the anomeric proton.

Figs. 22 and 23 illustrate the FTIR spectra obtained from two separate samples, one indicating the presence of a sulfated compound (Fig. 22) and the other indicating the presence of a partially desulfated analog (Fig. 23).

Fig. 24A illustrates the mass spectrum of a methylated derivative of the sample obtained from Fig. 11 in a solvent matrix comprised of DTT:thioglycerol (1:1).

Fig. 24B illustrates the mass spectrum of the solvent matrix only.

Figs. 25A and 25B illustrate the mass spectrum of the same sample in a different solvent matrix comprised of methylnitrobenzyl alcohol, Fig. 25A being the mass spectrum of the sample plus the solvent matrix and Fig. 25B being the mass spectrum of the solvent matrix only.

Fig. 26 illustrates the results of experiments comparing the effectiveness of disaccharide 9392 and 1020 to improve the AA scores of female Lewis rats suffering from experimentally induced adjuvant arthritis.

Fig. 26A illustrates the effect of disaccharide 0895 on the AA scores of rats suffering from experimentally induced AA relative to control (PBS).

Fig. 26B illustrates the effects of glucosamine treatment in the improvement of the AA score of Lewis rats under various dosages of glucosamine.

Fig. 26C, similarly, shows the effect of galactosamine at different dosages on the AA score of Lewis rats.

Figs. 26D and 26E illustrate the results of further experiments carried out with disaccharide 9392 in which the disaccharide is administered either weekly or daily beginning at day zero (i.e., start of induction of AA) or at day 12 (i.e., when the rat is already suffering from AA).

Figs. 26F and 26G illustrate the results of a separate comparative set of experiments that were carried out on groups of Lewis rats to determine the effectiveness of disaccharide 9392 administered weekly compared with the effectiveness of a known anti-inflammatory agent, dexamethasone phosphate, on the suppression of experimentally induced adjuvant arthritis.

Fig. 27 illustrates the effectiveness of subcutaneously injected disaccharide 1020 against liposaccharide (LPS) induced inflammation of rat corneas.

Fig. 27A presents the results of experiments relating to the radioprotective effects of glucosamine at various dosages relative to control (PBS).

Fig. 27B presents the results of similar irradiation experiments involving the administration of disaccharide 9392 at various dosages relative to control (PBS).

Figs. 28 and 28A illustrate the results of experiments that illustrate the ability of selected substances of the present invention to suppress allograft rejection. The results presented in Fig. 28 show that a 3 nanogram dose of disaccharide 9392 by subcutaneous injection one day before grafting and weekly thereafter, delayed the level of skin graft rejection at 50% by 5 days. However, a 300 nanogram dose of the same disaccharide failed to produce a significant difference at 50% rejection relative to control (PBS).

Fig. 29 illustrates the incidence of IDDM in groups of female NOD mice which had been separately treated with either disaccharide 9392, glucosamine or saline.

Fig. 29A presents the mortality rate of female NOD mice that, again, had been treated separately with the disaccharide 9392, glucosamine, or saline. It should be noted that in both Figs. 29 and 29A, the female NOD mice were approximately 3-1/2 months old, meaning that the mice as a group already endured a 20% incidence of IDDM.

Fig. 30 presents the respiratory distress (RD) score of six immunized rats challenged with aerosolized antigen with (B1, B2 and B3) and without (A1, A2 and A3) treatment by substance H-9392. See text for details.

Fig. 31 represents the structure of 4-O-(2-deoxy-6-O-sulfo-2-sulfoamino-α-D-glucopyranosyl)-(2-O-sulfo-β-D-glucopyranoside) uronic acid, prepared by the synthetic scheme of Example 6.23.

### 4. DETAILED DESCRIPTION OF THE INVENTION

The present invention thus concerns a compound of formula II: 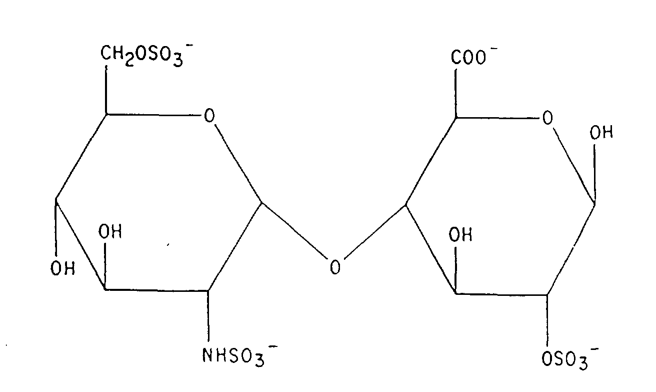 and its pharmaceutically acceptable salt.

The present invention also concerns a pharmaceutical composition comprising an effective amount of said compound of formula II or of its pharmaceutically acceptable salt; this composition being useful for inhibiting the production of an active cytokine in a subject and especially for inhibiting the production of TNF-α.

According to another object, the present invention concerns a method of using the above-described compound or its pharmaceutically acceptable salt for the preparation of a pharmaceutical composition for the prevention or treatment of a medical condition caused by or related to the inappropriate production of TNF-α, in particular, of a medical condition responsive to an inhibited production of TNF-α.

One functional expression of this effect can be seen in the inhibition in mice and humans of the delayed type hypersensitivity (DTH) reaction, a T cell dependent inflammatory reaction that may also be triggered by cells involving macrophages and other inflammatory cells. Treatment with the active substance at doses affecting active TNF-α production also was able to inhibit a model of autoimmune arthritis called adjuvant arthritis (AA). Active substance treatment also prolonged the survival of allogeneic heart transplants in rats and abrogated insulin dependent diabetes mellitus (IDDM) in NOD mice. Moreover, similar treatment prevented the induction of active TNF-α production by T cells and macrophages in response to the stimulus of damaged or residual subendothelial extracellular matrix. This residual extracellular matrix (RECM) that is responsible for signaling the onset of TNF-α induction (and resulting inflammation) is to be distinguished from the enzyme degraded extracellular matrix (DECM), selected components of which have been isolated herein and have been shown to shut down TNF-α activity.

Since TNF-α at the site of vascular injury probably has a role in the process of atherosclerosis, inhibition of TNF-α activity at the site of damaged subendothelial ECM will ameliorate the pathogenic process of atherosclerosis.

### 4.1. Fractionation of LMWH and Preparation of Degraded ECM or Degraded Heparin. Discovery of Distinct Augmentative and Inhibitory Activities For and Against TNF-α Activity

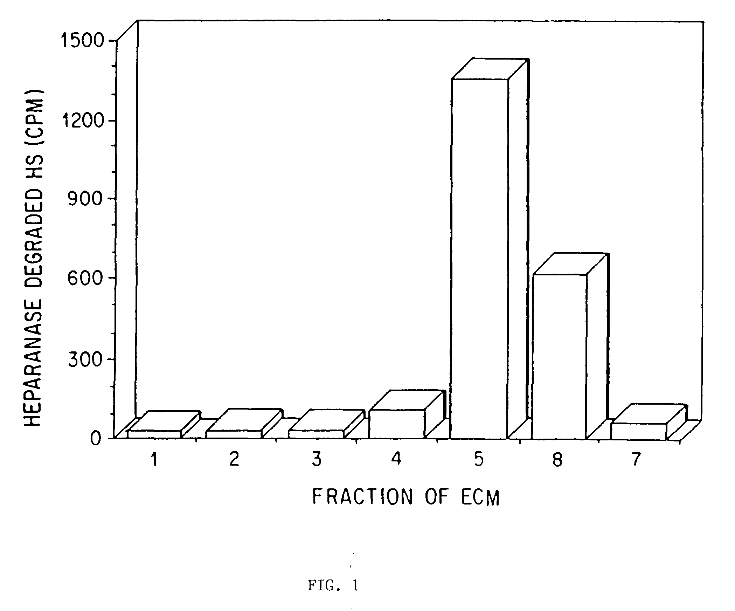

A Sepharose 4B size-exclusion gel chromatographic separation was carried out on the degradation products obtained from heparanase-treated ECM labeled with ³⁵S-containing sulfate groups. Several types of heparanase enzyme were used in the present investigation. These enzymes include MM5 (Mammalian heparanase from human placentas, obtained commercially from Rad-Chemicals, Weizmann Industrial Park, Ness Ziona, Israel), PC3 (Bacterial endoglycosidase, as described in Shoseiov, O. et al., Biochem. Biophys. Res. Commun. (1990) 169:667-672), and an enzyme from a bacterial source obtained from IBEX Technologies, Quebec, Canada. A plot of the radioactivity (CPM) versus fraction number is presented in Fig. 1. Another plot superimposing the elution profiles of fractionated Fragmin and fractionated ECM-heparanase is shown in Fig. 2. The conditions for the Sepharose 4B low-pressure separation are listed in Table I, below.

**Table I**

| Sepharose 4B Chromatography Conditions | |
|---|---|
| Column | Sepharose 4B (35 cm x 0.7 cm ID) |
| Load | 1 - 1.5 ml |
| Flow | 5 ml/hr |
| Solvent | PBS (pH = 7.4) |
| Fraction | 0.2 - 0.5 ml/tube |
| Detector Absorption Setting | 206 nm, 280 nm |

The various fractions were assayed for their effect on TNF-α production and these results are presented in Table XV, below. Interestingly, fractions of similar elution properties from the two sources (i.e., F-39 and F-42 from Fragmin and Heparanase-degraded ECM) were found to have similar qualitative biological effects on TNF-α production and/or activity.

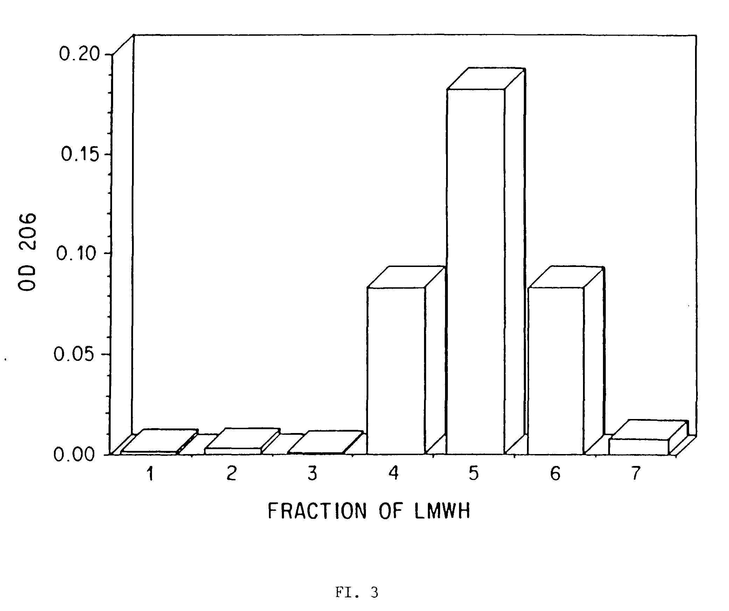

Figs. 3 and 1 illustrate one way of presenting the elution profile, obtained on Sepharose 4B columns, of LMWH (Fragmin) and ³⁵S-sulfate labeled oligosaccharides of ECM, produced by purified MM5 heparanase, respectively. It can be seen in Fig. 1 that the heparan sulfate of the ECM (the substrate of heparanase) is degraded by the enzyme to produce heparan sulfate fragments with elution properties comparable to fractionated LMWH.

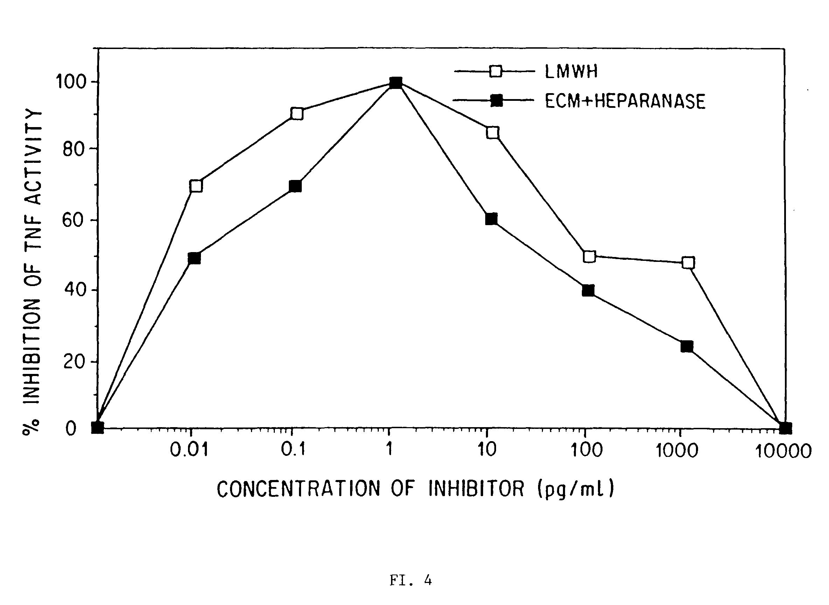

Fig. 4 shows that an oligosaccharide product (Sepharose 4B fraction #5, Fig. 1), obtained from the ECM + heparanase "soup" (i.e., the mixture obtained from the heparanase degradation of ECM), has a substantially similar dose/response characteristic as LMHW in its effects on the secretion of active TNF-α: that is, both display a bell-shaped dose/response curve and both exhibit maximal inhibition of about 90% at a concentration of about 1 pg/ml with less activity at either lower or higher concentrations. It is advantageous, thus, that the administration of these active substances includes dosages falling within an easily determined "window" of physiological effect.

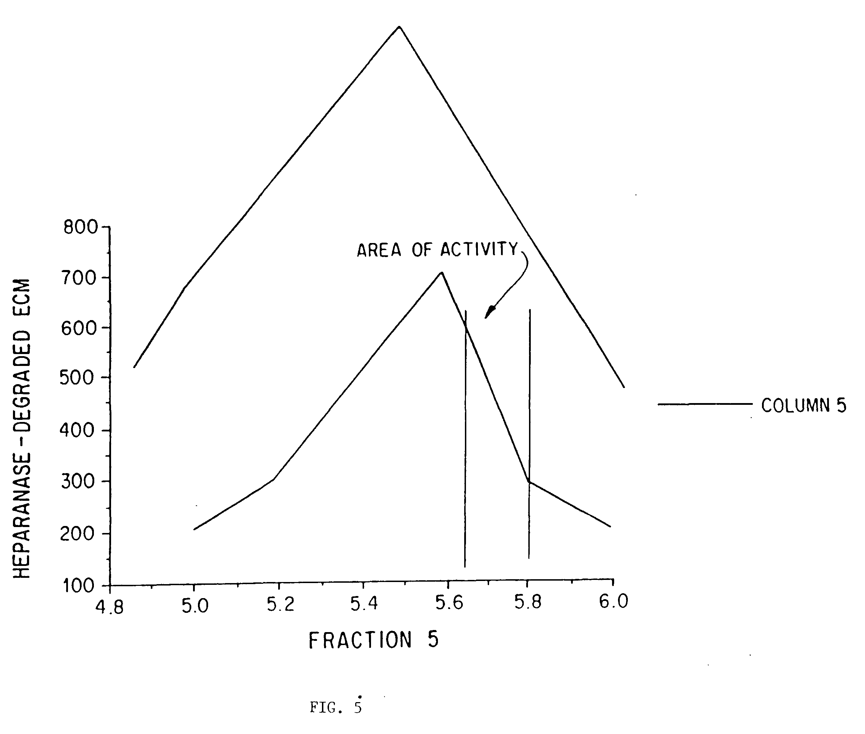

Fig. 5 shows that the anti-TNF-α effect of the ECM-degradation products is highest in the area of a subfraction (between about 5.65 and about 5.80) of the fragments under peak number 5 of Fig. 1.

### 4.2. HPLC Separation of LMWH Fractions and Fragments Obtained from DECM and DH

High performance liquid chromatography ("HPLC") techniques were utilized to obtain better resolution of the fractions from the LMWH (e.g., Fragmin), ECM-degradation, and heparin-degradation samples. Initially, two types of HPLC conditions were used. Under the first set of HPLC conditions, a 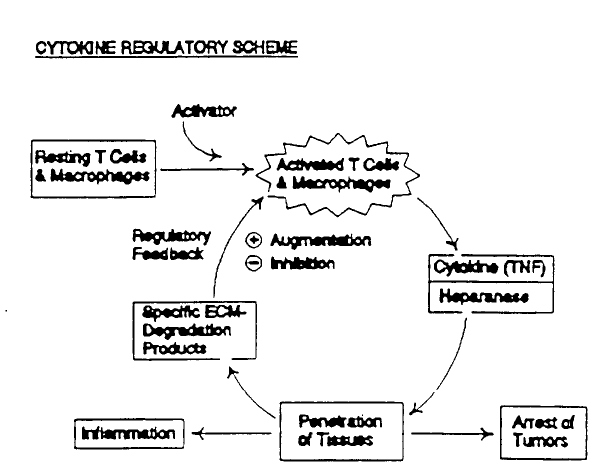 number of individual fractions were separated and isolated; their ability to regulate the secretion of active TNF-α was then examined. To the great surprise of the present inventors, it was discovered that selected fractions can augment the activity of TNF-α in the host while others inhibited TNF-α activity. A second set of HPLC conditions was then utilized to better separate the various components according to their molecular weight.

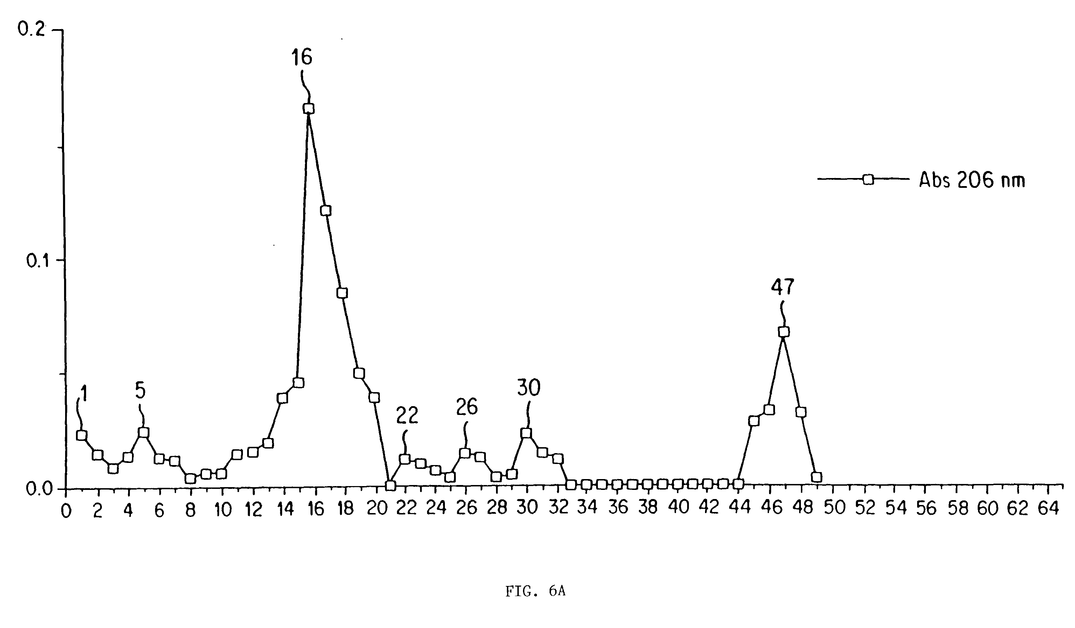

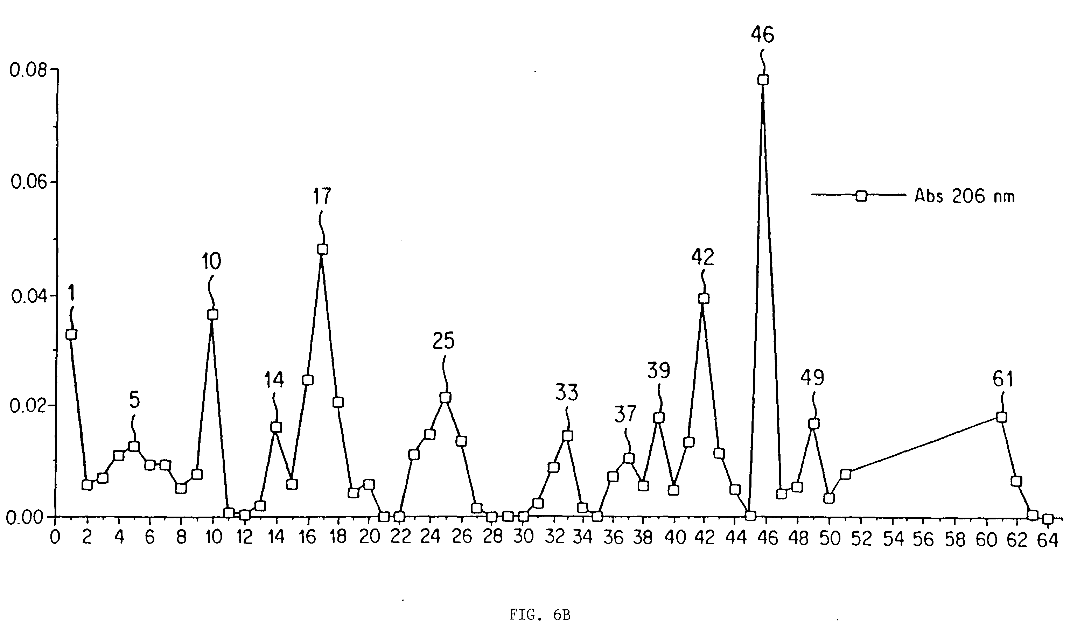

In the first set of HPLC conditions, a TSK-GEL® G-Oligo-PW column (30 cm x 7.8 mm I.D.) equipped with a Guardcolumn Oligo (4 cm x 6 mm I.D.) was used. The conditions ("HPLC I") are provided in Table II, below. A representative chromatogram for the HPLC I separation of Fragmin and ECM + MM5 Heparanase is illustrated in Figs. 6A and 6B, respectively.

**Table II.**

| HPLC I Chromatography Conditions | |
|---|---|
| Column | TSK-GEL G-Oligo-PW 30 cm x 7.8 mm ID |
| Guard | |
| Column | Guardcolumn Oligo 4 cm x 6.0 mm ID |
| Loop | 200 µl |
| Flow | 0.5 ml/min. |
| Solvent | 0.2 M phosphate buffer (pH = 7.0) |
| Fraction | 0.5 ml/tube |
| Detector Absorption Setting | 190 nm - 400 nm |

The second set of HPLC conditions ("HPLC II") are described in Table III, below, and utilized conditions similar to those described by Rice, K. G. et al. in Analytical Biochem. (1985) 150:325-331. Hence, two columns connected in series were used: a Toyo Soda TSK-Gel G3000SW (7.5 mm x 50 cm) column connected to a G2000SW (7.5 mm x 50 cm) column. These columns, together with a 7.5 mm x 10 cm guard column attached to the inlet end of the G2000 column, were obtained from Phenomenex. Further experimental details are described in Sections 5.10 and 5.11, below.

**Table III.**

| HPLC II Chromatography Conditions | |
|---|---|
| Column | Toyo Soda TSK-GEL G3000SW (50 cm x 7.5 mm ID) and a G2000SW (50 cm x 7.5 mm ID) in series Guard |
| | |
| Column | Guardcolumn (10 cm x 7.5 mm ID) |
| | |
| Loop | 20 or 100 µl |
| Flow | 1 ml/min. |
| Solvent | degassed 0.5 M NaCl |
| Fraction | 0.5 ml/tube |
| Detector Absorption Setting | 205 nm, 232 nm |

Under these conditions, smaller substances are retained longer than larger molecules.

In yet another set of HPLC conditions ("HPLC III"), the purity of selected desalted HPLC fractions was examined with the aid of a strong anion exchange (SAX) HPLC column. Such SAX HPLC columns are known to separate similarly sized molecules according to the number of negatively charged groups which are present in the molecules. The greater the number of negatively charged groups in a substance, the longer it is retained in the column. The HPLC III conditions are outlined in Table IV, below.

**Table IV.**

| HPLC III Chromatography Conditions | |
|---|---|
| Column | SAX-HPLC column (25 cm x 4.6 mm ID, packed with Spherisorb, 5 µm particle size) |
| Loop | 1 ml |
| | |
| Flow | 1.5 ml/min. |
| Solvent | linear gradient, below |
| Fraction | 1 ml/tube |
| Detector Absorption Setting | 205 nm, 232 nm |
| Linear Gradient (*See*, Section 6.15, below) | |

It will also be apparent to one of ordinary skill in the art, after considering the disclosure presented herein, that other HPLC conditions can be contemplated and applied to the separation and purification of the active substances of the present invention. In particular, reverse-phase conditions can also be utilized to good advantage. *See*, for example, Rice, K.G. et al., *supra.*

It also follows that the biological activity of TNF-α may be inhibited by converse processes, including not only competition offered by the active inhibitory substance for the receptors of TNF-α (e.g., the inhibitory substance acting as or inducing the production of another substance that acts as an antagonist of TNF-α) but also the formation of a complex of TNF-α and the inhibitory substance which is less active than free TNF-α.

### 4.3. Determination of Activity

The active substance of the present invention has been isolated and purified from mixtures containing it. In some cases, this active substance has been purified to substantial homogeneity by the powerful HPLC techniques described herein.

As a further indication of the purity of this active substance, its specific inhibition activity was determined.

Initially, however, a carbazole assay, performed in a manner similar to that disclosed by Carney, S. L. in Proteoglycan Analysis, A Practical Approach, Chaplin, M. F. and Kennedy, J. F. (Eds.) IRL Press, Oxford, Washington, D.C. (1986) p. 129, was utilized to determine the amount of oligosaccharide material present (e.g., amount of sugar present) in a given test sample. Picogram (pg) quantities of sugar can be quantified in this manner.

Next, the apparent activity associated with that quantity of substance is determined below, to provide a dose/response profile. These bioassays may either be carried out in vitro or under in vivo conditions.

It has, thus, been found that the observed inhibition of TNF-α activity, expressed as a percentage of the activity of TNF-α observed in the absence of the substance of the present invention, depends on the concentration or dose of such substance present in the test sample. The apparent activity profile that results is approximately bell-shaped as illustrated in Fig. 4. The maximum value of percent inhibition observed for each substance is designated Inhₘₐₓ.

As described further, below, the bioassay used to establish the "ideal" unit dose (i.e., the one that corresponds to Inhₘₐₓ) can be based on the *in vitro* or *in vivo* inhibition of the activity of TNF-α or DTH assay in mice.
Alternatively, an *in vitro* assay based in human cells (described further, below) may also be used. The specific regulatory activity or "R" value is, as defined herein, the ratio of the Inhₘₐₓ and the "ideal" dose that gave rise to that maximum percent inhibition. For the *in vitro* assays, the "R" values are typically expressed in units of % x (pg/ml)⁻¹.

As stated above, the specific regulatory activity can also be established under *in vivo* conditions by monitoring the inhibition of experimental DTH reaction in mice or humans. It was found that the ability of a particular dose of an inhibitory composition to inhibit secretion of active TNF-α is positively correlated with its ability to inhibit the delayed type hypersensitivity (DTH) reaction, although the same composition may be more potent under one assay versus another (i.e., between *in vitro* and *in vivo* bioassays). Inhibitory activity in this *in vivo* cell-mediated inflammatory reaction is of great importance because the DTH reaction is an expression of the processes involved in autoimmune diseases, graft rejection, some types of blood vessel inflammation and allergy. Thus, activity in this test is indicative of utility in these types of diseases and possibly others, as described further below.

Moreover, the new quantity, the specific inhibitory activity, which is defined as the ratio between the Inhₘₐₓ value and the amount or concentration of substance (the "ideal" dose) which gave rise to that maximum percent value, can serve to distinguish the novel active substance of the present invention from those substances that may have been known, but unrecognized in the art as possessing the cytokine inhibitory activity disclosed herein. This specific ratio is referred to herein as the "R" value, for short. Hence, the novel substance or composition of the present invention can be described in terms of a *minimum* "R" value, which can be calculated from the apparent activity versus dose profile, and which "R" value will exceed the "R" value that can be associated, by reference to the teachings of the present disclosure, with known compositions.

### 4.4. Types of Disorders That May Benefit From the Present Invention

The disorders that can be prevented or treated according to the invention are all disorders linked to pathological processes involving induction of active TNF-α secretion, including atherosclerosis and vasculitis and pathological processes related thereto; autoimmune diseases, e.g., rheumatoid arthritis, diabetes type I (insulin-dependent diabetes mellitus or IDDM), multiple sclerosis, lupus erythematosus, Graves disease; allergy; graft rejection; acute and chronic inflammatory diseases, e.g. uveitis, bowel inflammation; anorexia nervosa; hemorrhagic shock caused by septicemia, and HIV infection in AIDS. In AIDS, the active substances will suppress replication of HIV thereby preventing the development of AIDS-related complex (ARC). Other disorders that may benefit from a treatment designed to regulate cytokine activity include, but are not limited to, psoriasis, pemphigus, asthma, renal diseases, liver diseases, bone marrow failure, vitiligo, alopecia, and myositis.

In the clinical application of the active substances of the present invention, it should be kept in mind that the successful treatment of certain types of disease consists, in large part, in the restoration of homeostasis. To the endocrinologist, this implies the judicious administration or antagonism of specific hormones. For example, an insulin-dependent diabetic may be effectively treated by insulin replacement therapy; a patient with Graves' disease may be helped by pharmacological measures that inhibit thyroxine release. Only rarely can disease be alleviated by administration of hormones that were never deficient to begin with.

Clearly, our understanding of the role for TNF-α is still evolving and, doubtless, new and useful uses of the hormone and the substances able to regulate its activity will be uncovered. While it goes without saying that all uses of the claimed compositions and pharmaceutical preparations are within the scope of the present invention, those uses that either alleviate the symptoms of disease, prevent the onset of disease, or provide a cure for the disease are especially contemplated.

### 4.5. Topical Applications of the Substance of the Present Invention

The substance of the present invention also find use in topically administered compositions, such as those preparations for the treatment of edema or inflammation. Indeed, above and beyond a purely therapeutic application, the substance of the present invention may also find utility in supplementing the protective action of cosmetic compositions, such as sunscreen or suntan lotions. Few, if any, sunscreen preparations are fully effective in blocking out all the harmful wavelengths (e.g., 290-320 nm) present in the ultraviolet region of the electromagnetic spectrum. Hence, overexposure to the sun often gives rise to an acute condition known as solar erythema and prolonged, repeated exposure can, of course, lead to leathery looking skin or, worse, skin cancer.

Thus, the incorporation of the active substance of the present invention in cosmetic preparations is specifically contemplated both for the purpose of preserving and protecting the skin, as well as alleviating a medical condition, such as solar erythema. In sunscreen or suntan preparations, it would be advantageous to include an effective amount of the oligosaccharide of the present invention along with conventional sunscreen agents. Generally, an amount of active substance would be present to provide a dose of about 1 µg to about 100 mg per kilogram of subject, preferably from about 0.01 mg to about 10 mg per kilogram of subject, and most preferably about 0.1 mg to about 1 mg per kilogram of subject.

The cosmetic compositions, may contain conventional ingredients known to those of ordinary skill in the art, such as those described in Kirk-Othmer, Encyclopedia of Chemical Technology, Third Edition (1979), Vol. 7, pp. 143-176. In sunscreen preparations, the addition of the active substances of the present invention increases the minimum erythemal dose (MED) and, consequently, the sun protection factor (SPF). Specific ingredients, including typical sunscreens, are listed in Kirk-Othmer, *supra,* at pp. 153-154. In addition, topical preparations and cosmetic formulations may be prepared as described in U. S. Patent Nos. 4,199,576, 4,136,165, and 4,248,861, the complete disclosures of which are incorporated by reference herein. It would, of course, be apparent to those of ordinary skill in the art of cosmetology that the resulting compositions can be in many forms, including, but not limited to, solutions, lotions, cremes, pastes, emulsions, sprays, or aerosols.

### 4.6. Exemplary Dosage Regimens

For the substance of the present invention, the following is a way to predict the effective dose of substance for treatment of humans from the *in vivo* DTH bioassay.

An isolated fraction (F15) *in vivo* inhibits the DTH in mice at a range of 0.1-5.0 µg/mouse/week. Since our mice weigh 25 gm, the *in vivo* dose is approximately (0.1 ö 0.025 kg) 4-200 µg/kg mouse/week (the equivalent of 0.01-10 pg/ml *in vitro*).

To correct for the surface area difference between mice and humans, we have to divide the mouse dose/kg by 12:
4-200 µg/kg mouse - 0.33-16.67 µg/kg human. Thus, a 70 kg human should receive up to about 1.2 mg (about 1,200 µg). To be certain that we could cover the difference between people, we might increase this dose to about 5 mg, an amount that is well below any doses of heparinoids used for their effects on coagulation or thrombosis. Hence, the dose for a 70 kg human, will be about 5 mg or less, preferably about 3 mg or less, more preferably about 1.5 mg or less, and most preferably about 1 mg or less.

In fact for the highly purified materials of the present invention, including those that have been obtained from HPLC chromatography, the preferred dosages may be even less. For example, the disaccharide, described in greater detail below, has been found to exhibit inhibitory activity, when administered by injection, at about 0.1 µg to about 0.5 µg per kilogram mouse. Hence, the dosage for humans are estimated to be about 0.01 µg to about 0.05 µg per kilogram man or about 0.7 µg to about 3.5 µg for a 70 kilogram man for the purified disaccharide. A general range of dosage for a 70 kg man, then, may be about 0.1 µg to about 100 µg, preferably about 1 µg to about 10 µg for the disaccharide. The dosages may be somewhat higher for the known disaccharide "markers," discussed further below.

The doses recited above may be given a number of times a day or at daily, weekly or even greater intervals depending on the responsiveness of the individual.

The invention will now be illustrated by the following non-limiting examples.

### 5. Experiments Using LMWH (Fragmin) And Other Active Substances

### 5.1. Preparation of ECM-Coated Plates

ECM-coated wells were prepared as follows. Freshly dissected bovine eyes were obtained from a slaughter house within a few hours after slaughter. The eyes were dissected in a hood to remove the cornea. The cornea were then scratched or scraped with a scalpel to obtain the corneal endothelial cells. These cells were cultured on tissue culture plates with approximately 5 ml of media comprising DMEM supplemented with 10% fetal calf serum, 5% calf serum and antibiotics, such as 1% streptomycin or 1% neostatin, together with 1% glutamine as a stabilizer. The cells settled to the bottom of the plates after approximately 2 days of seeding, were fed with fresh media every four days, and incubated at 37 °C in 5% CO₂ humidified incubators. If desired, some fibroblast growth factor may also be added to the media, although the addition of FGF is not crucial. When the cells were confluent (approximately 2 weeks later), the supernatant was aspirated off, and the cells were then trypsinized with 1-2 mls of trypsin.

Eighty percent of these primary cells (the fate of the remaining 20% of the primary cells is described immediately below) were taken and divided onto 5 flat-bottomed 96-well plates. The cells were cultured in DMEM supplemented with 4% dextran T-40, 10% fetal calf serum and 5% calf serum. After about 7 days of incubation at 37 °C in a 10% CO₂ humidified incubator, the resulting confluent layers of endothelial cells were lysed. The lysing buffer, comprising 0.025 M NH₄OH containing 0.25% Triton X in PBS, was allowed to remain over the cells for 10 minutes and then decanted. The contents of the plates were then washed three times with PBS chilled to 4 °C. The preceding procedure left the ECM intact, firmly attached to the entire area of the well. The resulting ECM was also free of nuclei and cellular debris. The ECM-coated plates can be stored at 4 °C for at least three months.

The remaining 20% of the primary cells were left on a single plate and cultured in approximately 5 ml of media comprising DMEM supplemented with 10% fetal calf serum, 5% calf serum and antibiotics as described above. This secondary crop of cells was allowed to become confluent and was treated with trypsin as described above. Again, the trypsinized cells were divided, 80% being cultured in 5 plates in the growth media containing 4% Dextran T-40, and 20% being cultured in a single plate as before. It is possible to perform this 80/20 division yet one more time from this single plate.

### 5.2. Degradation of Sulfated Proteoglycans

³⁵(S)O₄-labelled ECM was incubated with 5 *µ*l of MM5 heparanase (4 u/ml) in 1 ml PBS and 100 µl 8.2 M phosphate-citrate buffer (pH 6.2) for 48 hrs. at 37 °C. The medium was then collected, centrifuged at 10,000 g for 5 min. (optional) and analyzed by gel filtration on Sepharose 4B columns. Two ml fractions were eluted with PBS at a flow rate of 5 ml/hr and were counted for radioactivity using Bio-Fluor Scintillation fluid.

This ³⁵(S)O₄-labelling experiment showed that the ECM was actually being degraded, that the resulting degradation products were successfully being released, and, furthermore, were being properly filtered through the Sepharose 4B columns. Subsequent experiments related to the degradation of sulfated proteoglycans were carried out on non-labeled ECM, with the degradation products being monitored by their absorption at 206 or 232 nm, instead.

Enzyme degradation experiments were carried out as above and, in addition, the degradation products (DECM) were purified further by loading the degraded proteoglycans that were eluted from the Sepharose columns onto HPLC columns. HPLC analysis of the Sepharose column fractions was carried out. Two experiments were performed utilizing two sets of high performance liquid chromatography conditions. The initial type of column used was a 30 cm x 7.8 mm I.D. TSK-Gel® G-Oligo-PW column with a 4 cm x 6 mm I.D. guard column. The column was eluted with 0.2 M phosphate buffer, pH 7.0, at a flow of 0.5 ml/min. The fractions collected were each 0.5 mls in volume.

The second type of HPLC used was Toyo Sada TSL-Gel G3000SW (7.5 mm x 50 cm) and G2000SW (7.5 mm x 50 cm) columns (in series) with a 7.5 mm x 10 cm guard column from Phenomenex. The column was eluted at 1 ml/min. with carefully degassed 0.5 M NaCl. Fractions were collected at 0.5 ml per fraction. The detector was set at 232 nm with 0.02 AUFS and retention times measured to ± 0.1 sec. The void and total volumes were measured by blue dextran and sodium azide. The collections were also subjected to detection at 206 nm under the same conditions as the 232 nm setting. Detection of the degradation products was achieved by monitoring their absorption at 206 nm.

Additional enzyme degradation experiments were carried out with similar results using PC3 enzyme and heparanase obtained from IBEX.

### 5.3. Purification of Human CD4⁺ T Cells

CD4⁺ T cells were obtained from peripheral blood mononuclear leukocytes obtained from healthy human donors as follows. The mononuclear cells were isolated on a Ficoll gradient, washed in RPMI supplemented with 10% FCS and antibiotics in petri dishes and incubated at 37 °C in a 10% CO₂ humidified atmosphere. After 1 h, the non adherent cells were removed and incubated on nylon-wool columns (Fenwall, IL) for 45-60 min at 37 °C in a 10% CO₂ humidified atmosphere. Non adherent cells were eluted and washed. CD4⁺ T cells were negatively selected by exposure of the eluted cells to a mixture of the following monoclonal antibodies (mAb): anti-CD8, CD19, and CD14 conjugated to magnetic-beads (Advanced Magnetics, Cambridge, MA). Unbound cells were recovered and their phenotypes were examined. The resultant purified cells were predominantly (>90%) CD3⁺CD4⁺ as determined by FACScan analysis.

### 5.4. Bioassay of TNF-α Activity Using Human CD4⁺ T Cells Derived from PBLs.

Two hundred fifty thousand human CD4⁺ T cells were preincubated with 150 µl of ECM degradation products at various concentrations for 1.5 h at 37 °C, under a 7% CO₂ atmosphere. Then 100 µl of PHA (Wellcome Co., England, 1 µg/ml) were added for 3 h incubation, in flat-bottomed 96-well plates (Costar). Subsequently, the contents of the wells (3-6 wells per experimental group) were collected, centrifuged, and the media were assayed for TNF-α secretion. The TNF-α bioassay is based on the cytotoxic effect of TNF-α cycloheximide (CHI)-sensitized cells and its quantitation by the neutral red uptake assay as described by Wallach D., J. Immunol. (1984). 132:2464-2469. Briefly, the killing of CHI-sentitized HeLa cells by TNF-α present in the supernatants of the cells is measured, the concentration of TNF-α in the supernatants being determined by comparison to titration curves of TNF-α exogenously added. Cell viability is determined by incubation with neutral red for two hours, washing away excess dye, extracting the neutral red that was taken up by the cells with Sorenson's citrate buffer-ethanol mixture, and quantitating it calorimetrically at 570 nm with a Microelisa Autoreader. Briefly, supernatants of cultured lymphocytes were added to cultures of mouse fibrosarcoma cell clones (BALB/c.CL7). BALB/c.CL7 cells are sensitive to killing by TNF-α in the presence of actinomycin D (0.75 µg/ml). Nophar, Y. et al. J. Immunol. (1988) 140(10):3456-3460. The death of these cells, in the presence of the test media, was calibrated in comparison to titration curves of added exogenous TNF. Cell viability is determined by incubation with MTT tetrazolium (Sigma, Cat. No. M2128) for two hours, extracting the dye that was taken up by the cells with isopropanol-HCl mixture and quantitating it calorimetrically (at 570 nm) with a Microelisa Autoreader. TNF-α typing was done by examining the neutralizing effect of anti-murine TNF-α mAb (diluted 1/400; Genzyme, MA).

### 5.5. Degradation of Heparin

One milligram of heparin (Sigma) in 1 ml of PBS and 100 µl 25 M phosphate-citrate buffer (pH 6.2) was incubated with 20 µl MM5 (5 u/ml) for 48 hrs. at 37 °C. The products of the reaction were then analyzed by gel filtration on Sepharose 4B columns. Two ml fractions were eluted with PBS at a flow rate of 5 ml/hr. To further characterize the degradation products, the peaks eluted from the Sepharose column were subjected to HPLC separation using Toyo Soda-Gel G3000SW and G2000SW HPLC columns, as described in Section 5.2.

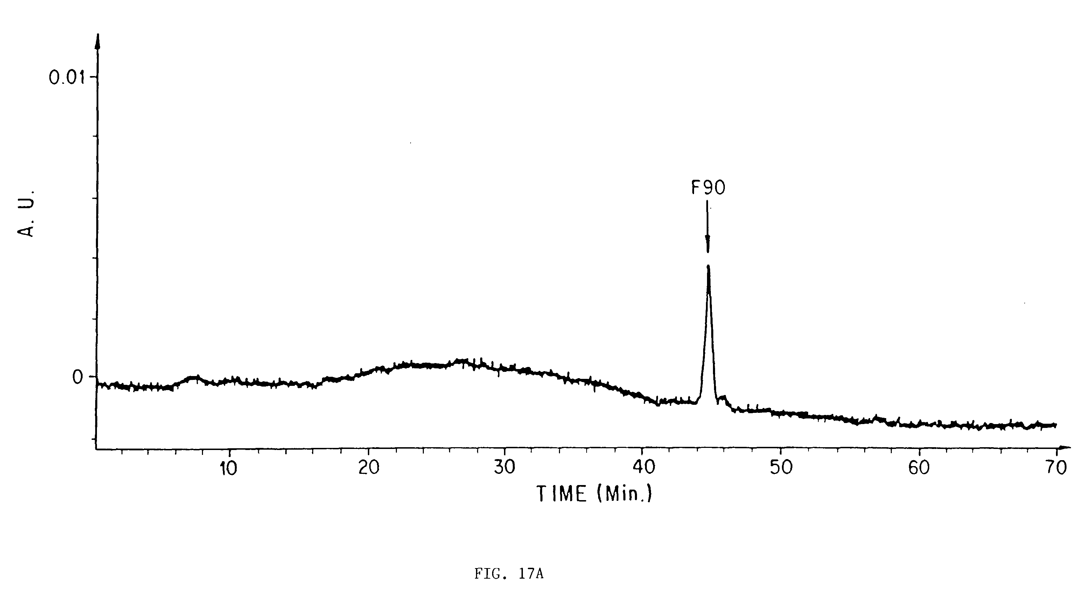

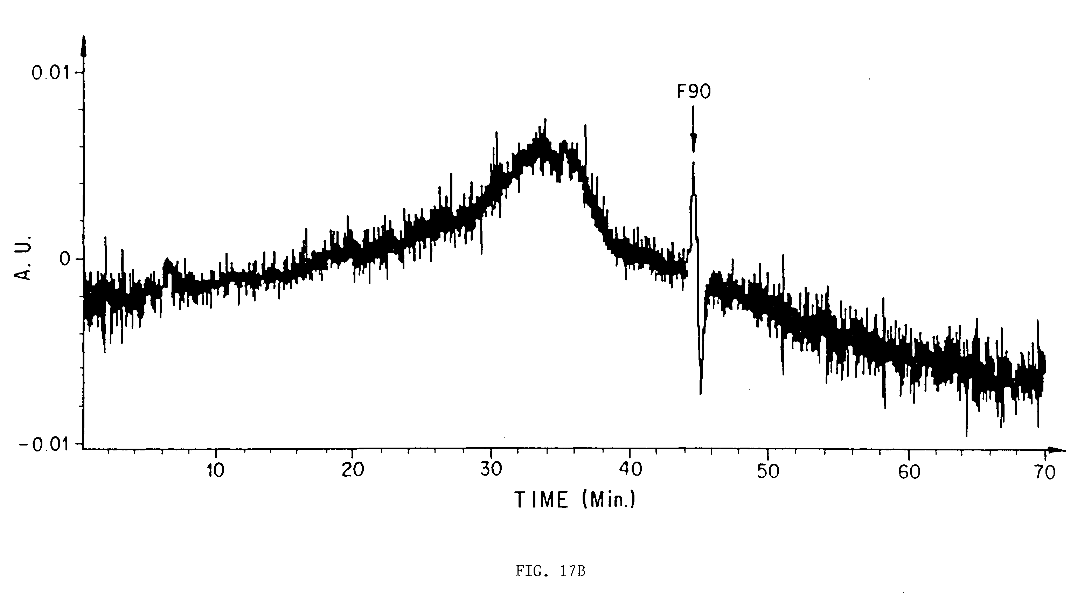

Additional experiments were carried out using 20 µl of PC3. The PC3 enzymatic reaction was carried out with 1 mg of heparin under the same conditions as described above for the MM5 except that the reaction was incubated for 24 hrs instead of 48 hrs. The products were then analyzed by gel filtration on Sepharose 4B columns (Fig. 17). The *in vitro* bioassay results are shown in Table VIII below.

### 5.6. Elicitation of DTH Response in Mice and Examining Inhibitory Effects

BALB/c mice (at least 5 mice per group) were sensitized on the shaved abdomen with 3% 4-ethoxymethylene-2-phenyl oxazolone (OX; BDH Chemicals, GB) in acetone/olive oil applied topically. DTH sensitivity was elicited 5 days later as follows. Mice were challenged with 0.5% OX in acetone/olive oil. The ear was measured immediately before challenge and 24 h later with Mitutoyo engineer's micrometer (Japan). The individual measuring the swelling of the ear was blinded to the identity of the groups of mice. To interfere with DTH response, the low molecular weight immuno-regulatory fractions, diluted in PBS, were administrated subcutaneously into the back of the treated mice at the indicated time schedules and concentrations. Treated mice were inspected during and after (>2 months) the treatment and no major side-effects were observed clinically.

### 5.7. Additional Experiments Involving the Fractionation of Fragmin and Heparanase-Degraded ECM

Fragmin and heparanase-degraded ECM were fractionated by gel filtration on Sepharose 4B columns. Fractions of 0.2 ml were eluted with PBS at a flow rate of 5 ml/hr, and monitored for absorbance at 206 nm. (No absorbance was detected at 280 nm). A plot of the fraction number versus absorption at 206 n.m. appears on Fig. 2. The results of the bioassays for selected fractions are presented in Table V below.

**Table V.**

| Effect of Sepharose 4B Fractions of Fragmin and DECM on the Secretion of Active TNF Using Human PBL Bioassay. | | | |
|---|---|---|---|
| Test Fraction | conc (pg/ml) | Bioassay of | |
| | | TNF Activity (%) | "R" value % x (pg/ml)⁻¹ |
| Frag/Seph.4B-F39 | 100 | Inhₘₐₓ (60%) | 0.6 |
| DEMC/Seph.4B-F39 | 10,000 | Inhₘₐₓ (85%) | 0.0085 |
| Frag/Seph.4B-F42 | a | No effect | 0 |
| DECM/Seph.4B-F42 | a | No effect | 0 |

| | | | |
|---|---|---|---|
| ^{a} At a conc range of 1 µg/ml - 0.001 pg/ml. | | | |

### 5.8. Separation of Active Substances From ECM Using High Performance Liquid Chromatography

Separation of active substances from ECM by high performance liquid chromatography was carried out as discussed in section 5.2.

A plot of the ECM HPLC fraction number versus absorption at 206 nm appears on Fig. 6B. The results of the bioassays for selected fractions are presented in Table VI, below. It is evident from the results presented that certain substances isolated from heparanase-mediated degradation of ECM are able to inhibit the activity of TNF-α while others are able to augment its activity. The results also show that certain fractions obtained from the HPLC separation exhibit no effect on the activity of TNF.

**Table VI.**

| Effect of DECM HPLC Fractions on the Secretion of Active TNF Using Human PBL Bioassay. | | | |
|---|---|---|---|
| DECM Fraction | conc (pg/ml) | Bioassay of | |
| | | TNF Activity (%) | "R" value % x (pg/ml)⁻¹ |
| HPLC-F1 | a | No Effect | 0 |
| HPLC-F5 | a | No Effect | 0 |
| HPLC-F10 | 10 | Inhₘₐₓ (60%) | 6 |
| HPLC-F14 | 10 | Inhₘₐₓ (70%) | 7 |
| HPLC-F17 | a | No Effect | 0 |
| HPLC-F25 | 1000 | Augₘₐₓ (40%) | 0.04 |
| HPLC-F33 | 100 | Augₘₐₓ (40%) | 0.4 |
| HPLC-F37 | 10 | Augₘₐₓ (100%) | 10 |
| HPLC-F39 | 100,000 | Inhₘₐₓ (60%) | 0.0006 |
| HPLC-F42 | a | No Effect | 0 |
| HPLC-F46 | 1000 | Augₘₐₓ (30%) | 0.03 |
| HPLC-F49 | 1000 | Augₘₐₓ (30%) | 0.03 |
| HPLC-F61 | a | No Effect | 0 |

| | | | |
|---|---|---|---|
| ^{a} At a conc range of 1 µg/ml - 0.001 pg/ml. | | | |

### 5.9. Carbazole Quantitative Sugar Assay

Briefly, 1500 µl of borate sulphuric acid reagent is cooled on an ice bath. The test solution (250 µl containing 20 µg of uronic acid/ml) is then carefully layered onto the surface of the boric acid reagent and allowed to diffuse for 10 minutes. The solutions are then thoroughly mixed, put in a boiling bath for 10 minutes, and then cooled in an ice bath. Chilled carbazole (50 µl) is then added to the mixture, vortexed, and put in a boiling bath for 15 minutes. The solution is then cooled and the absorbance is read at 525 nm. The results are compared with calibrated solutions.

### 5.10. Isolation of a Disaccharide from ECM Degradation Products

A disaccharide substance was isolated by HPLC from bovine corneal endothelial ECM that had been subjected to mammalian heparanase (MM5).

More specifically:

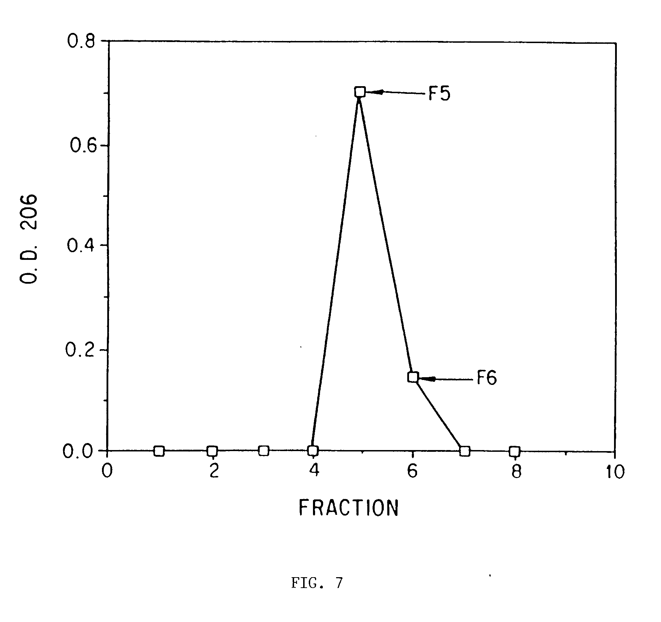

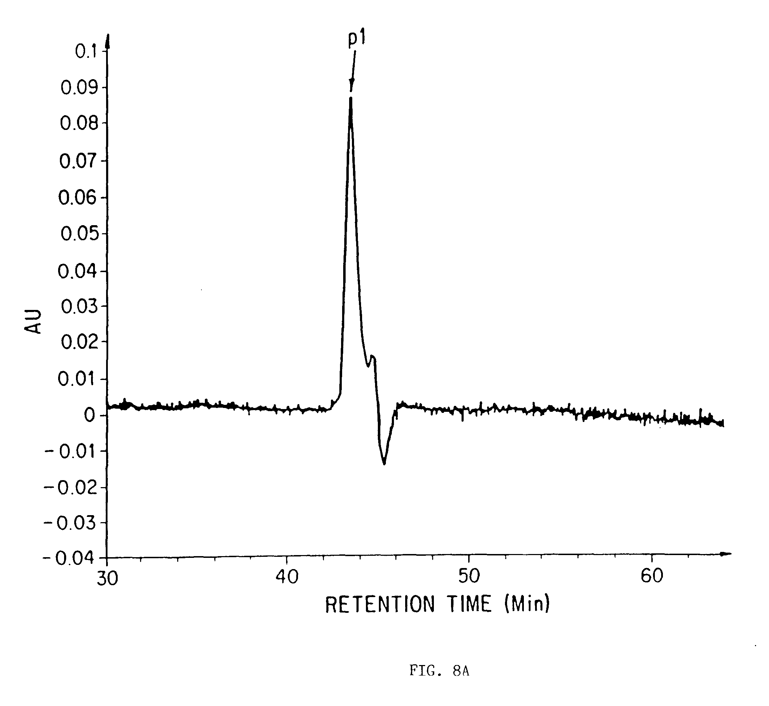

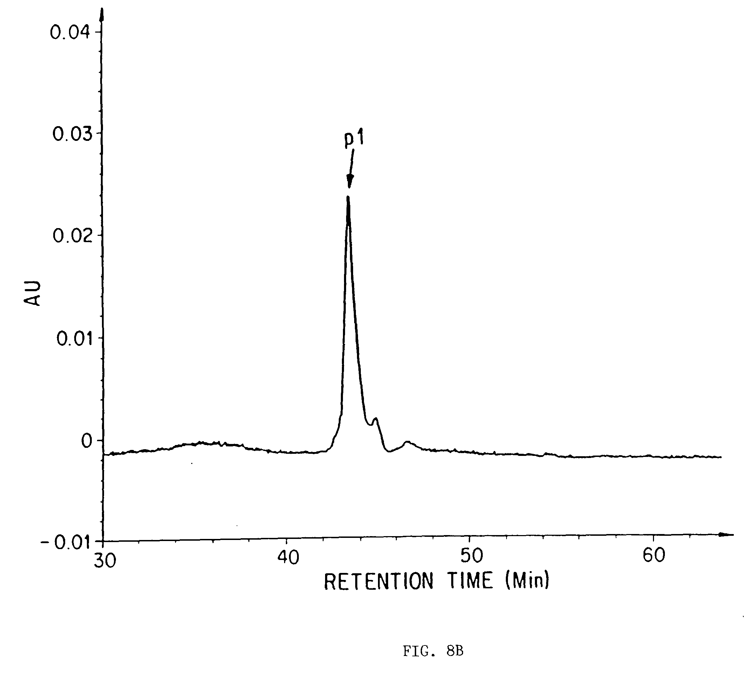

An ECM-coated plate was incubated with 20 µl of mammalian heparanase (0.5 mg/ml) in 1 ml PBS buffer (that was preadjusted to pH 6.2 by citric acid) for 48 hours at 37 °C. The medium was then collected and applied on a Sepharose-4B column (35 cm x 0.7 cm I.D.). The mobile phase was PBS buffer at a flow rate of 5 ml/hr. Fractions of 2.2 ml were collected and monitored at 206 nm (Fig. 7). A sample (100 ul) from Sepharose 4B fraction no. 5 (8.8-11 ml elution volume) were injected into an HPLC column (Toyo Soda TSK-Gel G3000SW (7.5 mm x 50 cm) and G2000SW (7.5 mm x 50 cm), in series with 7.5 mm x 10 cm guard from Phenomenex). The mobile phase was 0.5 M NaCl at a flow rate of 1 ml/min. One ml fractions were collected and monitored at 206 and 232 nm (Fig. 8A and 8B). Peak no 1. (P1) was freeze dried in a 25 ml flask. The sample contains ca. 20 µg of oligosaccharide (determined by carbazole assay) in 60 mg NaCl.

The sample has an elution profile that is similar to a disaccharide standard or molecular weight "marker" obtained commercially from the depolymerization of heparin (Sigma).

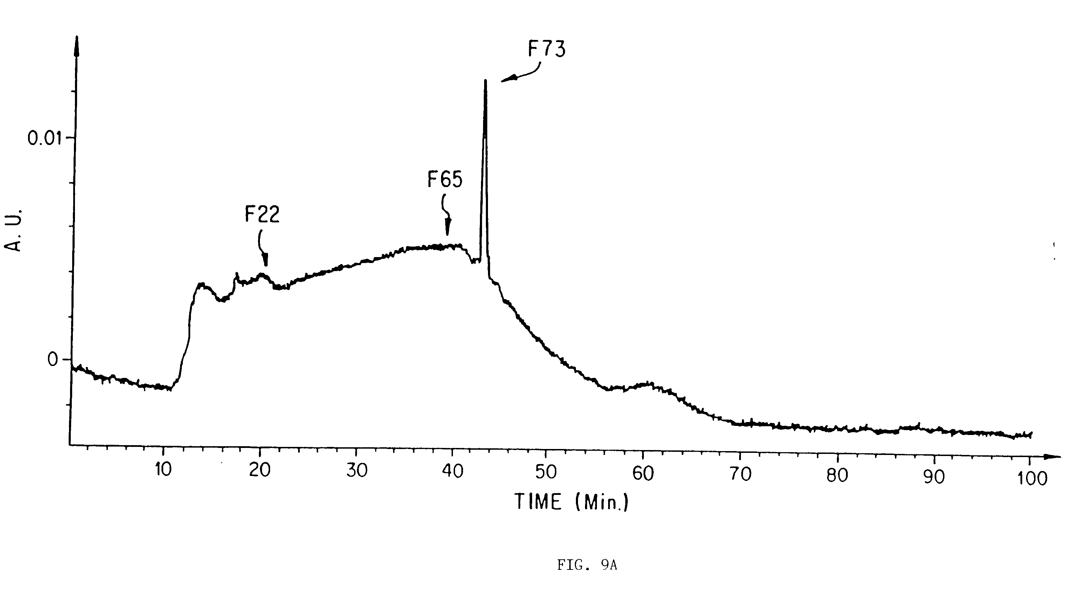

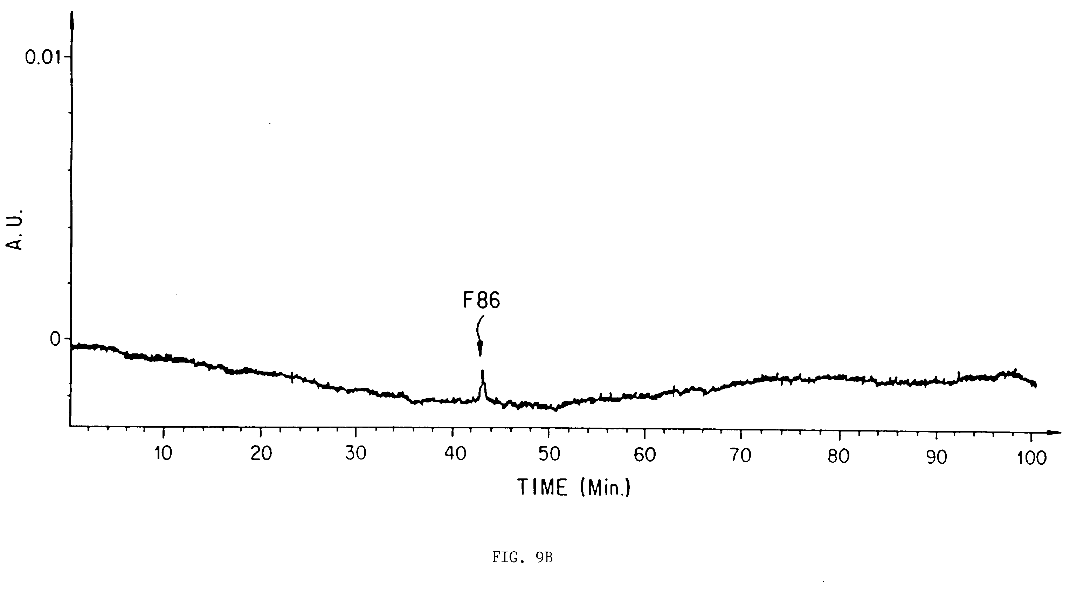

The Inhₘₐₓ value of the substance, based on similarly obtained samples (*See*, for example peak F73 in Fig. 9A (elution time of about 44 minutes) and entry F5/HPLC-F73 in Table VII, below), was estimated at 87% in an *in vitro* TNF-α inhibition assay using human PBLs, at a concentration of about 10 pg/ml. The bioassay was conducted as previously described, above. This sample may be purified further using a SAX-HPLC column, as described below.

### 5.11. Isolation of a Disaccharide from ECM Degradation Products Including SAX-HPLC Chromatography

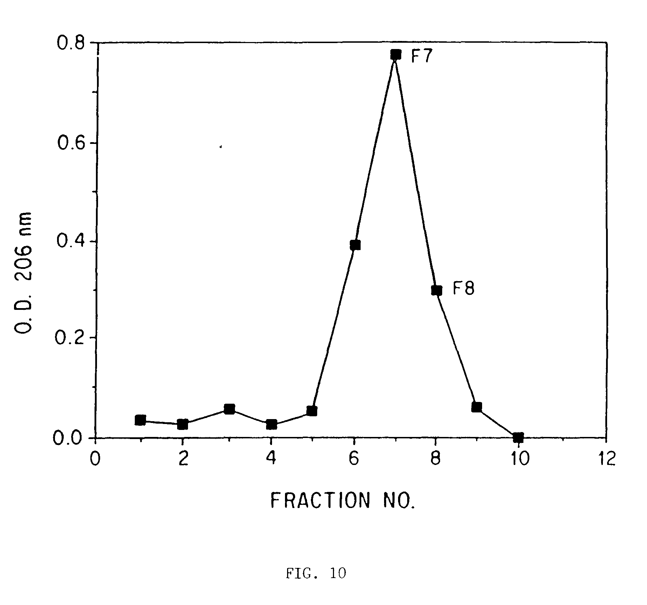

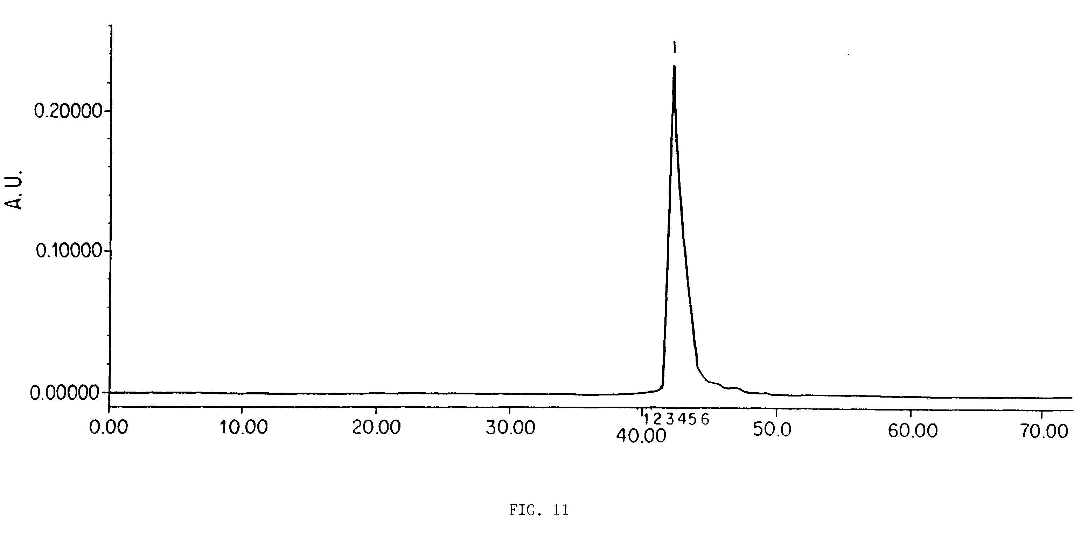

An ECM-coated plate was incubated with 20 *µ*l of mammalian heparanase (0.5 mg/ml) in 1 ml PBS buffer (which had been preadjusted to pH 6.2 by citric acid) for 48 hours at 37 °C. The medium was then collected and applied on a Sepharose-4B column (0.7 x 35 cm). The mobile phase was PBS buffer at a flow rate of 5 ml/hr. Fractions of 1.6 ml were collected and monitored at 206 nm. (Fig. 10). Fractions nos. 7-8 were combined and freeze-dried; the powder was resuspended in 1/10 of the initial volume. Samples (100 µl) were injected into an HPLC column (Toyo Soda TSWK-Gel G3000SW 7.5 mm x 50 cm and G2000SW 7.5 mm x 50 cm, in series with 7.5 mm x 10 cm guard from Phenomenex), as before. The mobile phase was 0.5 M NaCl at a flow rate of 1 ml/min. One ml fractions were collected and monitored at 206 and 232 nm (Fig. 11). The peak labeled "1" was collected from ten identical runs. The substantially homogeneous fractions were combined and freeze dried.

The material was resuspended in 2 ml double deionized water and desalted on a Sephadex G-10 column (26 x 150 mm) eluted at 1.6 ml/min with double deionized (DD) water. One ml fractions were collected and monitored at 232 nm and for conductivity (to determine NaCl content). Desalted fractions were combined, freeze dried and resuspended in 1 ml DD H₂O. A 1 ml sample, prepared by combining 100 µl of the resuspended solution and 900 µl of 0.2 M NaCl at pH 3.5, was injected into an analytical SAX-HPLC column (4.6 x 250 mm, packed with Spherisorb, 5-µm particle size). The flow rate was 1.5 ml/min and an NaCl linear gradient program was employed as follows:

| Time/Buffer (min) | A (%) | B(%) | C(%) |
|---|---|---|---|
| 0 | 100 | 0 | 0 |
| 2 | 100 | 0 | 0 |
| 35 | 38 | 62 | 0 |
| 40 | 38 | 62 | 0 |
| 45 | 0 | 100 | 0 |
| 47 | 0 | 100 | 0 |
| 50 | 0 | 0 | 100 |
| 55 | 0 | 0 | 100 |
| 58 | 100 | 0 | 0 |
| 60 | 100 | 0 | 0 |
| A = 0.2 M NaCl, pH 3.5 | | | |
| B = 1.5 M NaCl, pH 3.5 | | | |
| C = H₂O | | | |

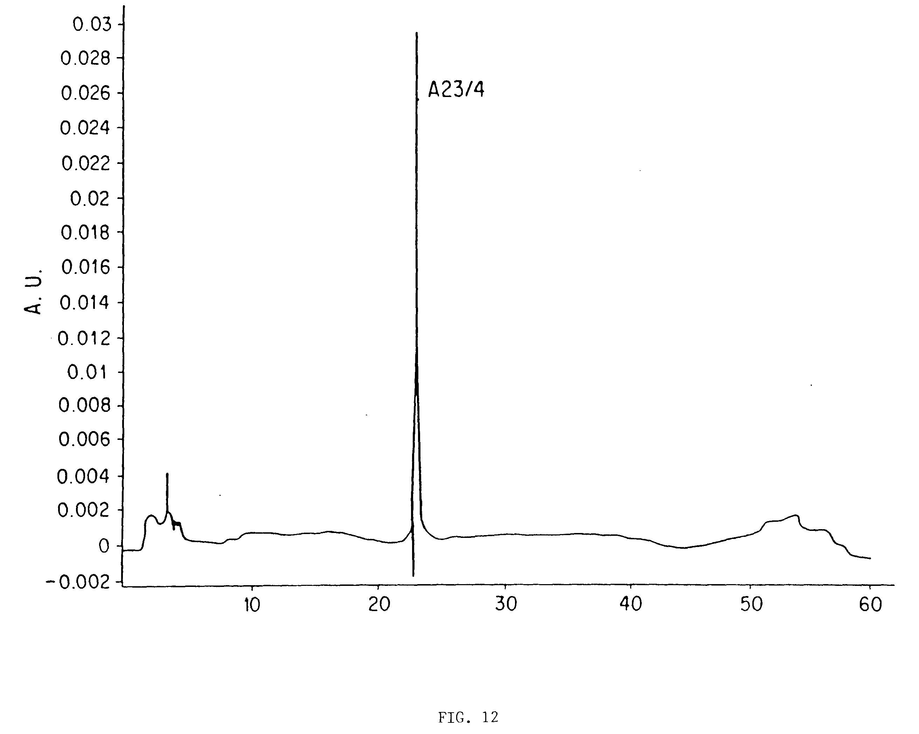

The column eluent was monitored at 232 nm (Fig. 12) and peak A23/4 was collected and tested for TNF inhibition. The Inhₘₐₓ for this SAX-HPLC fraction was found to be 60% at a concentration of 0.1 pg/ml, giving an "R" value of 600 % x (pg/ml)⁻¹.

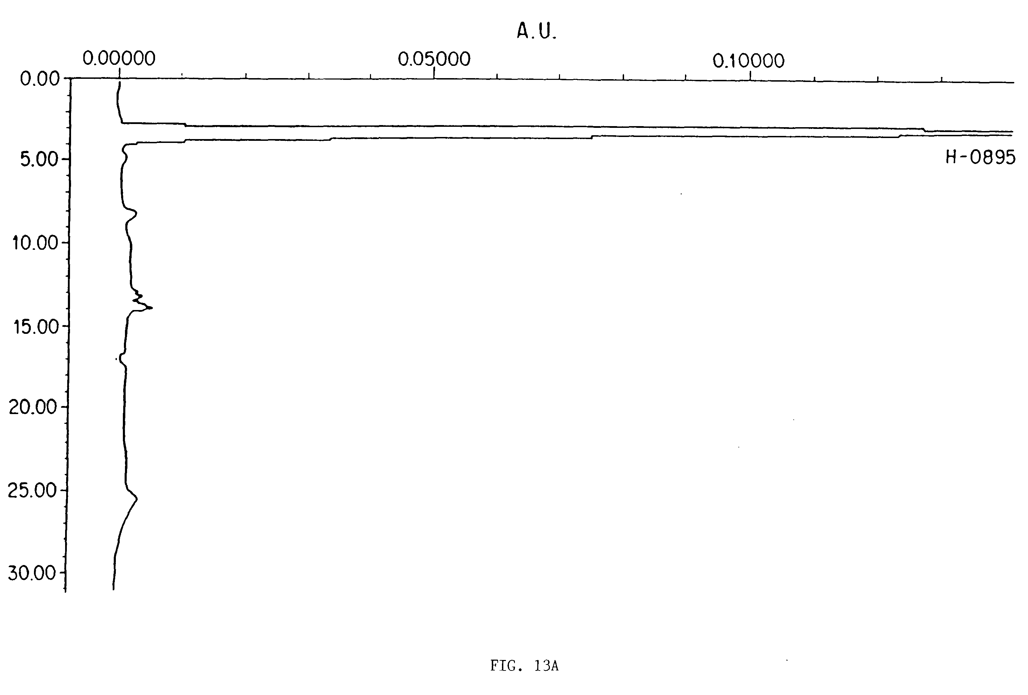

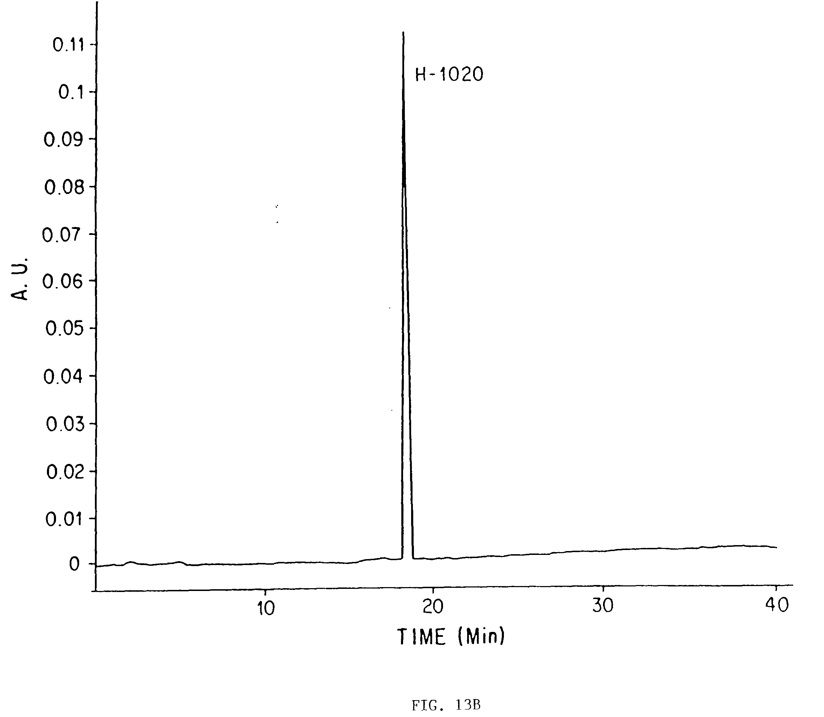

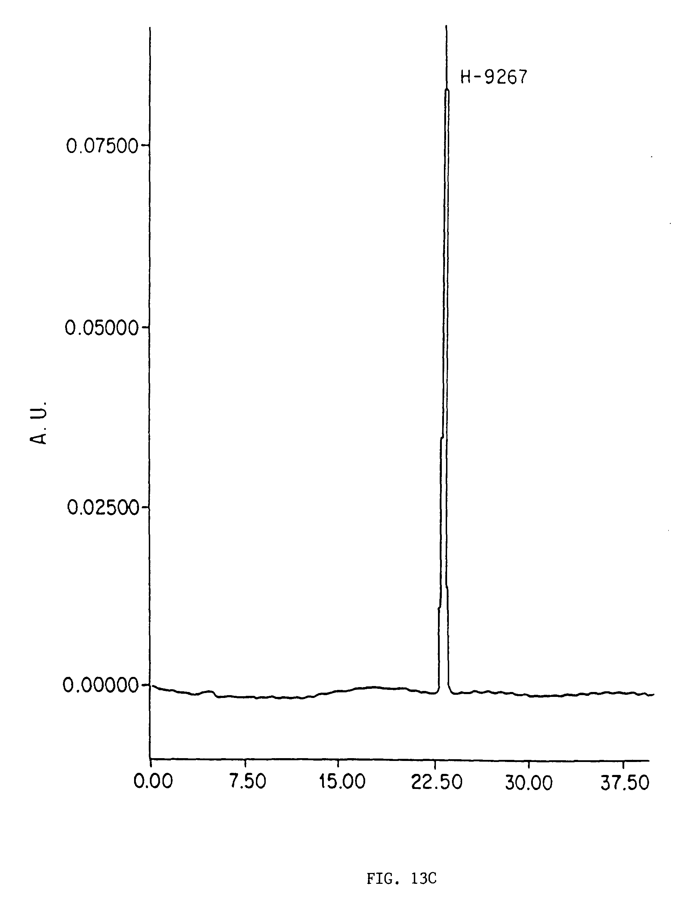

Heparin disaccharide standards with different levels of sulfation were injected into the SAX-HPLC column under identical conditions. The elution profiles of these standards are presented in Figs. 13A-C. As can be seen from these Figures, the disaccharide standards gave different retention times, with an unsulfated disaccharide (Sigma Product No. H-0895) eluting fastest (Fig. 13A), a disulfated disaccharide (Sigma Product No. H-1020) eluting at less than 20 minutes (Fig. 13B), and a trisulfated disaccharide (Sigma Product No. 9267) eluting last (Fig. 13C). The trisulfated disaccharide standard H-9267 provided a retention time that was very similar to that obtained for peak A23/4 (i.e., 23.07 min. vs and 23.10 min., respectively).

### 5.12. Results of In Vitro Human PBL Bioassays for Various Substances

The results of *in vitro* bioassays using human PBLs for various active substances and starting "mixtures" are presented in Table VII for the products obtained from the degradation of ECM, including a peak "P1" from Fig. 8. Whereas, P1 provided an "R" value of 10 % x (pg/ml)⁻¹, the starting DECM "soup" gave an "R" value of 0.000053 % x (pg/ml)⁻¹.

**Table VII.**

| Effect of ECM + MM5 Heparanase (DECM "Soup"), Sepharose 4B Fractions of "Soup", and HPLC Fractions of Sepharose 4B Fractions on the Secretion of Active TNF Using Human PBL Bioassay. | | | |
|---|---|---|---|
| Test Material | conc (pg/ml) | Bioassay of | |
| | | TNF Activity (%) | "R" value % x (pg/ml)⁻¹ |
| DECM "Soup" | 1 x 10⁶ | Inhₘₐₓ (53%) | 5.3 x 10⁻⁵ |
| Seph.4B-F5 | 100 | Inhₘₐₓ (50%) | 0.5 |
| Seph.4B-F6 | 100 | Inhₘₐₓ (60%) | 0.6 |
| Seph.4B-F7,8 | 100 | Inhₘₐₓ (81%) | 0.8 |
| F5/HPLC-F73 | 10 | Inhₘₐₓ (87%) | 8.7 |
| F5/HPLC-F65 | 10 | Inhₘₐₓ (78%) | 7.8 |
| F5/HPLC-F22 | 10 | Inhₘₐₓ (33%) | 3.3 |
| F6/HPLC-F86 | 10 | Inhₘₐₓ (43%) | 4.3 |
| P1 | 10 | Inhₘₐₓ (100%) | 10.0 |

### 5.13. Isolation of Oligosaccharides from Heparin Degradation Products

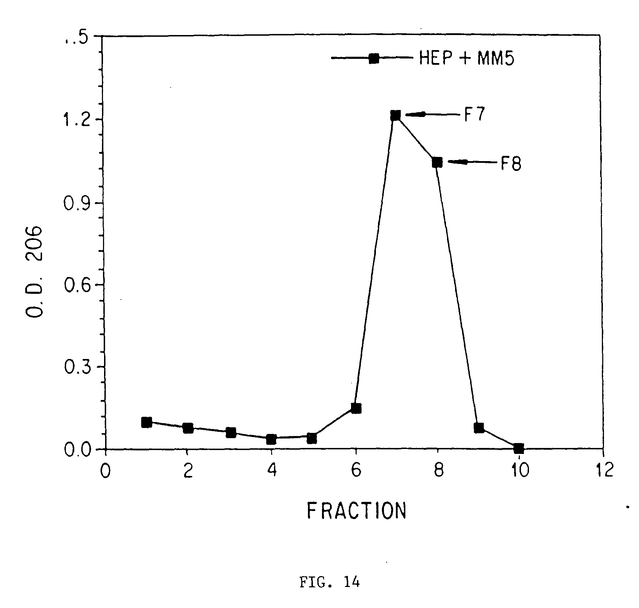

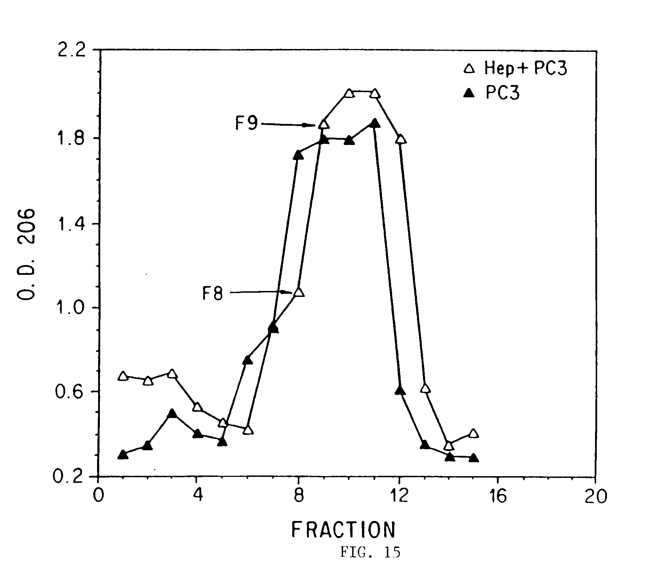

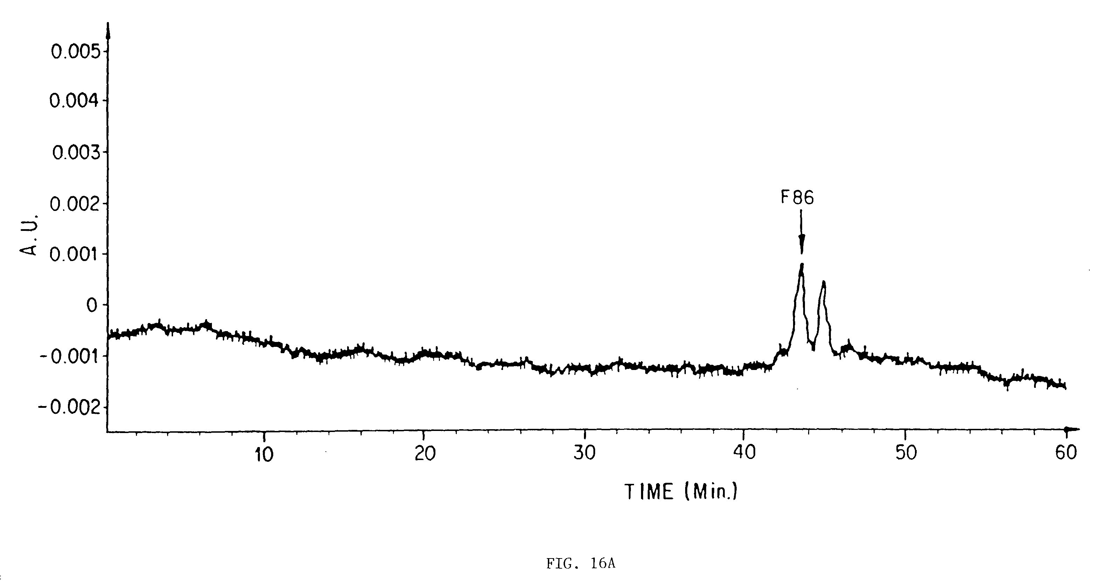

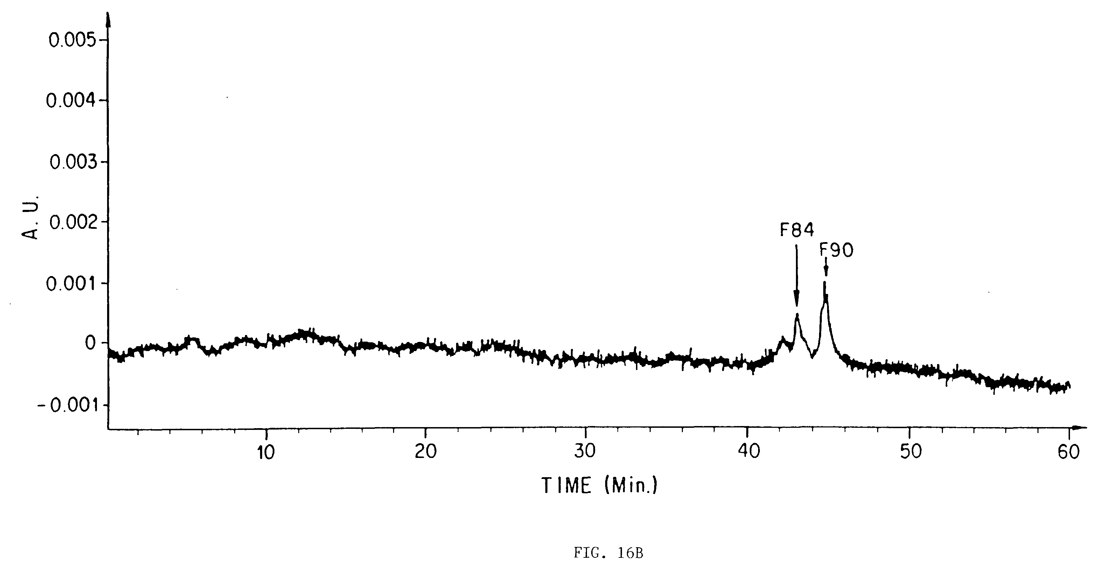

In a manner similar to that described, above, for the degradation of ECM, intact heparin was treated with heparanase enzymes obtained from various sources, designated herein as MM5 and PC3 (*See*, Shoseyov, O. et al., Biochem., Biophys. RES. COMM. (1990) 169:667-672, for the preparation of PC3 enzyme). Some of the starting degradation mixtures were separated on Sepharose 4B (*See*, Fig. 14 for Heparin + MM5 Sepharose 4B fractions F7 and F8, and Fig. 15 for Sepharose 4B chromatography of Heparin + PC3 and PC3 alone). Still other fractions were further separated by the HPLC II methods described above (*See,* Fig. 16A and 16B for fractions F7/HPLC-F86, -F84, and -F90). "Intact" heparin and Fragmin were also subjected to direct HPLC II conditions and selected fractions were likewise isolated (HPLC-F90 from Fragmin is presented in Fig. 17). The results of *in vitro* bioassays using human PBLs for various active substances and starting "mixtures" are presented in Table VIII, including the products obtained from the degradation of heparin.

**Table VIII.**

| Effect of Intact Heparin, Heparin + MM5 or PC3 "Soups", and Selected Sepharose 4B and HPLC Fractions of Same on the Secretion of Active TNF Using Human PBL Bioassay. | | | |
|---|---|---|---|
| Test Material | conc (pg/ml) | Bioassay of | |
| | | TNF Activity (%) | "R" value % x (pg/ml)⁻¹ |
| Intact Heparin | a | No effect | 0 |
| Hep/HPLC-F90 | a | No effect | 0 |
| | | | |
| Additional Heparin | | | |
| Fractions | | | |
| F7/HPLC-F86 | 0.1 | Inhₘₐₓ (26%) | 260 |
| F8/HPLC-F84 | a | No effect | 0 |
| F8/HPLC-F90 | a | No effect | 0 |
| | | | |
| Hep/PC3 "Soup" | 0.1-10 x 10⁶ | No effect | 0 |
| Seph.4B-F9 | 100 | Inhₘₐₓ (50%) | 0.5 |
| Seph.4B-F8 | 100 | Inhₘₐₓ (40%) | 0.4 |
| | | | |
| PC3 only | a | No effect | 0 |
| Seph.4B-F8 | a | No effect | 0 |
| Seph.4B-F9 | a | No effect | 0 |

| | | | |
|---|---|---|---|
| ^{a} At a conc range of 1 µg/ml - 0.01 pg/ml | | | |

### 5.14. Results of In Vivo DTH Reactivity of Mice Treated with Various Substances

A variety of substances were tested under *in vivo* bioassay conditions and found to inhibit the experimental DTH sensitivity of mice to different extents depending on their state of purification. The mice were treated with an active substance. Generally, the substances that have been purified to substantial homogeneity by high pressure liquid chromatography provide "R" values in the tens of thousands. The results for one group of experiments are presented in Table IX.

**Table IX.**

| Weekly Treatment of Mice with Various Substances and Their Effect on the DTH Sensitivity of Mice. | | | | |
|---|---|---|---|---|
| Test Material | Dose (µg/gm mouse) | DTH Response (10⁻²mm) | Inhibition | |
| | | | of DTH (%) | "R" value % x (µg/gm)⁻¹ |
| None | - | 17.2 ± 2 | 0 | - |
| | | | | |
| (-) Control | | 2 | - | - |
| | | | | |
| 0.5 M NaCl | - | 16.5 ± 1.5 | 5 | - |
| | | | | |
| Intact Heparin | a | - | No effect | 0 |
| | | | | |
| Fragmin | | | | |
| Batch 38609 | 0.2 | 3 ± 1 | 85 | 425 |
| DECM | | | | |
| MM5 "Soup" | a | - | No effect | - |
| | | | | |
| Seph.4B-F6 | 0.032 | 16.5 ± 5 | 5 | - |
| | 0.016 | 16 ± 2 | 10 | - |
| | 0.0032 | 14 ± 2 | 20 | - |
| | 0.0006 | 7.1 ± 1 | 65 (max) | 110,000 |
| | | | | |
| F6/HPLC-F9 | 0.032 | 11 ± 2 | 40 | - |
| | 0.01 | 17 ± 2 | 0 | - |
| | 0.002 | 6 ± 0.5 | 70 | - |
| | 0.0006 | 6 ± 1.2 | 70 (max) | 120,000 |
| | | | | |
| F6/HPLC-F11 | 0.02 | 17 ± 3 | 0 | - |
| | 0.01 | 13 ± 1 | 25 | - |
| | 0.001 | 9.5 ± 2.5 | 55 | - |
| | 0.0006 | 2.8 ± 0.5 | 90 (max) | 150,000 |
| | | | | |
| F6/HPLC-F12 | 0.02 | 18 ± 2 | 0 | - |
| | 0.01 | 15 ± 2 | 15 | - |
| | 0.001 | 8.2 ± 1.5 | 60 | - |
| | 0.0006 | 4 ± 1 | 80 (max) | 130,000 |

| | | | | |
|---|---|---|---|---|
| ^{a} At a dosage range of 0.04-0.0004 µg/gm mouse. | | | | |

As noted in Table IX, intact heparin and the starting ECM + MM5 "soup" exhibited no *in vivo* effect. In the latter case, no effect is obtained most likely because of the counterbalancing effects of inhibitory and augmentative components. A fresh sample of Fragmin (Batch 38609) exhibited a modest "R" value, comparable to that obtained in earlier experiments (*See*, first entry, Table IX). A Sepharose 4B fraction manifested a slightly lower "R" value than the corresponding fractions obtained under HPLC II conditions.

The results from another set of experiments, listed in Table X, confirmed the absence of any effect from the starting ECM + MM5 "soup". Notably, an HPLC II fraction, no. F5/HPLC-L22, showing very high specific regulatory activity when injected subcutaneously into mice ("R" value = 454,545 % x (µg/gm)⁻¹), also demonstrated oral activity albeit at a higher dose ("R") value + 5,000 % x (µg/gm)⁻¹). It is also apparent from Table XXI that active substances isolated from the ECM have greater *in vivo* specific regulatory activity than those obtained from Fragmin. Hence, the apparent desulfation reduces the specific inhibitory activity of the active substances of the present invention. In fact, under *in vitro* bioassay conditions, augmentative "R" values are obtained from such "desulfated" disaccharides.

Further experiments have also demonstrated that galactosamine, a monosaccharide or simple sugar having no sulfate groups, is capable of acting as an antagonist of the inhibitory activity of the sulfated oligosaccharide of the present invention. Thus, it is also possible that the desulfated oligosaccharides act as direct augmentative components or as antagonists of the specific inhibitory activity of the carboxylated and/or sulfated oligosaccharides. The observations of the present investigators are also consistent with a mechanism by which certain substances (e.g., a trisulfated disaccharide) behave as agonists of an as yet unidentified natural inhibitor of active TNF-α secretion.

**Table X.**

| *In Vivo* DTH Reactivity Data for Mice Treated Subcutaneously With Various Substances | | | | |
|---|---|---|---|---|
| Test Material | Dose (µg/gm mouse) | DTH Response (10⁻² mm) | Inhₘₐₓ (%) | "R" value % x (µg/gm)⁻¹ |
| ECM + MM5 "Soup" | a | 20.4 ± 0.7 | No effect | 0 |
| F5/HPLC-L22 | 0.008^{b} | 13.2 ± 1.3 | 40 ± 12% | 5,000 |
| F5/HPLC-L22 | 0.000132 | 9.7 ± 1.3 | 60 ± 17% | 454,545 |
| | | | | |
| FRAGMIN | | | | |
| FR/HPLC-2 | 0.00048 | 8.5 ± 1.3 | 70 ± 20% | 145,833 |

| | | | | |
|---|---|---|---|---|
| ^{a} At a dose range of 0.04 - 0.0004 µg/gm mouse. | | | | |
| ^{b} Administered orally. Positive control group had DTH response of 20.0 ± 1.1 and the negative control group had a DTH response of 2.0 ± 1.0. | | | | |

### 5.15. Comparative In Vivo Activity of SAX-HPLC Fractions Versus Known Disaccharide Markers

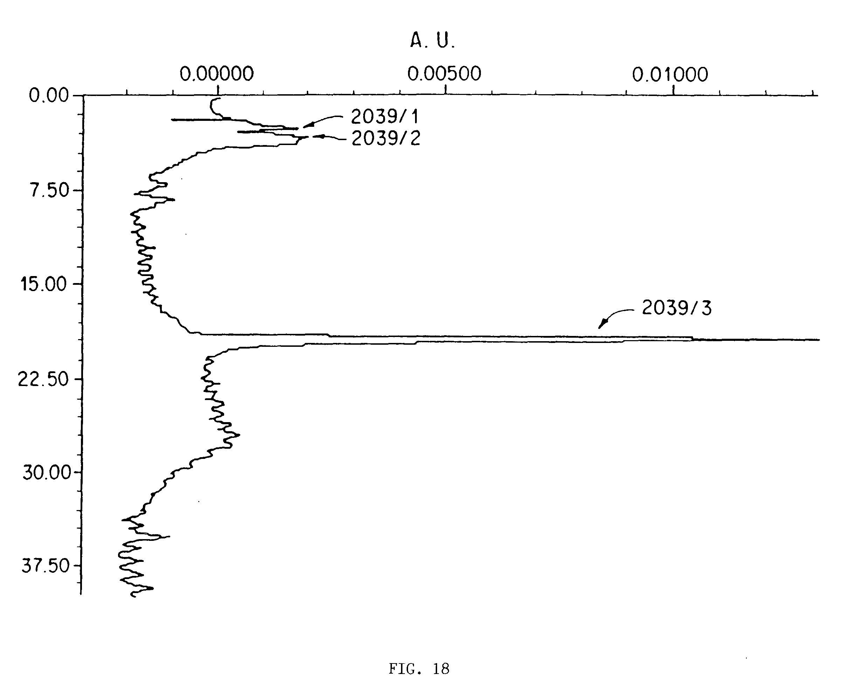

Two disaccharide markers were tested under *in vivo* conditions to determine their ability inhibit the relative DTH reactivity of mice. As shown in Table XXII, the two markers (H-1020 and H-9267) exhibited moderate activity having "R" values between 140,000 - 160,000 % x (µg/gm)⁻¹ when injected subcutaneously into mice. The marker, H-1020, was also tested orally and found to have modest activity ("R" value = 531 % x (µg/gm)⁻¹). The H-1020 marker is an O,N-di-sulfate, whereas the H-9267 marker is an O,O,N-trisulfate. Their structures are depicted, below. 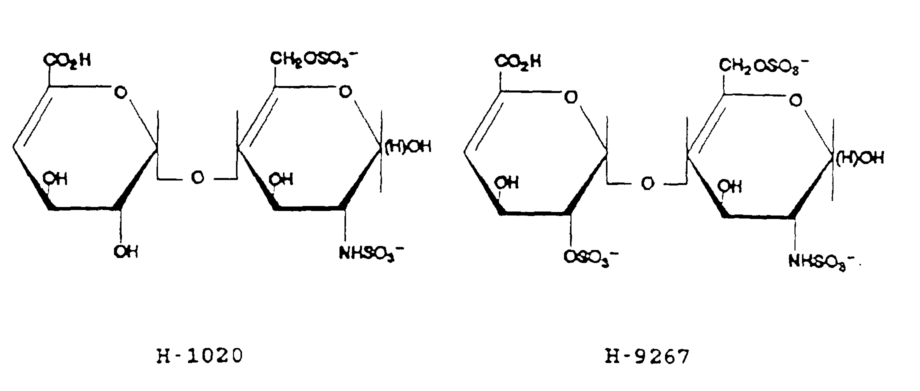 As presented in Table XI, the SAX-HPLC fraction L22/SAX-A23/4 (Fig. 12) provided further improvement over the already high specific regulatory activity of F5/HPLC-L22, giving an "R" value of 630,303 % x (µg/gm)⁻¹ compared with an "R" value of 454,545 % x (µg/gm)⁻¹ for F5/HPLC-L22 (Table XXI). It was discovered, however, that this disaccharide substance, with a retention time through the SAX-HPLC column which is almost identical to the retention time of H-9267, loses its sulfate groups at pH -3.5 over a few days at room temperature. Thus, reanalysis of an aged sample through a SAX-HPLC column revealed that the original peak at 23.10 min. had given way to three major peaks, designated 2039/1, 2039/2, and 2039/3 in Fig. 18, all having retention times shorter than A23/4. Peak 2039/3, having a retention time similar to the H-1020 marker, is likely to have lost an N-sulfate group. Peaks 2039/1 and 2039/2 likely correspond to monosulfated or fully desulfated disaccharides. (Their retention times are comparable to a disaccharide marker, H-0895, an N-acetylglycosaminoglycan having no sulfate groups.)

These "desulfated" substances were each collected and tested under the *in vivo* DTH bioassay conditions and found, surprisingly, to have only moderate or no inhibitory activity. (*See*, Table XI). Indeed, under the *in vitro* human PBL assay, all three peaks manifested augmentation of active TNF-α secretion. These *in vitro* results are given, immediately below:

| SAX-HPLC Peak | conc (pg/ml) | Augₘₐₓ (%) | "R" value % x (pg/ml)⁻¹ |
|---|---|---|---|
| 20391/1 | 1 | 5 | 5 |
| 2039/2 | 1 | 35 | 35 |
| 2039/3 | 1 | 42 | 42 |

**Table XI.**

| Additional *In Vivo* Results Using a Variety of Disaccharides Administered Subcutaneously | | | | |
|---|---|---|---|---|
| Test Material | Dose (µg/gm mouse) | DTH Response (10⁻²mm) | Inhₘₐₓ (%) | "R" Value % x (µg/gm)⁻¹ |
| PBS (Pos. Control) | - | 18.6 ± 0.7 | - | - |
| Naive (Neg. Control) | - | 1.4 ± 0.2 | - | - |
| | | | | |
| SAX-HPLC Fractions | | | | |
| A23/4 | 0.000132 | 3 ± 1 | 83 | 630,303 |
| 2039/3 | 0.0005 | 3.1 ± 1.1 | 83 | 166,000 |
| 2039/1 | 0.000132 | 18.5 ± 1.1 | No effect | 0 |
| | | | | |
| "Markers" | | | | |
| H-1020 | 0.0005 | 4.7 ± 0.7 | 73 | 146,000 |
| H-1020 | 0.128^{a} | 5.9 ± 0.9 | 68 | 531 |
| H-9267 | 0.0005 | 3.5 ± 1 | 80 | 160,000 |

| | | | | |
|---|---|---|---|---|
| ^{a} Administered orally. | | | | |

### 5.15.1. Further Results of the Ability of Selected Disaccharides to Regulate the In Vivo Production of Active TNF-α.

Additional experiments were performed in which selected disaccharide molecules, commercially available from Sigma Chemical Co. and identified herein by their respective Sigma Catalog Nos., were tested for their the ability to inhibit or augment the experimental DTH reaction in mice and, thus, offer an indication of their ability to regulate the production by these mammals of active TNF-α.

In particular, CD1 mice (available from the Weizmann Institute Animal Breeding Center, Rehovot, Israel), 4-12 mice per group, were treated as described in Section 6.5.

The results of the various experiments are summarized in Table XIIA, below. As can be seen, four of the eleven disaccharides tested exhibited an inhibitory effect on the swelling of the ears of the mice in response to the administered oxazolone. The inhibition of the T cell-mediated inflammatory response is thus seen as an indication that the disaccharides exhibiting a non-zero "R" value can down regulate the production of active TNF-α. From the results listed in the Table, the "R" values range from a relatively modest 65,000 % x (µg/gm)⁻¹ to over about 1,500,000 % x (µg/gm)⁻¹. It should be pointed out that a high "R" value is not necessarily the most desirable characteristic of the active compounds of interest. In particular, the dose "window" within which a particular compound exhibits physiological effects should be as broad as possible so that there is less likelihood that the dose administered will fall outside the effective dose range. As indicated in the footnotes of Table XIIA. the molecule H-9392 appears to have the broadest dose window, 0.000132-0.004 µg/gm, of the compounds tested.

Equally evident from the results is the surprising ability of one of the eleven disaccharides tested to augment the swelling caused by the experimental DTH T cell reaction. Compound H-0895, which has no sulfate groups, displays a tremendous effect on the degree of swelling at the very low dose of about 1.2 picograms disaccharide per gram mouse. The resulting "R" value of about 76,700,000 % x (µg/gm)⁻¹ is presently unrivaled.

**TABLE XIIA.**

| Additional *In Vivo* Results Using a Variety of Disaccharide Markers Administered Subcutaneously. | | | | |
|---|---|---|---|---|
| Test material | Dose (µg/gm) | DTH (10⁻² mm) | Inhₘₐₓ (%) | "R" value % x (µg/gm)⁻¹ |
| H-9392^{a} | 0.0012 | 4.2 ± 0.7 (17.8 ± 0.9) | 78 | 6.5 x 10⁴ |
| H-1020^{b} | 0.0004 | 6.7 ± 1.1 (19.6 ± 1.2) | 66 | 1.65 x 10⁵ |
| H-9267^{c} | 0.0004 | 7.2 ± 1 (19.6 ± 1.2) | 64 | 1.60 x 10⁵ |
| H-9517^{d} | 0.00004 | 7.6 ± 1 (20.4 ± 0.7) | 62 | 1.55 x 10⁶ |
| H-0895^{e} | 0.0000012 | *37.3* ± *0.7* (18.9 ± 0.7) | +92 | 7.67 x 10⁷ |
| H-9017^{f} | N.E. | | | 0 |
| H-8642^{g} | N.E. | | | 0 |
| H-9142^{h} | N.E. | | | 0 |
| H-8767ⁱ | N.E. | | | 0 |
| H-8892^{j} | N.E. | | | 0 |
| H-1145^{k} | N.E. | | | 0 |

| | | | | |
|---|---|---|---|---|
| ^{a} H-9392 is 2-O-Sulfate-4-deoxy-4-en-iduronic acid-(α-1,4)-2-deoxy-2-N-sulfateglucosamine. The PBS (positive control) value for this data set is presented in parenthesis. The swelling of naive (unimmunized) mice was 2.0 ± 0.5 mm. This value was used for all the above calculations. The effective dose range for inhibition ≥50% of control was 0.000132-0.004 µg/gm. | | | | |
| ^{b} H-1020 is 4-deoxy-4-en-iduronic acid-(α-1,4)-2-deoxy-2-N-sulfate-6-O-sulfateglucosamine. The effective dose range for inhibition ≥50% of control was 0.00004-0.0004 µg/gm. The "R" value presented in this Table compares favorably with the early "R" value reported previously in Table XXIII, above. These results indicate a remarkable reproducibility of the *in vivo* test method in different groups of CD1 strain mice. | | | | |
| ^{c} H-9267 is 2-O-sulfate-4-deoxy-4-en-iduronic acid-(α-1,4)-2-deoxy-2-N-sulfate-6-O-sulfateglucosamine. The effective dose range for inhibition ≥50% of control was narrow. | | | | |
| ^{d} H-9517 is 2-O-sulfate-4-deoxy-4-en-iduronic acid-(α-1,4)-2-deoxy-2-N-acetyl-6-O-sulfateglucosamine. The effective dose range for inhibition ≥50% of control was 0.00004-0.00012 µg/gm. | | | | |
| ^{e} H-0895 is 4-deoxy-4-en-iduronic acid-(α-1,4)-2-deoxy-2-N-acetylglucosamine. *This result indicates an augmentation of the DTH reaction.* The effective dose range for augmentation ≥50% of control was 0.000001-0.00004 µg/gm. | | | | |
| ^{f} H-9017 is 4-Deoxy-4-en-iduronic acid-(α-1,4)-2-deoxy-6-O-sulfateglucosamine. "N.E." indicates that no effect was observed at the tested dosage range of 0.000004-4 µg/gm mouse. | | | | |
| ^{g} H-8642 is 4-deoxy-4-en-iduronic acid-(α-1,4)-2-deoxy-2-N-acetyl-6-O-sulfateglucosamine. | | | | |
| ^{h} H-9142 is 2-O-sulfate-4-deoxy-4-en-iduronic acid-(α-1,4)-2-deoxyglucosamine. | | | | |
| ⁱ H-8767 is 2-O-sulfate-4-deoxy-4-en-iduronic acid-(α-1,4)-2-deoxy-2-N-acetylglucosamine. | | | | |
| ^{j} H-8892 is 2-O-sulfate-4-deoxy-4-en-iduronic acid-(α-1,4)-2-deoxy-6-O-sulfateglucosamine. | | | | |
| ^{k} H-1145 is 4-deoxy-4-en-iduronic acid-(α-1,4)-2-deoxy-2-N-sulfateglucosamine. | | | | |

The structures of four inhibitory disaccharide compounds, H-9392, H-9517, H-1020 and H-9267, are presented below.

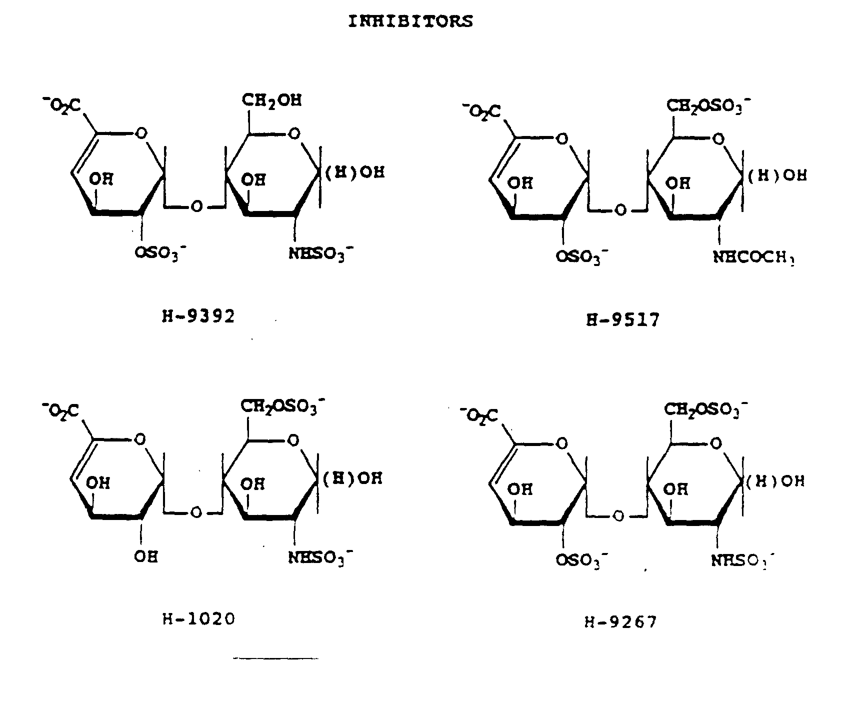

Of the eleven disaccharides tested six failed to exhibit any consistent effects and, thus, may be classified as "neutral." The structures of these neutral compounds are presented below.

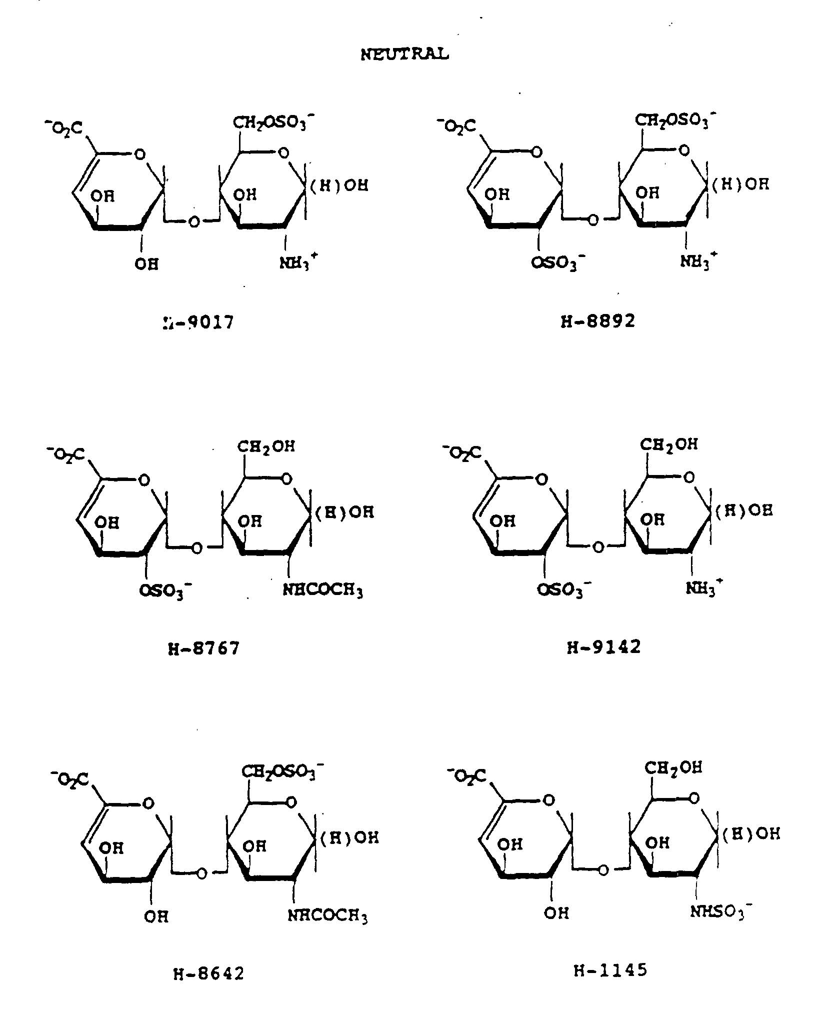

Of the eleven, one disaccharide augmented the effects of the experimental DTH reaction. This compound, H-0895, has the structure presented below.

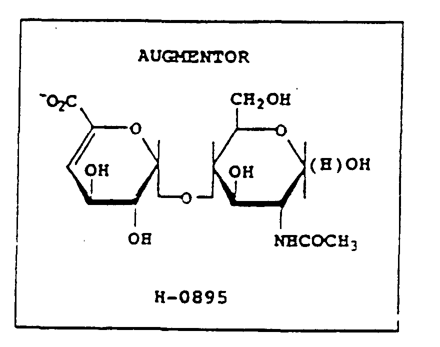

It is thus possible to propose a generic formula that embodies the structural characteristics of the inhibitory compounds. This generic formula (H-GENUS) is shown below: 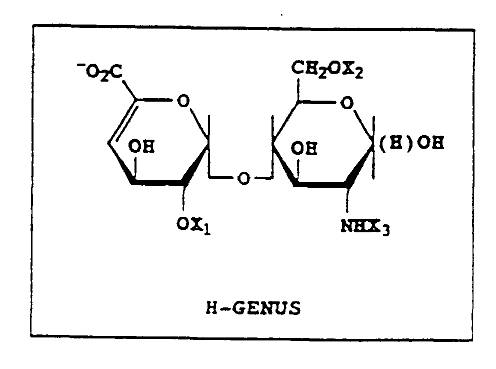 in which, X₁ is H or SO³⁻ ; X₂ is H or SO³⁻; and X₃ is SO³⁻ or COCH₃, provided that if X₃ is SO³⁻, then at least one of X₁ or X₂ is SO³⁻ and if X₃ is COCH₃, then both X₁ and X₂ are SO³⁻. In terms of a compound having a fairly broad window of effective dosages, X₁ is preferably SO³⁻, X₂ is preferably H, and X₃ is preferably SO³⁻ (i.e., H-9392 has the broadest window of effective inhibitory dosages).

One may also observe from the results presented above that, in terms of inhibition, the preferred substituent at the glycosamine nitrogen is sulfate. With a sulfate at the 2-N position of glucosamine (X₃), inhibitory activity is observed with the presence of just one other sulfate either at the 2-position of the iduronic acid residue or the 6-position of the glycosamine. The presence of two additional sulfates at both hydroxyl positions would also work, but the absence of any additional sulfate (as in H-1145) produces a "neutral" compound.

By contrast, the introduction of an acetyl group at the 2-N position of glucosamine requires the presence of two additional sulfates, one each for 2-position of the iduronic acid (X₁) and the 6-position of the glucosamine (X₂). The absence of any substituent at the 2-N position of glucosamine, giving rise to a positively charged ammonium group, effectively cancels any inhibitory effect, as evidenced by the fact that all of the test compounds, H-9017, H-8892, and H-9142, were "neutral." The presence of one or two sulfate groups at the 2-position of the iduronic acid or the 6-position of the glucosamine had no apparent effect.

Finally, the presence of an acetyl group at X₃ combined with the absence of sulfate groups at X₁ and X₂ give rise to an augmenting regulatory activity (H-0895).

One should note the strong correlation between the negative charges present in the disaccharide and its ability to inhibit the production of TNF-α. The presence of the positively charged ammonium substituent gives rise to "neutrality," whereas the charge-neutral compound H-0895 augments the production of active TNF-α.

**TABLE XIIB.**

| Empirical Rules Gleaned From the Results of *In Vivo* DTH Studies Involving Commercially Available Disaccharides. | | | |
|---|---|---|---|
| Identity of Xₙ in H-GENUS | | | Observed Activity |
| X₃ | X₂ | X₁ | |
| SO³⁻ | SO³⁻ | SO³⁻ | Inhibition |
| SO³⁻ | SO³⁻ | H | Inhibition |
| SO³⁻ | H | SO³⁻ | Inhibition |
| SO³⁻ | H | H | Neutral |
| COCH₃ | SO³⁻ | SO³⁻ | Inhibition |
| COCH₃ | SO³⁻ | H | Neutral |
| COCH₃ | H | SO³⁻ | Neutral |
| COCH₃ | H | H | Augmentor |
| H²⁺ | SO³⁻ | SO³⁻ | Neutral |
| H²⁺ | SO³⁻ | H | Neutral |
| H²⁺ | H | SO³⁻ | Neutral |

### 5.15.2. Results of the Ability of Selected Monosaccharides to Regulate the In Vivo Production of Active TNF-α.

Additional experiments were performed in which selected monosaccharides, commercially available from Sigma, were tested for their ability to regulate the in vivo production of active TNF-α. Using substantially the same procedure described in the preceding Section, CD1 mice, 6 to a group, were inoculated and treated with a variety of control and test substances to determine the effect, if any, of the subcutaneously injected substances on the experimental DTH reaction of the test animals. The results of these experiments are presented in the Table below.

**TABLE XIIC.**

| Additional *In Vivo* Results Using a Variety of Monosaccharides Administered Subcutaneously | | | | |
|---|---|---|---|---|
| Test material (µg/gm)⁻¹ | Dose (µg/gm) | DTH (10⁻² mm) | Inhₘₐₓ (%) | "R" value % x |
| GlcN^{a} | 0.000012 | 5 ± 0.7 (19.7 ± 1.2) | 75 | 6.25 x 10⁶ |
| | | | | |
| | 0.4 | 2.3 ± 0.9 (19.7 ± 1.2) | 88 | 2.2 x 10² |
| | | | | |
| GlcN-2S^{b} | N.E. | | | 0 |
| | | | | |
| GlcN-3S^{c} | N.E. | | | 0 |
| | | | | |
| GlcN-6S^{d} | N.E. | | | 0 |
| | | | | |
| GlcN-2,3S^{e} | 0.0012 | 6.3 ± 1 (19.7 ± 1.2) | 68 | 5.67 x 10⁴ |
| | | | | |
| GlcN-2,6S^{f} | 1.2 | 7.7 ± 1 (19.7 ± 1.2) | 61 | 5.08 x 10 |
| | | | | |
| NAc-GlcN^{g} | N.E. | | | 0 |
| | | | | |
| GalN^{h} | 0.00004 | 8.9 ± 0.6 (18.9 ± 0.7) | 50 | 1.25 x 10⁶ |
| | | | | |
| | 0.12 | 6.5 ± 0.7 (18.9 ± 0.7) | 67 | 5.58 x 10² |

| | | | | |
|---|---|---|---|---|
| ^{a} GlcN is glucosamine or 2-amino-2-deoxy-D-glucose. Two effective dose ranges were observed for inhibition ≥50% of control: 0.000004-0.00004 µg/gm and 0.004-4 µg/gm. | | | | |
| ^{b} GlcN-2S is D-Glucosamine-2-N-sulfate. "N.E." indicates that no effect was observed at the tested dosage range of 0.000004-4 µg/gm mouse. | | | | |
| ^{c} GlcN-3S is D-Glucosamine-3-sulfate. | | | | |
| ^{d} GlcN-6S is D-Glucosamine-6-sulfate. | | | | |
| ^{e} GlcN-2,3S is D-Glucosamine-2,3-disulfate. The effective dose range for inhibition ≥50% of control was narrow. | | | | |
| ^{f} GlcN-2,6S is D-Glucosamine-2,6-disulfate. The effective dose range for inhibition ≥50% of control was narrow. | | | | |
| ^{g} NAC-GlcN is N-Acetylglucosamine. | | | | |
| ^{h} GalN is D-Galactosamine. Two effective dose ranges were observed for inhibition ≥50% of control: 0.00004-0.00012 µg/gm and 0.04-0.4 µg/gm. | | | | |

The stereochemistry of the hydroxyl at the 4-position of the monosaccharide determines whether the sugar is glucosamine (α-face) or galactosamine (β-face), as indicated, below.

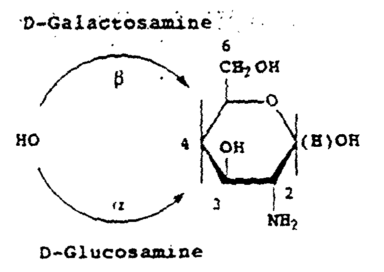

It thus appears that N-acetylation or the presence of one sulfate in the monosaccharide interferes with the ability of glucosamine to inhibit the DTH reaction in mice.

### 5.15.3. Treatment of Adjuvant Arthritis (AA) in Rats With Selected Monosaccharides and Disaccharides.

AA was induce in female Lewis rats, 6-8 weeks old, as described below Groups of rats, 5-10 rats per group, were treated with a test substance by subcutaneous injection 1 day before induction of the experimental arthritis and subjected to repeat treatments weekly thereafter. The effects, if any, were scored as described below.

Adjuvant arthritis is an experimental disease inducible in some strains of rats by immunizing them to antigens of Mycobacterium tuberculosis (Pearson, C. M., Proc. Soc. Exp. Biol. Med. (1956) 91:91). This experimental disease is considered to be a model of human rheumatoid arthritis (Pearson, C. M., Arthritis Rheum. (1964) 7:80). The arthritis appears to be caused by T lymphocytes that recognize an antigen of M. tuberculosis that is cross-reactive with structures in the joint tissues (Cohen I. R., et al., Arthritis Rheum. (1985) 28:841).

Lewis rats were immunized with M. tuberculosis (1 mg) in oil to induce adjuvant arthritis (Pearson, C. M., Proc. Soc. Exp. Biol. Med. (1956) 91:91). Five days later the rats were inoculated subcutaneously as indicated and scored for the development of arthritis on a scale of 0-16 as described (Holoshitz, J., et al., Science (1983) 219:56).

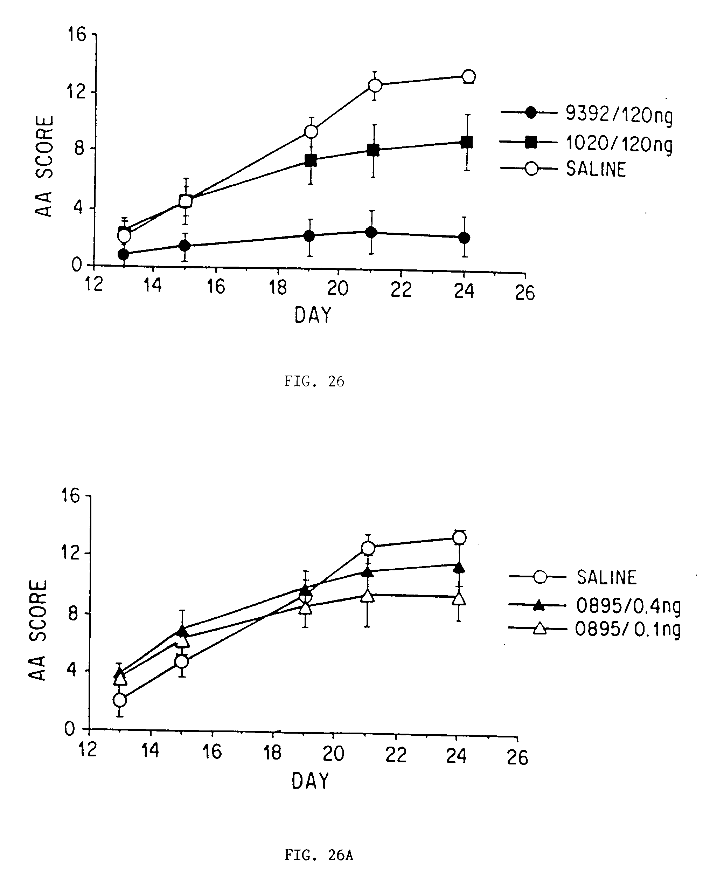

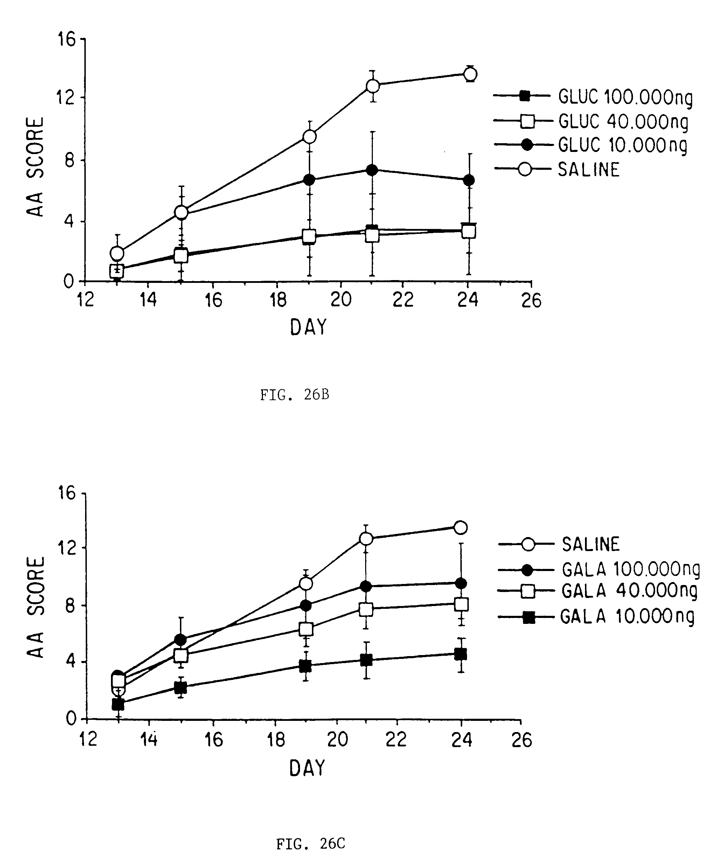

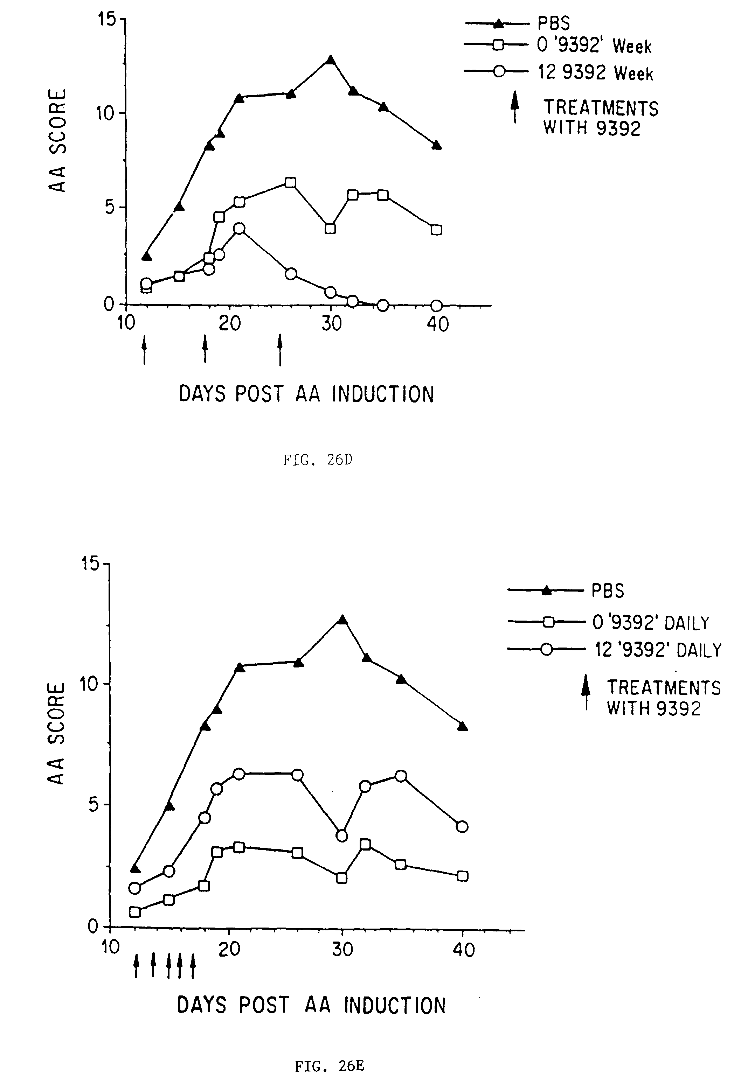

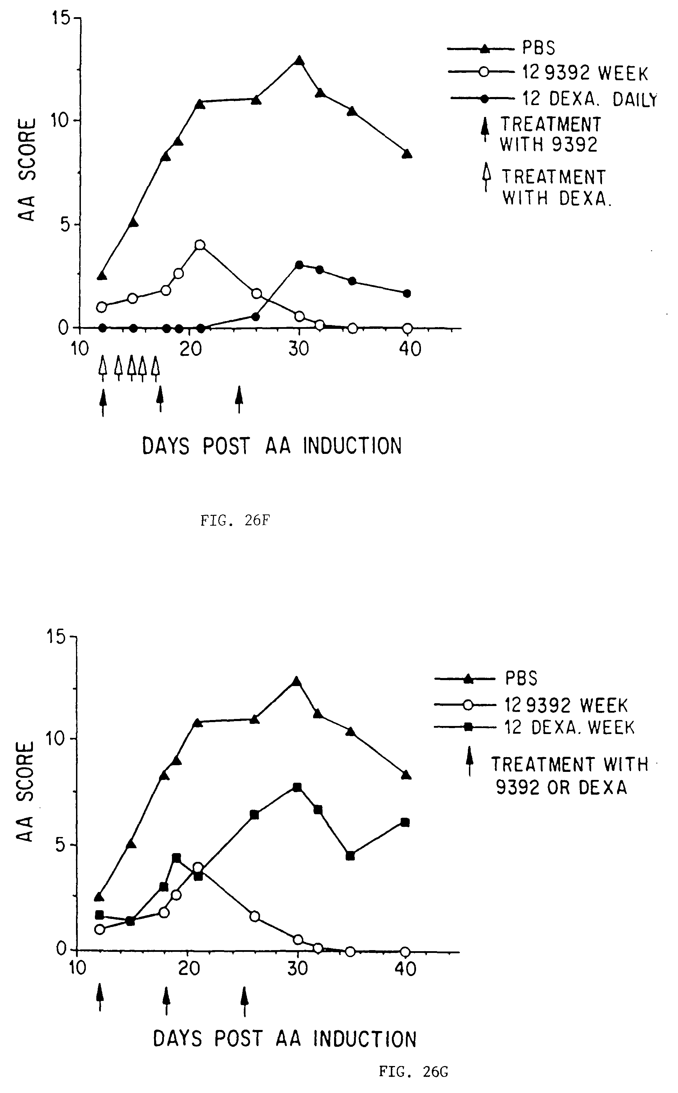

Of the three disaccharides tested, H-9392 showed the most pronounced effect in lowering the AA score relative to control groups of rats which received only saline (0.1 ml). As illustrated in FIG. 26, H-9392, administered at 120 ng/rat or 0.6 ng/gm rat, suppressed the destructive inflammation of AA almost completely (up to about 90%) 24 days after induction and H-1020 inhibited the development of AA by about 30% relative to control at day 24. In contrast, the augmentor, H-0895, showed an increased level of AA development within about 2 weeks of induction at 2 dosage levels: 0.1 and 0.4 ng/rat. This effect, however, faded rapidly after that until, at day 24, the AA score was not substantially different from control levels. (*See*, FIG. 26A.)

On the other hand, certain monosaccharides were also found to exhibit *in vivo* inhibitory effects in this rat model. As shown in FIG. 26B, glucosamine treatment at three dosage levels inhibits the development of AA by about 60-80% of control levels. Galactosamine also exhibits inhibitory effects but to a much lesser degree than that shown by glucosamine. (*See*, FIG. 26C.)

Most interestingly, further experiments carried out with H-9392, in which the disaccharide was administered either weekly or daily beginning at day 0 (start of induction of AA) or at day 12 (the rat is already suffering from AA), showed positive suppression of the severity of AA in all cases. The results of these experiments are presented in FIGS. 26D (weekly) and 26E (daily). As indicated in FIG. 26D, weekly administration of H-9392 beginning at day 12, that is, even after the rat is already afflicted with AA, is at least as effective as weekly treatment at the start of induction (day 0) relative to control. FIG. 26E shows that while daily treatment of afflicted rats was not as effective as daily treatment beginning at the start of induction, the daily treatment of rats with established arthritis was still highly effective at lowering the score of the AA relative to the control group.

These results show dramatically that the substances are effective not only in preventing the development of severe arthritis but are also effective in treating established arthritis. Furthermore, the present work also demonstrates that while the LMWHs described previously show inhibitory characteristics only when administered weekly, the disaccharides of the present invention are able to manifest useful inhibitory activity when administered weekly or daily.

As a further illustration of the superiority of the compounds in the treatment of experimentally-induced AA, a separate, comparative set of experiments was carried out in which groups of Lewis rats (5 rats per group) were treated by subcutaneous injection with either dexamethasone phosphate (purchased from Sigma, a known antiinflammatory agent) or the disaccharide 9392. Treatments were begun 12 days after induction of the adjuvant arthritis (AA) disease and consisted of two treatment regimens: the first involving daily injection of the known antiinflammatory agent; the second involving weekly injection of the known antiinflammatory agent or the disaccharide. In all cases, 100 µg of the known antiinflammatory agent in 0.1 ml of phosphate buffer solution was administered to each rat, while 120 ng of the disaccharide, also in 0.1 ml of phosphate buffer solution, was administered per rat. As a control, a group of rats was injected with 0.1 ml of phosphate buffer solution only. For the daily dose regimen of known antiinflammatory agents, treatments were ended after day 17 post-induction and for the weekly dosage regimen of known antiinflammatory agent or disaccharide, the treatments were ended after day 26 post-induction.

The results of the above experiments are illustrated in Figures 26F and 26G. Upon examination of Figure 26F, one sees that the *weekly* administration of the disaccharide 9392 compares well with the *daily* administration of dexamethasone phosphate during about the first week of treatment. Note, however, that after treatment has ended (after day 26), the group of rats that received daily dexamethasone phosphate suffered a relapse while the disaccharide group continued to improve. At 30 days post-induction of AA, the disaccharide group fared better than the dexamethasone phosphate group.

What is more, comparing the effectiveness of weekly dexamethasone phosphate vs. weekly disaccharide 9392, as illustrated in Figure 26G, one sees that at 30 days post-induction of the AA, weekly administration of dexamethasone phosphate resulted in only a moderate reduction in the severity of the AA score. In contrast, the weekly administration of disaccharide 9392 at 30 days post-induction of the AA gave rise to an almost complete suppression of the experimentally-induced adjuvant arthritis. Again, of particular note, after weekly treatment was ended for the dexamethasone phosphate, the rat suffered a relapse of the adjuvant arthritis. As noted previously, the rats treated with the disaccharide 9392, however, continued to improve even after administration of the disaccharide had ceased.

Thus, the weekly administration of the disaccharide 9392 is manifestly superior over the daily or weekly administration of dexamethasone phosphate over the long term. Whereas the rats treated with dexamethasone phosphate, either daily or weekly, suffered a relapse of the disease after treatment was ended, the rats treated with the disaccharide continued to exhibit improved AA scores, reflecting a continued post-treatment inhibition of the disease.

### 5.15.4. Results of Experiments Relating to the Lipopolysaccharide (LPS)-Induced Inflammation of the Rat Cornea.

LPS-induced inflammation of the cornea is TNF dependent, as shown by the work of Vanderhagen, C. and coworkers in the Netherlands, "Kinetics of Intraocular TNF and IL-6 in Endotoxin-Induced Uveitis in the Rat," submitted for publication. Using a 30-gauge needle, LPS (5 ng) was injected into the cornea of Lewis rats. After one day, separate groups of rats, 2 to a group (or 4 eyes to a group), were then injected subcutaneously with phosphate-buffered saline (0.05 ml) or H-1020 (at a dose of either 50 ng/rat or 200 ng/rat). The effects, if any, were scored as follows:

| | |
|---|---|
| edema | 0-3 points |
| neovascularization | 0-3 points |
| redness | 1/0 points |
| swelling | 1/0 points |
| hemorrhage | 1/0 points |
| miosis | 1/0 points |
| synaechi | 1/0 points (iris adherent to lens or cornea) |
| hypopyon | 1/0 points (pus or blood in anterior chamber) |
| hazy cornea | 1/0 points |

in which the sum of the points is used as the total score.

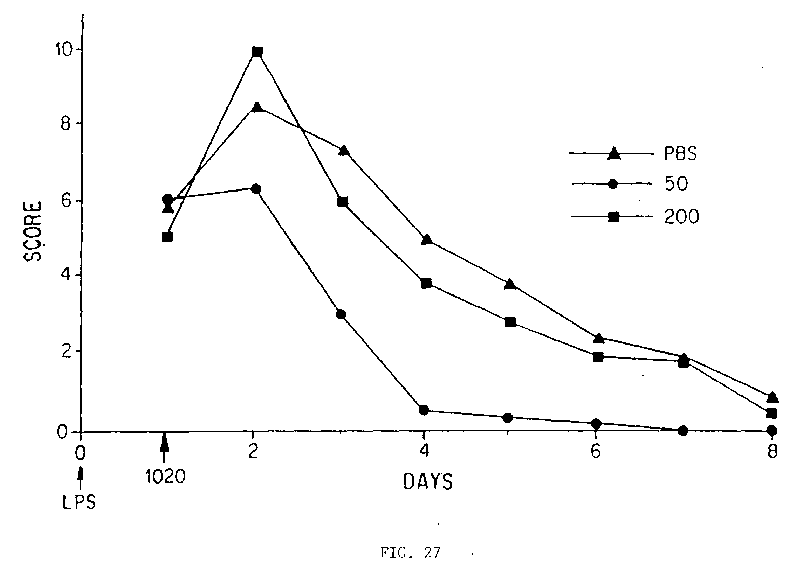

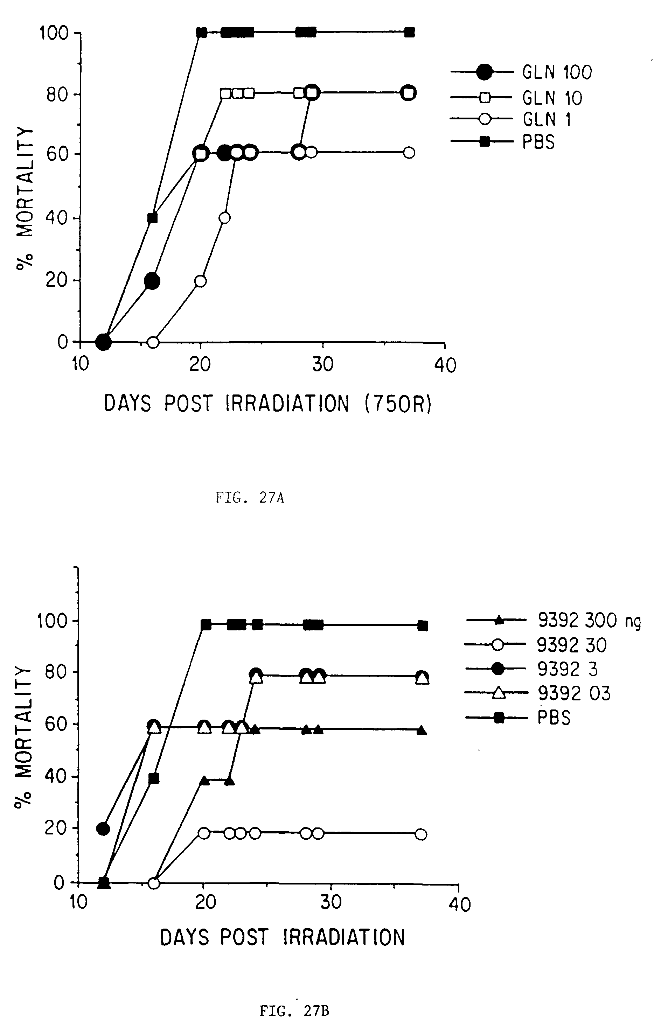

As can be seen from the graphical representation of the results (FIG. 27), the 50 ng/rat dose was effective to suppress the effects of the *local* LPS-induced inflammation relative to control. Interestingly, a dose of 200 ng/rat failed to provide a significant effect.

### 5.15.5. Results of Experiments Relating to the Lipopolysaccharide (LPS)-Induced Uveitis in Rats.

Uveitis is the inflammation of the anterior chamber of the eye in response to LPS given systemically. Like the inflammation produced in the preceding Section by the local administration of LPS, uveitis is TNF-dependent. In the present experiment, groups of Lewis rats, 8 to 10 weeks old, 8 eyes per group, were treated at Day 1 with either H-9392 (at a dose of 32 ng/rat or 500 ng/rat) or saline (0.1 ml). At Day 2, a 2 mg/ml solution of LPS (50 µl) was injected into each foot pad; each rat received a total of 200 µg LPS. At Day 3, each eye was tapped and the concentration of total protein was measured as a quantitative assay of the degree of inflammation. The results of these experiments are provided immediately below.

| Rats | Treatment | Median Protein (mg/ml) |
|---|---|---|
| No LPS | - | 0.36 |
| LPS | Saline | 18.4 |
| LPS | 32 ng H-9392 | 5.2 |
| LPS | 500 ng H-9392 | 4.8 |

Hence, a single administration of H-9392, at either dosage, was effective to suppress the inflammation produced by the systemic administration of LPS in the rat by over about 70% relative to control.

### 5.15.6. Results of Experiments Relating to the Radioprotective Effects of Selected Substances.

Groups of female BALB/c mice, 8 weeks old, 5-10 mice per group, were injected subcutaneously with either saline (0.1 ml, control), H-9392 (30 ng/mouse), or glucosamine (10,000 ng/mouse) 1 day before irradiation, and weekly thereafter until termination of the experiment on day 30. All the mice were irradiated to a dose of 700 rads using a ⁶⁰Co gamma radiation source.

The mortality within the different groups of mice was then scored over a 30 day period. The results showed that the mice that received only saline suffered a 100% mortality rate by day 30, whereas the mice that had been pretreated with H-9392, showed only a 40% death rate in the same period. The group of mice which had been injected with glucosamine also fared better than the control group, showing a 20% mortality rate in the same period.

Thus, pretreatment with the test substances, allowed the pretreated mice to survive a radiation regimen that would have ordinarily resulted in a 100% mortality rate for the group within 30 days.

In a separate experiment, groups of BALB/c mice (5 mice to a group) were likewise exposed to 750 rads of gamma-rays, except that these groups of mice were treated with test substances (saline at 0.1 ml per mouse; H-9392 at 0.3, 3, 30 and 300 ng/mouse; and glucosamine at 1, 10 and 100 µg/mouse) on the day before irradiation, on the sixth day after irradiation, and once again on the thirteenth day after irradiation only. All treatment ceased after the third and last administration. The results of this experiment are illustrated in FIGS. 39X (saline and glucosamine) and 39Y (saline and H-9392) and indicate that while all the animals in the control group had died by the twenty-second day after irradiation, only one mouse in the 30 ng H-9392 group had died by the thirtieth day after irradiation. The 300 ng H-9392 and the 1 µg glucosamine treatment regimens showed moderate activity in suppressing mortality after irradiation.

These results thus indicate a possible utility of the substances in cancer therapy in which the toxicity associated with radiation treatment may be reduced dramatically by proadministration of the instant compounds. This approach may, perhaps, allow an increase in the dosage of the radiation to higher more effective levels without observing toxic side effects. As illustrated by the experiments described above, the compounds of the present invention are highly effective at certain very low dosages even when treatment is limited to three-administrations of disaccharide.

### 5.15.7. The Ability of Selected Substances to Suppress Allograft Rejection.

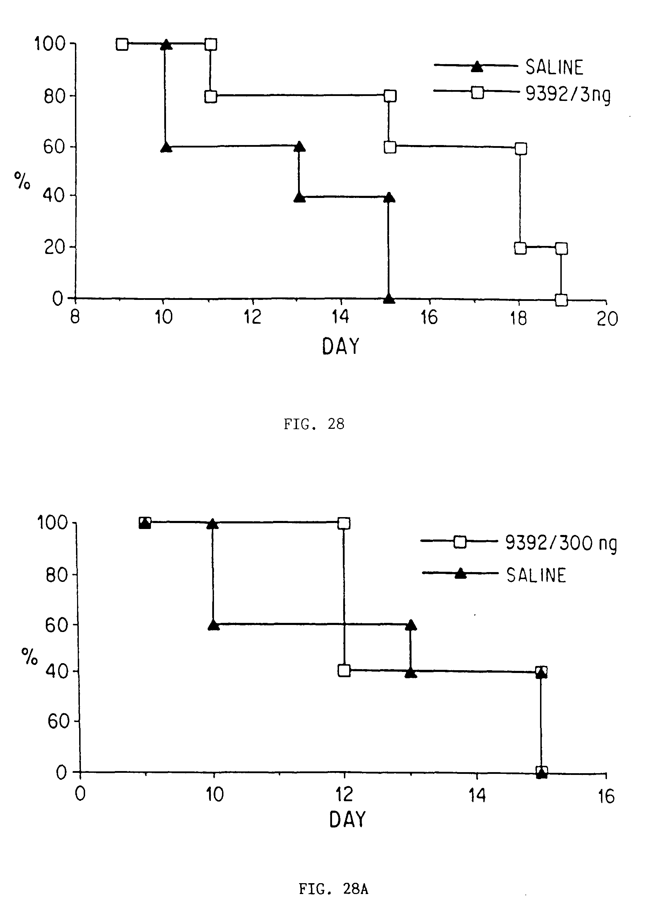

The effect of H-9392 was also tested in skin graft rejection experiments in mice. In particular, skin grafts from C57BL/6 donor mice (H-2^{b}) were applied to recipient BALB/c mice (H-2^{d}) according to the method of Baharav, E. et al. J. Immunol. Methods (1986) 90:143-144. The number of days to rejection was measured by the sloughing of the graft. The number of days to rejection was determined for a control group (injected with saline only, 0.1 ml), and test groups that received 3 ng or 300 ng of H-9392 by subcutaneous injection one day before grafting and weekly thereafter. The results, which are graphically represented in FIGS. 28 and 28A, reveal that the 3 ng/mouse dose delayed the level of skin graft rejection at 50% by 5 days! However, the same compound, administered at 300 ng/mouse failed to produce a significant difference at 50% rejection relative to control. These results are very significant given that rejection of a fully allogeneic skin graft is recognized to be one of the most powerful immune responses known.

### 5.15.8. The Ability of Selected Substances to Suppress the Development of IDDM in NOD Mice.

It is well known that NOD mice serve as a faithful model of human diabetes Type I. Indeed, all female NOD mice in our colony develop diabetes spontaneously within about 4-5 months of age. Because Type I diabetes or insulin-dependent diabetes mellitus (IDDM) is recognized as an autoimmune disease that may be precipitated by autoreactive T cells, selected compounds were tested for their ability to regulate this T cell-mediated autoimmune reaction.

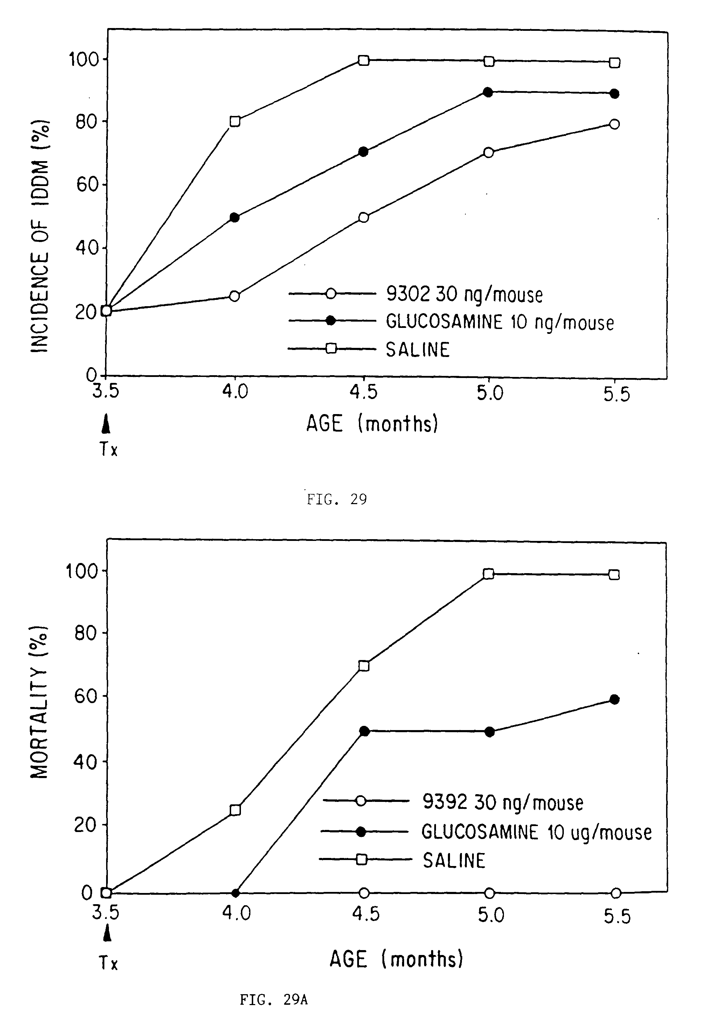

Hence, groups of female NOD mice, 6-12 to a group, were treated by subcutaneous injection with saline (0.1 ml), H-9392 (30 ng/mouse), or glucosamine (10,000 ng/mouse). All the mice were about 3.5 months old, as shown in FIG. 29, meaning that the mice as a group already endured a 20% incidence of IDDM. The incidence of IDDM can be monitored by the level of glucose in the blood of the mice. Non-diabetic mice exhibit a mean glucose level in the blood of about 140 ± 10 mg/ml. A mouse is considered a diabetic if its blood glucose level is equal to or exceeds 200 mg/ml (i.e., is greater than about three times the standard deviation of the "normal" level). For greater convenience, glucose urine levels were measured using the ClinstixTM dipstick (Ames). This test provides scores of 0 to +3, with a score of +2 or greater on two separate occasions taken as a positive indication of diabetes.

It was thus very surprising to discover that both the H-9392 and glucosamine inhibited the onset of diabetes in NOD mice such that at 4.5 months, when all the control mice were considered diabetic, only about 65% of the glucosamine-treated mice had become diabetic, while among the H-9392-treated mice, less than 50% were afflicted with the disease.

Put another way, FIG. 29A shows that by age 5 months, all the control mice had died from their diabetic condition. In contrast, only about half of the mice treated with 10,000 ng of glucosamine had died within the same time frame. Quite strikingly, none of the mice treated with 30 ng of H-9392 died within the same time frame; that is, the plot for the H-9392 results is coincident with the x-axis.

### 5.15.9. Effect of Selected Disaccharides on TNF-α-induced Expression of Adhesion Molecules, ICAM-1 and ELAM-1, by Endothelial Cells (EC).

The adhesion molecules, such as ICAM-1 and ELAM-1, are critical in the recognition and subsequent "rolling" (i.e., adherence to endothelium and migration through the endothelium) of leukocytes involved in the inflammatory response. In response to active TNF-α, endothelial cells-(EC) express ICAM-1 and ELAM-1. Thus, TNF-α can augment inflammation by up regulating the signals for leukocyte adherence and migration. To determine the effect of the disaccharides of the present invention on the TNF-α-induced expression by EC of ICAM-1 and ELAM-1, the following experiment was carried out.

Freshly isolated human umbilical vein EC were grown in M-199 (Gibco Laboratories) supplemented with 10% FCS, 8% human serum, antibiotics and 50 µg per ml endothelial cell growth factor (EC-GM; Sigma, St. Louis, MO). The EC were seeded by adding 0.1 ml of the EC-GM medium (3.5 x 10⁵ cells per ml.) to flat-bottom 96-well plates (Nunk Roskilde, Denmark).

Confluent monolayer cultures were washed and incubated with selected disaccharide compounds at various concentrations in 50 µl of M-199 at 37°C for 1 h. The compounds were then washed away, and the cultures were incubated over-night with preformed TNF-α, 200 IU per ml, in EC-GM. The cells were then washed three times at 37°C with Hank's solution containing 1% FCS (Hank's 1%), and fixed with 2% glutaraldehyde in PBS. The cells were then washed three times with Hank's 1%, blocked with 2.5% BSA in PBS, and rewashed twice with Hank's 1%. Anti-ICAM-1 and ELAM-1 mAb (Genzyme, Cambridge MA; diluted 1/1000 in PBS) were incubated with the cells for 1 h at 22°C and, then, washed off three times with Hank's 1%. Peroxidase-conjugated goat anti-mouse Ab sigma, diluted 1/1000) was incubated with cells for 1 h and, subsequently, washed off. After adding the o-Phenylenediamine (OPD) prepared by dissolving an OPD hydrochloride tablet in water (OPD is a substrate for peroxidase and can be obtained from Sigma, Cat. No. p9187), the absorbance was detected in an ELISA reader at 492 nm. Samples were assayed in triplicate, and the average of at least three different assays were calculated.

**Table XIID.**

| Effect of Disaccharides on the Expression of Adhesion Molecules by Endothelial Cells in Response to Preformed TNF-α. | | | |
|---|---|---|---|
| TNFα treatment of EC | Inhibitory compound [pg/ml] | Recombinant human TNFα-induced adhesion molecules (O.D 492)*: | |
| None | None | 0.12±0.01 | 0.18±0.01 |
| Yes | None | 1.2±0.1 | 2.2±0.2 |
| Yes | 9392 [50] | 0.6±0.1 | 1.0±0.04 |
| Yes | 9392 [100] | 0.9±0.07 | 1.4±0.03 |
| Yes | 1020 [50] | 0.7±0.05 | 1.2±0.1 |
| Yes | 1020 [100] | 0.9±0.03 | 1.5±0.07 |

| | | | |
|---|---|---|---|
| * Expression of ELAM-1 and ICAM-1 detected by ELISA binding of specific monoclonal antibodies | | | |

The experiments described above demonstrate that pretreatment of EC with disaccharide compounds 9392 and 1020 endows the EC with significant resistance to preformed TNF-α. Hence, the up regulation of EC expression of adhesion molecules induced by TNF-α was inhibited by up to 50%. These results mean that these compounds can influence the target cells of TNF-α (e.g., EC), as well as the cells that produce TNF-α (e.g., T cells, macrophages) to inhibit not only the production of active TNF-α but also the propensity of the target cells to respond to TNF-α (i.e., regulation of peripheral reception of the cytokine). Certain disease states, then, may benefit from the administration of the substance of the present invention by imparting on the target cells of TNF-α a type of resistance against the cell-induced inflammatory response initiated by the activated T-cells and macrophages.

### 5.15.10. The Ability of Substance H-9392 to Suppress the Signs of Experimental Allergic Asthma in Rats

Experimental allergic bronchial asthma is an immediate type hypersensitivity reaction in rats which have been immunized and then rechallenged by inhalation of the priming antigen in a aerosolized solution. (Edelman, et al., Am. Rev. Resp. Dis. (1988) 137:1033-37). The etiology and pathophysiology of this experimental disease closely parallel the naturally occurring human counterpart.

To assess the ability of substance H-9392 to prevent a bronchial asthma attack, 6 male Brown Norway rats were primed to ovalbumin (OVA) by subcutaneous injection of 1 mg of OVA in suspension with 200 mg AlOH/ml 0.9% saline solution accompanied by intraperitoneal injection of 1 ml solution containing 6x10⁶ heat killed *Bordetella pertussis* bacteria (Pasteur Merieux, S.V.), on day zero. Subsequent challenges consisted of a 5 minute period of inhalation of OVA (1 mg/ml solution) aerosolized in a Devilbiss Nebulizer operated at an airflow of 6 L/min. Respiratory Distress (RD) responses were scored as Grade 0, no signs of distress; Grade 1, tachypnea; Grade 2, moderate labored breathing; Grade 3 severe labored breathing with mouth open; Grade 4, loss of consciousness and muscular tone. Sixteen days following primary immunization all animals were exposed to an initial aerosolized challenge to establish the positive control. The animals were coded so that the observer was ignorant of the subject animal's history. All rats were sensitive to OVA and asthma was uniformly induced in all animals.

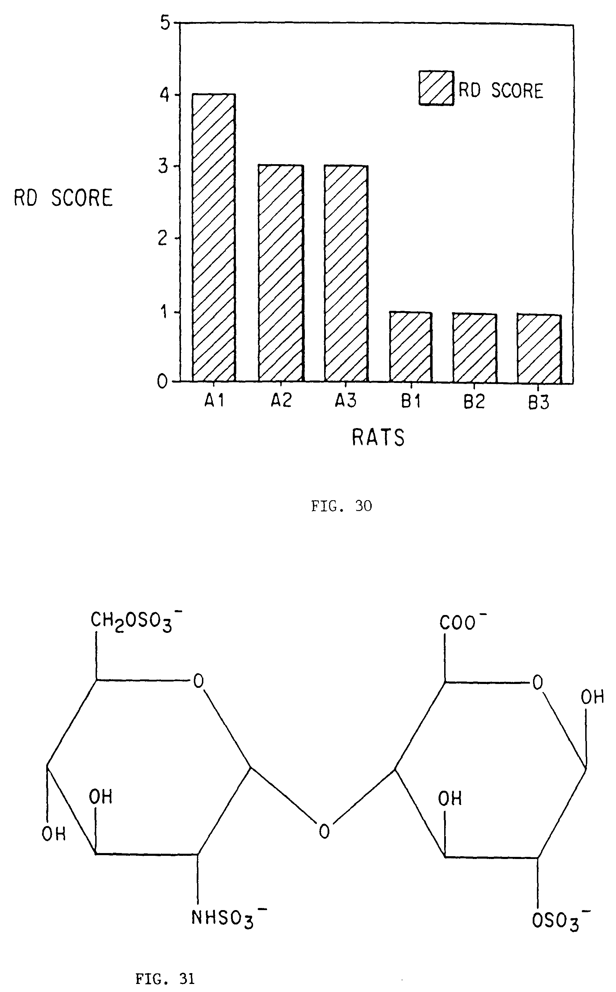

On day thirty, the animals were divided into 2 groups and given either saline (control Group A) or 30 ng of substance H-9392, s.c., (Group B) and challenged on day 35 as above. The results are presented in Fig. 30. As shown in Fig. 30, the animals which received saline only on day 30 experienced Grade 3 or 4 respiratory distress. The animals treated with substance H-9392 displayed mere tachypnea. The results presented show that administration of substance H-9392 (5 days prior to secondary challenge), to an animal with established hypersensitivity, blocks an asthmatic attack.

### 5.16. Results of In Vitro Human PBL Bioassay on Commercially Available Heparin Derived Oligosaccharides

Commercially available samples of heparin derived disaccharides and polysaccharides ranging in molecular weight from 1,800 and 18,000 were tested for biological activity. The results are presented in Table XIII. As is evident from the data obtained, all the samples tested, with one exception, gave either no effect or inconsistent effects. As explained in the table footnote, a test result for a particular entry was designated inconsistent if the same qualitative result was not obtained for all three bioassay runs. (Each entry in all the Tables included in this disclosure, which present results of bioassays, was the product of at least three tests. In the human PBL bioassay, each run used blood obtained from different individuals.)

**Table XIII.**

| Effect of commercially Available Heparin Disaccharides on the Secretion of Active TNF Using Human PBL Bioassay. | | | |
|---|---|---|---|
| Test Material^{a} | conc (pg/ml) | Bioassay of | |
| | | TNF Activity (%) | "R" value % x (pg/ml)⁻¹ |
| H 9517 | ^{b} | Inconsistent^{c} | - |
| H 8642 | ^{b} | Inconsistent | - |
| H 8767 | ^{b} | Inconsistent | - |
| H 0895 | ^{b} | Inconsistent | - |
| H 8892 | ^{b} | No effect | 0 |
| H 9017 | ^{b} | No effect | 0 |
| H 9142 | ^{b} | Inconsistent | - |
| H 9267 | ^{b} | Inconsistent | - |
| H 1020 | 1 | 25% to 35% | 25 to 35 |
| H 9392 | ^{b} | Inconsistent | - |
| MW 1,800^{d} | ^{e} | Inconsistent | - |
| MW 2,400 | ^{e} | Inconsistent | - |
| MW 3,000 | ^{e} | Inconsistent | - |
| MW 3,600 | ^{e} | Inconsistent | - |
| MW 4,200 | ^{e} | Inconsistent | - |
| MW 4,800 | ^{e} | Inconsistent | - |
| MW 5,400 | ^{e} | No effect | 0 |
| MW 6,000 | ^{e} | No effect | 0 |
| MW 9,000 | ^{e} | No effect | 0 |
| MW 16,000 | ^{e} | Inconsistent | - |
| MW 18,000 | ^{e} | Inconsistent | - |

| | | | |
|---|---|---|---|
| ^{a} Product Number found in Sigma Chemical Co. Product Catalog (1992). | | | |
| ^{b} At a conc range of 1 µg/ml - 0.01 pg/ml. | | | |
| ^{c} Three bioassays using PBLs obtained from three separate individuals were performed on each test material. A test result for any given test material was designated "inconsistent" if the same result (i.e., inhibition, augmentation, or no effect) was not obtained for all three bioassays. | | | |
| ^{d} Molecular weight designations for various Heparin Oligosaccharide Fragments, as described in Serbio Product Catalog (December 26, 1991). | | | |
| ^{e} At a conc range of 1 µg/ml - 0.01 pg/ml. | | | |

### 5.17. Results of In Vitro Human PBL Bioassay Versus Assay Kit Based on mAb

A comparison of the activity data obtained from the *in vitro* bioassay based on human PBLs described herein and the results of a conventional monoclonal antibody-based assay kit shows that much more protein is present in the media being tested than what is being detected by the human PBL bioassay as being "active" TNF. The results are presented in Table XIV. For example, the difference between the amounts of protein produced by T cells in "Control" levels (ca. 274 pg/ml) and when Fragmin HPLC-F16 is present (ca. 200 pg/ml) is not nearly as dramatic as the difference in activity detected by the human PBL assay for the same samples (a 100 percent change in activity). Hence, one may conclude from these results that although the activated immune effector cell may secrete significant amounts of TNF protein, even in the presence of the active substances of the present invention, only a small proportion of the secreted protein is active enough to kill TNF-sensitive cells. This conclusion supports the notion that TNF is produced in both active and inactive forms.

**Table XIV**

| Comparison of TNF Activity Detected by Human PBL Bioassay Versus Amount of Protein Detected by mAb Immunoassay Kit. | | | | |
|---|---|---|---|---|
| Test Material | | Bioassay of TNF Activity (% killed) | mAb Assay Kit TNF (pg/ml) | |
| None | 45 | Control | 273.9 | Control |
| Fragmin | | | | |
| HPLC-F1 | 72 | Augₘₐₓ (60%) | 284.5 | Augₘₐₓ (3.8%) |
| HPLC-F3 | 13.5 | Inhₘₐₓ (70%) | 224 | Inhₘₐₓ (17.9%) |
| HPLC-F16 | 0 | Inhₘₐₓ (100%) | 199 | Inhₘₐₓ (27%) |
| DECM | | | | |
| HPLC-F10 | 18 | Inhₘₐₓ (60%) | 225 | Inhₘₐₓ (17.5%) |
| HPLC-F14 | 13.5 | Inhₘₐₓ (70%) | 232 | Inhₘₐₓ (15%) |
| HPLC-F25 | 63 | Augₘₐₓ (40%) | 292 | Augₘₐₓ (6.5%) |
| HPLC-F37 | 90 | Augₘₐₓ (100%) | 301 | Augₘₐₓ (10%) |

### 5.18. Results of Preliminary Investigation of the Structural Characteristics of ECM-Derived Disaccharide

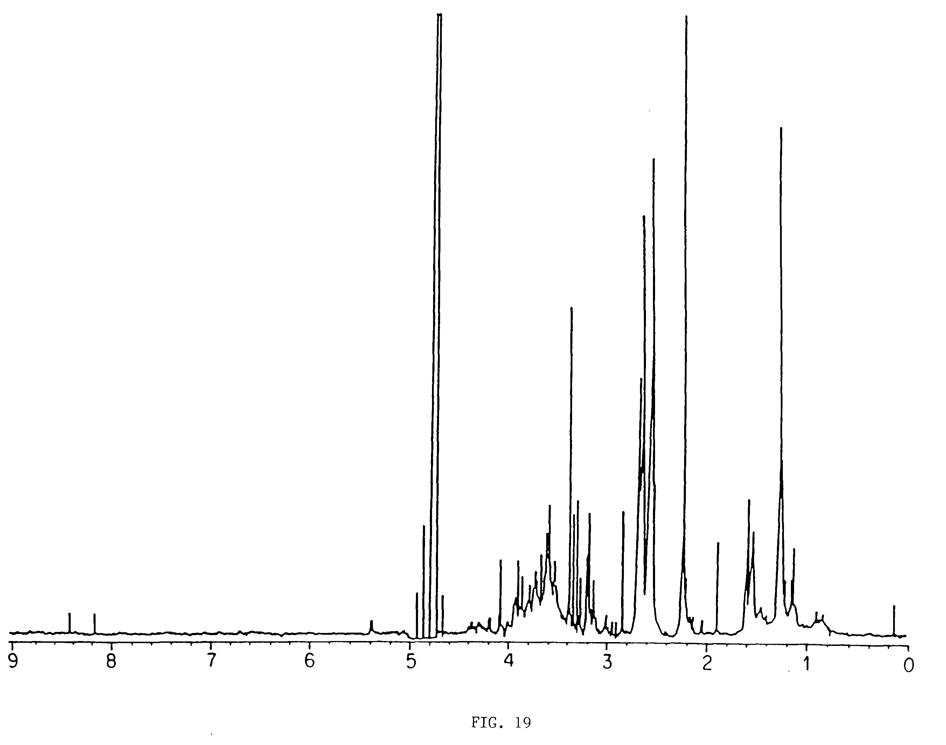

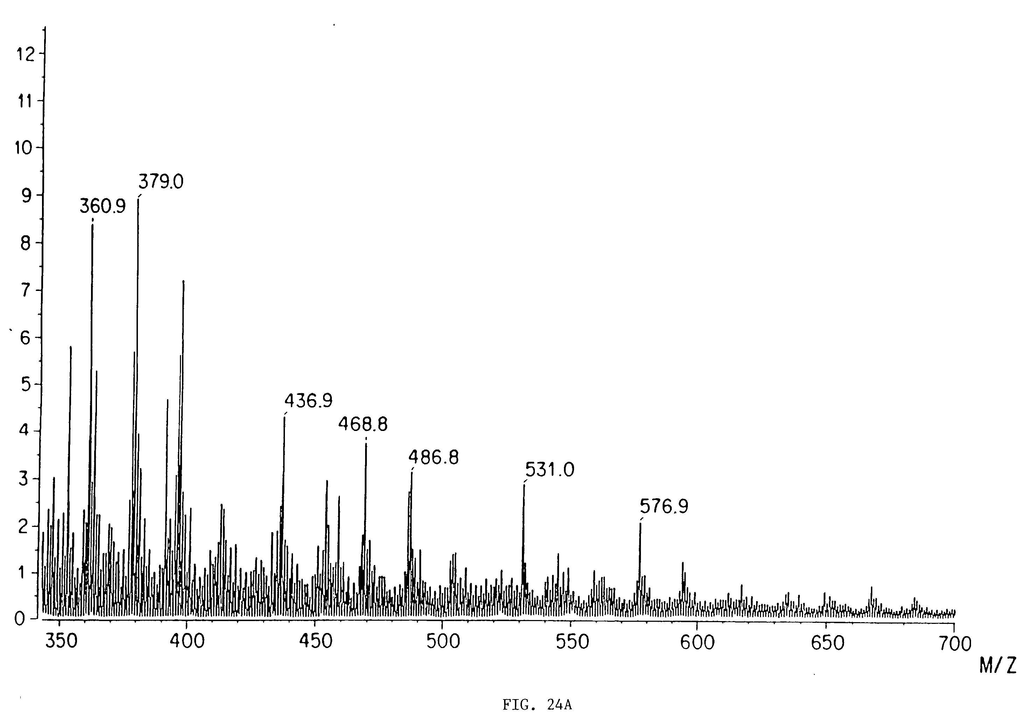

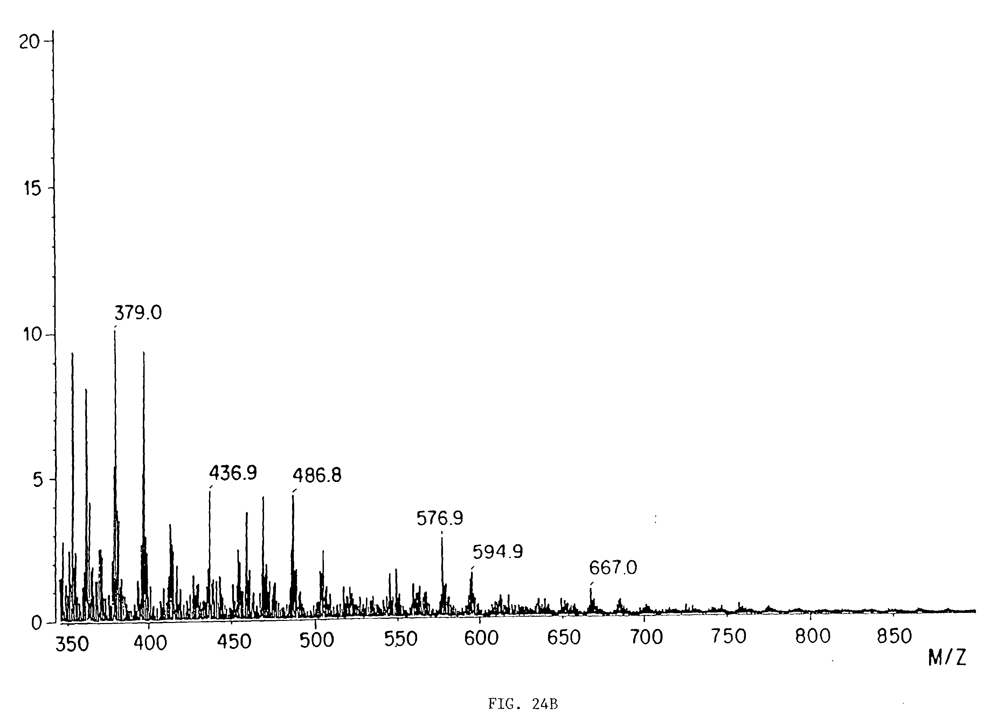

A 20 µg sample of an ECM-derived disaccharide was obtained after HPLC II chromatography (Fig. 11) and desalting, as previously described. Subsequent purification, under SAX-HPLC conditions, showed this sample to be greater than 90% pure (Fig. 24, peak A23/4 at 23.10 min.). However, the proton NMR spectrum of this sample (Fig. 19) shows that a contaminant having a repetitive aliphatic -CH₂- moiety is present. It is important to note, nevertheless, that the SAX-HPLC purification; tested positively for inhibition under both *in vitro* and *in vivo* bioassay conditions.

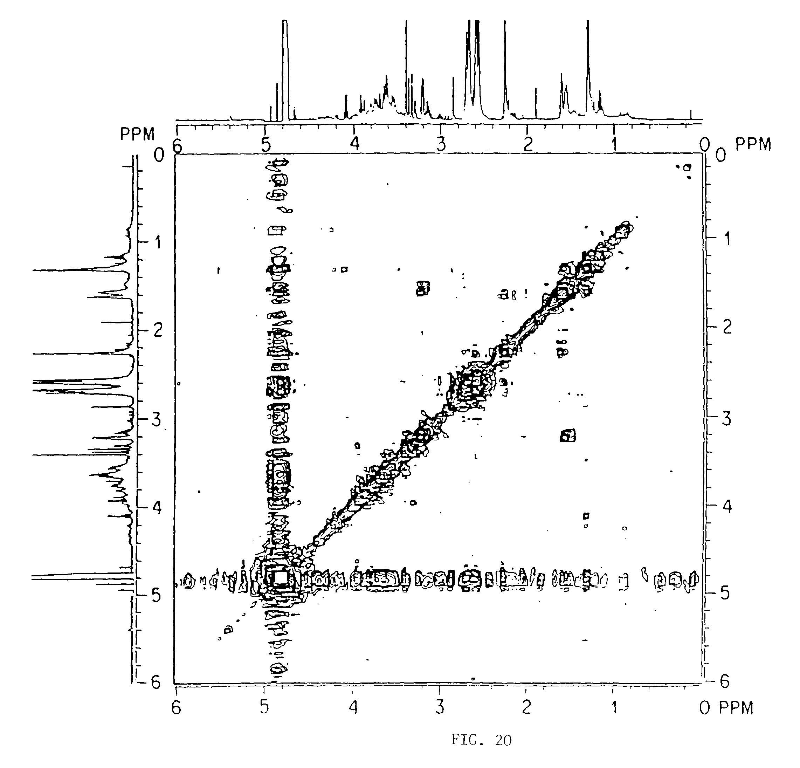

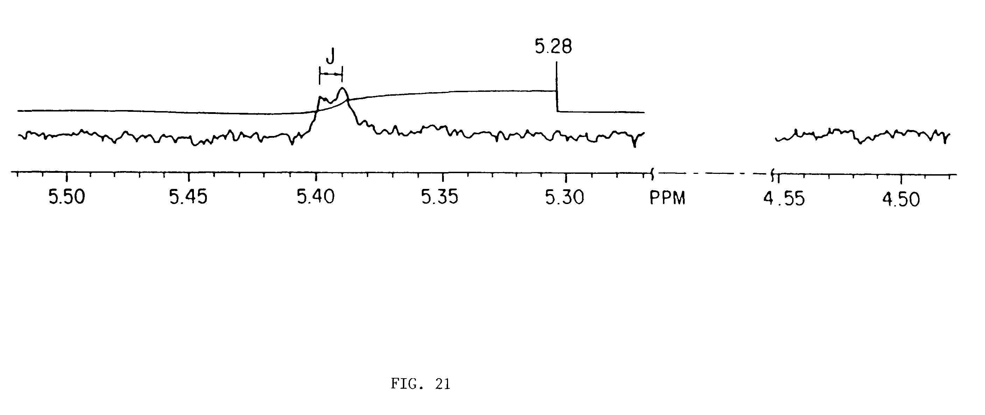

The proton NMR spectrum was recorded in D₂O at 500 MHz at 23 °C. A 2-dimensional COSY spectrum was also recorded. The final matrix size was 512 x 512, N-COSY with presaturation, 1536 scans (Fig. 20). Typical sugar signals are evident between 3-5.5 ppm in both the one dimensional and 2-D spectra. A doublet signal for the anomeric proton is found at 5.39 ppm having a coupling factor of 3 Hertz (Fig. 21), consistent with the presence of a glucosamine sugar unit in an alpha configuration.

The chemical shift of the anomeric proton, together with what is believed to be the beta-glucuronide specificity of placental heparanase, leads to the tentative conclusion that the disaccharide has a glucosamine at the non-reducing end, in an alpha configuration, attached 1-4 to a glucuronic acid residue at the reducing end. Furthermore, the absence of a signal at 6 ppm indicates that the disaccharide is saturated (i.e., there is no double bond at C₄-C₅ of the glucosamine residue). Thus, the heparanase is not an eliminase but apparently a hydrolase.

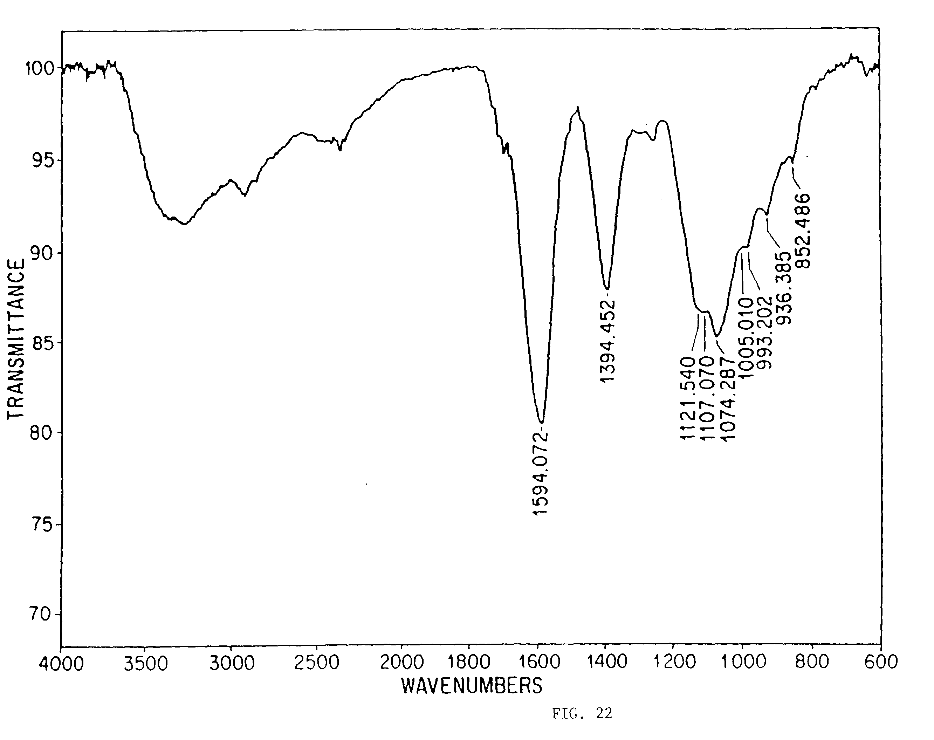

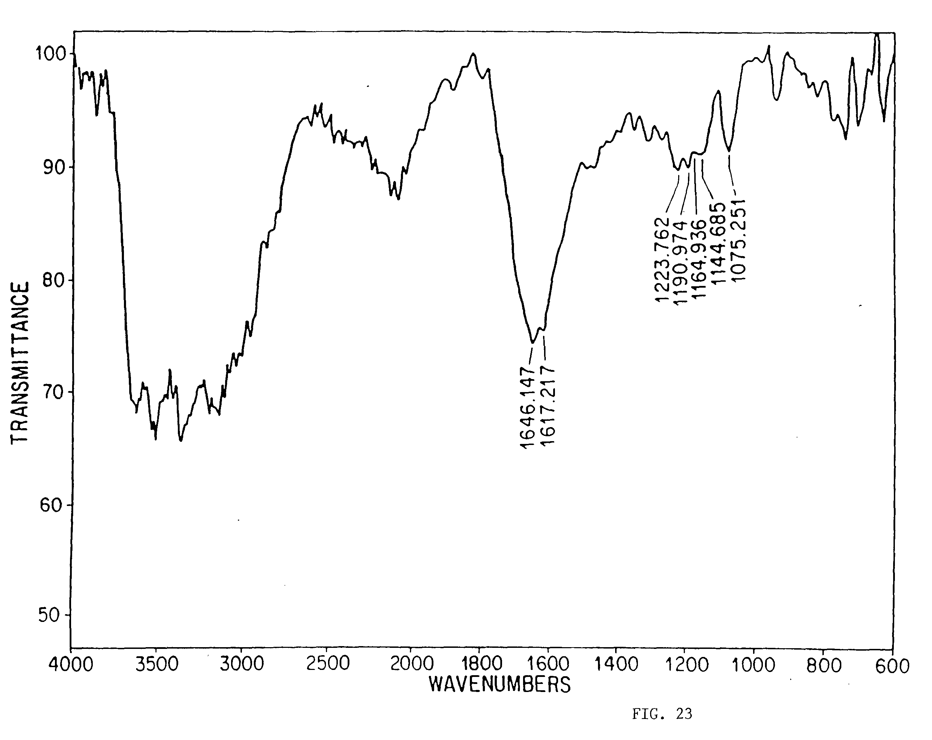

FTIR spectra were recorded on a Mattson Galaxy 6020, equipped with a transmission film detector; DTGS at resolution of 2 cm⁻¹; 128 scans using a ZnSe window. Figures 22-23 indicate the presence of a sulfated compound and a partially desulfated analog, respectively. Fig. 22, in particular, exhibits absorptions at 3500-3000 (characteristic of carboxyl and hydroxyl groups), 1594 (carbonyl), 1394, 1121, 1107, 1074, 1005, 993, 936, and 852 cm⁻¹, at least some of which, particularly the last, are associated with sulfate.

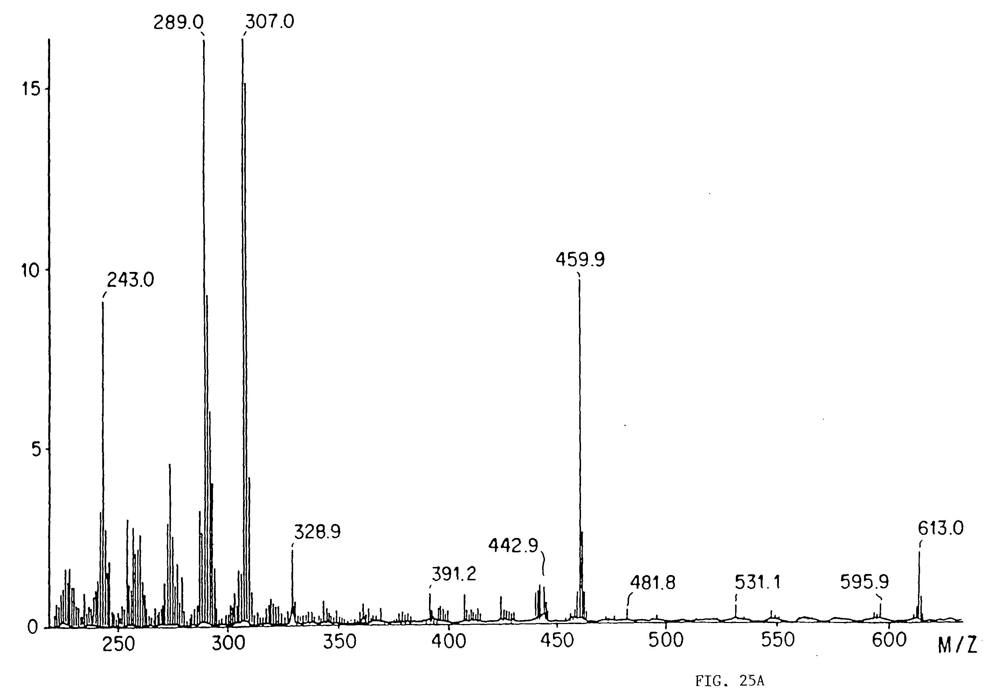

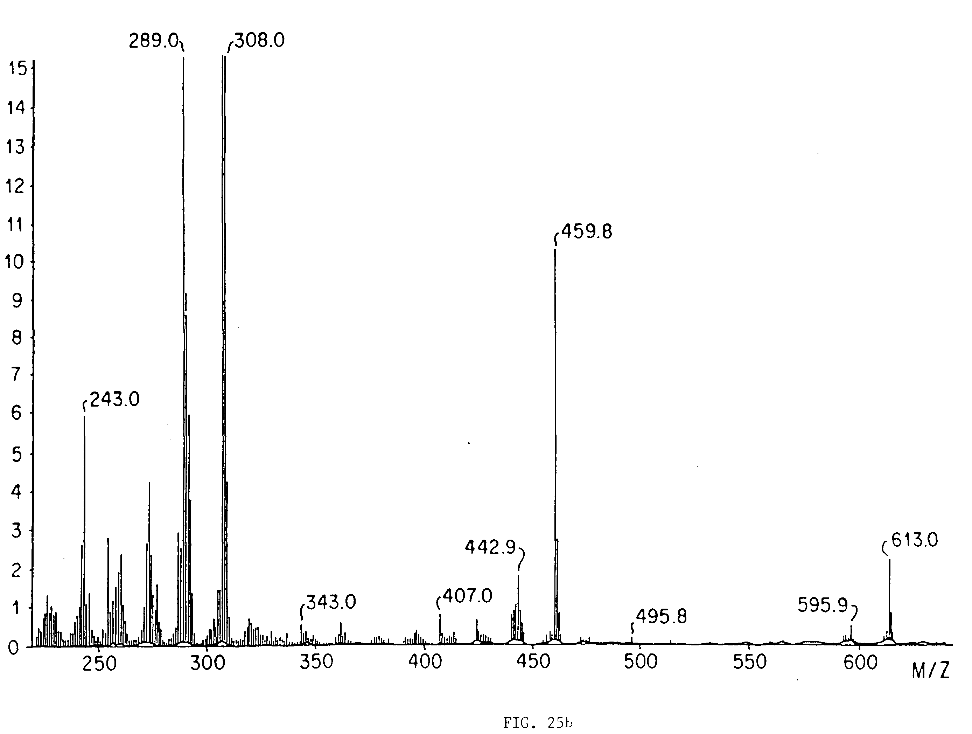

The mass spectrum of a methyl derivative, believed to have lost some sulfate, was obtained on a Jeol JMS HX/110A FAB. Xe beam was used at E = 6 kV, emission current = 10 mA, acceleration voltage was 10 kV. First, the methyl derivative was prepared by treating a sample of the oligosaccharide with diazomethane in acidic media. The methylated product was next extracted with ethyl acetate. A characteristic ion was observed above the background having M/Z (M + H⁺) = 531. Thus, it is believed that the data are consistent with a molecular weight, for a methylated derivative, of about 530. In order to verify the results, two different matrices were used; DTT:thioglycerol (1:1) and methylnitrobenzyl alcohol (Figs. 24 A-B and 25 A-B, respectively). The "A" spectra correspond to sample + matrix, whereas the "B" spectra relate to the specific matrix only.

Based on such mass spectral data, a tentative chemical formula for the methylated (partially desulfated) derivative can be proposed: C₁₃H₂₃NO₁₇S₂, MW = 529.47.

4-O-(2-deoxy-6-O-sulfo-2-sulfoamino-α-D-glucopyranosyl)-(2-O-sulfo-β-D-glucopyranoside) uronic acid is synthesized according to the following protocol.

### 5.19. Synthesis of 4-O-(2-deoxy-6-O-sulfo-2-sulfoamino-α-D-glucopyranosyl)-(2-O-sulfo-β-D-glucopyranoside) uronic acid

### 5.19.1. Preparation of 6-O-Acetyl-2-azido-3,5-di-O-benzyl-2-deoxy-β-D-glucopyranosyl chloride

2-O-Tosyl-1,6:3,4-dianhydro-β-D-galactopyranose **[2]** is prepared from 1,6-anhydro-*µ*-D-glucopyranose **[1],** according to Cerny et al. (1961) Coll. Chech. Chem. Cos. 26:2547. 2-O-Tosyl-1,6-anhydro-β-D-glucopyranose **[3]** is prepared from 2-O-Tosyl-1,6:3,4-dianhydro-β-D-galactopyranose **[2]** according to Cerny et al. (1965), Coll. Chech. Chem. Soc. 30:1151.

1,6:2,3-dianhydro-β-D-mannopyranose **[4]** is prepared from 2-O-Tosyl-1,6-anhydro-β-D-glucopyranose **[3]** according to Stanek and Cerny (1972) SYNTHESIS p. 698. 6-O-Acetyl-2-azido-3,4-di-O-benzyl-2-deoxy-β-D-glucopyranosyl chloride **[A]** is prepared from 1,6:2,3-dianhydro-β-D-mannopyranose **[4]** according to Paulsen and Slenzel (1978) Chem. Ber. 111:2334.

### 5.19.2. Preparation of Methyl-(benzyl-2-O-acetyl-3-O-benzyl-β and α-L-glucopyranosid)-uronate

1,2:5,6-Di-O-isopropylidene-α-D-glucofuranose **[6]** is prepared from D-glucose **[5]** according to Stevens (1978), Methods Carbohydr. Chem. 6:124. 3-O-Benzyl-1,2-O-isopropylidene-α-D-glucofuranose **[7]** is prepared from 1,2:5,6-Di-O-isopropylidene-α-D-glucofuranose **[6]** according to Whistler and Lake (1972), Methods Carbohydr. Chem. 6:286.

Methyl-(benzyl-2-O-acetyl-3-O-benzyl-β and α-L-glucopyranosid)-uronate is prepared from 3-O-Benzyl-1,2-O-isopropylidene-D-glucofuranose **[7]** according to Jacquinet et al. (1984), Carbohydr. Res. 130:221.

### 5.19.3. Condensation of Products of 5.19.1 and 5.19.2

Coupling of the products of Section 5.19.1 and 5.19.2 above is conducted according to Jacquinet et al. (1988), Carbohydr. Res. 174:253. O-deacetylation, hydrolysis, O-Sulfation, reduction, and debenzylation is conducted according to Jacquinet et al. (1984) (supra) to give 4-O-(2-deoxy-6-O-sulfo-2-sulfoamino-α-D-glucopyranosyl)-(2-O-sulfo-β-D-glucopyranoside) uronic acid, which is further purified by SAX-HPLC according to Rice et al. (1985), Anal. Biochem. 150:325. The structure of this product is given in Fig. 31 and it is believed it has the same biological activity as other compounds described herein.

The disclosure of all references cited herein are incorporated herein in their entirety by reference.

## Claims

1. A compound of the formula II 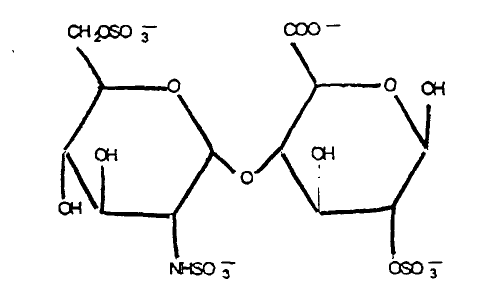 or its pharmaceutically acceptable salt.

2. A pharmaceutical composition comprising an effective amount of the compound of claim 1 or its pharmaceutically acceptable salt.

3. The pharmaceutical composition of claim 2 for inhibiting the production of an active cytokine in a subject.

4. The pharmaceutical composition of claim 3 for inhibiting the production of active TNF-α.

5. A method of using the compound of claim 1 or its pharmaceutically acceptable salt for the preparation of a pharmaceutical composition for the prevention or treatment of a medical condition caused by or related to the inappropriate production of TNF-α by inhibiting the production of said TNF-α.

6. A method of using the compound of claim 1 or its pharmaceutically acceptable salt for the preparation of a pharmaceutical composition for the treatment of a medical condition responsive to an inhibited production of TNF-α.

## Patentansprüche

1. Eine Verbindung der Formel II 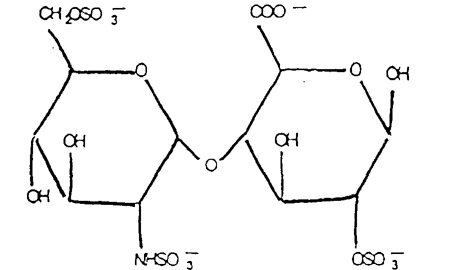 oder sein pharmazeutisch annehmbares Salz.

2. Eine pharmazeutische Zusammensetzung umfassend eine wirksame Menge der Verbindung gemäß Anspruch 1 oder dessen pharmazeutisch annehmbares Salz.

3. Die pharmazeutische Zusammensetzung gemäß Anspruch 2 für die Hemmung der Bildung von aktivem Cytokin in einem Subjekt.

4. Die pharmazeutische Zusammensetzung gemäß Anspruch 3 für die Hemmung der Bildung von aktivem TNF-α.

5. Ein Verfahren zur Verwendung der Verbindung gemäß Anspruch 1 oder dessen pharmazeutisch annehmbaren Salzes für die Herstellung einer pharmazeutischen Zusammensetzung für die Vorbeugung oder Behandlung eines durch unangemessene Bildung von TNF-α hervorgerufenen oder damit verbundenen medizinischen Zustandes, mittels Hemmung der Bildung von TNF-α.

6. Ein Verfahren zur Verwendung der Verbindung gemäß Anspruch 1 oder dessen pharmazeutisch annehmbaren Salzes für die Herstellung einer pharmazeutischen Zusammensetzung für die Behandlung eines auf die Hemmung der Bildung von TNF-α reagierenden medizinischen Zustandes.

## Revendications

1. Un composé de formule II : 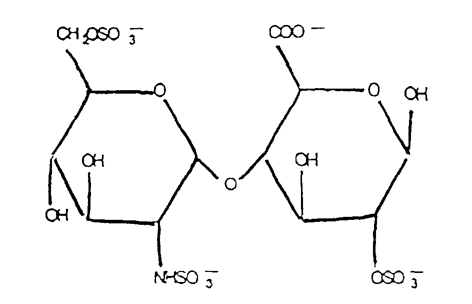 ou un de ses sels pharmaceutiquement acceptables.

2. Une composition pharmaceutique comprenant une quantité efficace de composé selon la revendication 1, ou un de ses sels pharmaceutiquement acceptables.

3. La composition pharmaceutique selon la revendication 2, pour inhiber la production d'une cytokine active chez un sujet.

4. La composition pharmaceutique selon la revendication 3 pour inhiber la production de TNF-α actif.

5. Une méthode d'utilisation du composé selon la revendication 1 ou de ses sels pharmaceutiquement acceptables pour la préparation d'une composition pharmaceutique destinée à la prévention ou au traitement d'un état médical provoqué par ou apparenté à une production inappropriée de TNF-α en inhibant la production dudit TNF-α.

6. Une méthode d'utilisation du composé selon la revendication 1 ou de ses sels pharmaceutiquement acceptables pour la préparation d'une composition pharmaceutique pour le traitement d'un état médical répondant à une inhibition de la production du TNF-α.
